(19) Europäisches Patentamt
European Patent Office
Office européen des brevets

(11) EP 4 363 415 B1

(12) **EUROPEAN PATENT SPECIFICATION**

(45) Date of publication and mention of the grant of the patent:
**10.12.2025 Bulletin 2025/50**

(21) Application number: **22753767.7**

(22) Date of filing: **28.06.2022**

(51) International Patent Classification (IPC):
***C07D 413/14*** (2006.01) ***C07D 417/14*** (2006.01)
***C07D 471/04*** (2006.01) ***A61K 31/4709*** (2006.01)
***A61P 31/06*** (2006.01)

(52) Cooperative Patent Classification (CPC):
**C07D 413/14; C07D 417/14; C07D 471/04**

(86) International application number:
**PCT/IB2022/055995**

(87) International publication number:
**WO 2023/275744 (05.01.2023 Gazette 2023/01)**

# (54) HETEROCYCLIC COMPOUNDS FOR THE TREATMENT OF TUBERCULOSIS

HETEROZYKLISCHE VERBINDUNGEN ZUR BEHANDLUNG VON TUBERKULOSE

COMPOSÉS HÉTÉROCYCLIQUES POUR LE TRAITEMENT DE LA TUBERCULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **29.06.2021 EP 21182608**
**29.06.2021 PT 2021117313**

(43) Date of publication of application:
**08.05.2024 Bulletin 2024/19**

(73) Proprietor: **Tecnimede, Sociedade Técnico-Medicinal, SA**
**2710-089 Sintra (PT)**

(72) Inventors:
- **PARDAL FILIPE, Augusto Eugénio**
**2710-089 Sintra (PT)**
- **DA COSTA PEREIRA ROSA, Carla Patrícia**
**2710-089 Sintra (PT)**
- **CORDEIRO SIMÕES, Ana Vanessa**
**2710-089 Sintra (PT)**
- **RAMOS DAMIL, João Carlos**
**2710-089 Sintra (PT)**
- **SILVA SERRA, João Pedro**
**2710-089 Sintra (PT)**
- **ALMEIDA FERREIRA, Ana Lúcia**
**2710-089 Sintra (PT)**
- **GOMES NEVES, Rita Isabel**
**2710-089 Sintra (PT)**
- **MARQUES HOMEM E SOUSA DOS SANTOS, Sara Alexandra**
**2710-089 Sintra (PT)**

(74) Representative: **Patentree**
**Edificio Net**
**Rua de Salazares, 842**
**4149-002 Porto (PT)**

(56) References cited:
**WO-A1-2019/243971 WO-A2-2013/060744**

- **LAKUM HARSHAD P. ET AL: "Convenient Synthesis of Novel Quinazoline Congeners via Copper Catalyzed C-N/C-S Coupling and Their Biological Evaluation : Novel Quinazoline Derivatives via Cu-Catalyzed C-N/C-S Coupling", JOURNAL OF HETEROCYCLIC CHEMISTRY, vol. 53, no. 1, 23 February 2015 (2015-02-23), US, pages 209 - 219, XP055864085, ISSN: 0022-152X, DOI: 10.1002/jhet.2404**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present disclosure relates to novel 3-substituted-5-(1-(quinolin-2-yl)piperidin-4-yl)-1,2,4-oxadiazole according to general Formula I, II, III, IV and V, or a pharmaceutically acceptable acid or base addition salts, hydrate, solvate, N-oxide, stereochemically isomer forms, in particular diastereoisomer, enantiomer or atropisomers, or mixtures thereof, or a polymorph or ester thereof.

The present disclosure also relates to a pharmaceutical composition comprising a compound or prodrug of Formula I, II, III, IV and V, a combination of said compound with at least one tuberculosis drug as well as its use in the treatment and/or prevention of tuberculosis. Prodrugs are described but not claimed.

## BACKGROUND

Mycobacterium tuberculosis (MTB) has been a major human pathogen since the beginning of human existence. As the human race began to thrive, MTB spread more easily and became one of the leading causes of death by the beginning of the twentieth century (Comas et al. Nat. Genet. 2013, 45, 1176-1182). The prognosis for patients with tuberculosis (TB) improved dramatically with the discovery and use of antitubercular drugs.

Tuberculosis (TB) is an airborne disease caused by Mycobacterium tuberculosis and seven closely related mycobacterial species, namely Mycobacterium bovis, Mycobacterium africanum, Mycobacterium microti, Mycobacterium caprae, Mycobacterium pinnipedii, Mycobacterium canetti and Mycobacterium mungi, which together comprise what is known as the Mycobacterium tuberculosis complex. The majority of TB cases are caused by tubercle bacilli (MTB).

*Mycobacterium tuberculosis* is carried in airborne particles (droplet nuclei) of 1-5 μm in diameter. Infectious droplet nuclei are generated by persons who have pulmonary or laryngeal TB disease. Transmission occurs when an individual inhales droplet nuclei containing *MTB,* and the droplet nuclei traverse the oro-nasal passages, upper respiratory tract, and bronchi to reach the alveoli. These tubercle bacilli are phagocytosed by alveolar macrophages; the majority of these bacilli are destroyed or inhibited. A small number may multiply intracellularly and are released after macrophage destruction. These tubercle bacilli may spread through the lymphatic system or the bloodstream to distant organs, frequently the regional lymph nodes, the lung (apex), the kidneys, the brain and the bones. This process of dissemination stimulates the immune system for a systemic response.

Individuals with latent tuberculosis infection (LTBI) are carriers of the bacteria but do not manifest symptoms of TB disease and do not spread the infection to others. LTBI begins when extracellular bacilli are ingested by macrophages and presented to other white blood cells. This triggers the immune response in which white blood cells kill or encapsulate most of the bacilli, leading to the formation of a granuloma. At this point, LTBI has been established. Latent tuberculosis infection (LTBI) can be detected by using the tuberculin skin test or an interferon-gamma release assay. Within weeks after infection, the immune system is usually able to halt the multiplication of the tubercle bacilli, preventing further progression. In some people, the tubercle bacilli overcome the immune system and multiply, resulting in progression from LTBI to TB disease. The progression from LTBI to TB disease may occur at any time from initial infection up to many years later. Without treatment, approximately 5% of individuals who have been infected with *MTB* will develop disease during the 1$^{st}$ or 2$^{nd}$ year after infection, and another 5% will develop the disease sometime later in life. Thus, without treatment, approximately 10% of individuals infected with *MTB* and having a normal immune system will develop TB disease at some point in their lives.

Tuberculosis disease can occur in pulmonary (more than 60%) and extrapulmonary sites. Patients with pulmonary TB usually have a cough and an abnormal chest radiograph and can be infectious. Although the majority of TB cases are pulmonary TB, TB can in fact occur in almost any anatomical site or as disseminated disease. Extrapulmonary TB disease occurs in places other than the lungs, including the larynx, lymph nodes, pleura, brain, kidneys, or bones and joints.

Individuals with extrapulmonary TB disease are usually not infectious unless they also have pulmonary disease. Individuals with extrapulmonary TB disease are usually also not infectious unless the extrapulmonary disease is located in the oral cavity or the larynx, or the extrapulmonary disease includes an open abscess or lesion in which the concentration of organisms is high, especially if drainage from the abscess or lesion is extensive, or if drainage fluid is aerosolized. Individuals with TB pleural effusions may have underlying pulmonary TB that is masked on chest radiograph because the effusion fluid compresses the lung.

Miliary TB occurs when tubercle bacilli enter the bloodstream and disseminate to all parts of the body where they grow and cause disease in multiple sites. This condition is rare but serious. It is most common in infants and children younger than 5 years of age, and in severely immunocompromised persons. Miliary TB can be detected in a single organ, including the brain, in several organs, or throughout the whole body. The condition is characterized by a large amount of TB bacilli which may easily be missed, and is fatal if untreated. Up to 25% of patients with miliary TB may have meningeal

infection.

When TB occurs in the tissue surrounding the brain or spinal cord, it is called tuberculous meningitis. Tuberculous meningitis is often seen at the base of the brain on imaging studies. Symptoms include headache, decreased level of consciousness, and neck stiffness. The duration of illness before diagnosis is variable and relates in part to the presence or absence of other sites of involvement. In many cases, patients with meningitis have abnormalities on a chest radiograph consistent with old or current TB, and often have miliary TB.

Demographics of individuals with increased risk of progression from LTBI to TB are: human immunodeficiency virus (HIV)-infected individuals; children; individuals recently infected with *MTB* (< 2 years); history of untreated or inadequately treated TB disease, including fibrotic changes on chest X-ray consistent with prior TB disease; individuals on immunosuppressive therapy (e.g., tumour necrosis factor-alpha antagonists, systemic corticosteroids [≥ 15 mg of prednisone per day]; individuals on immunosuppressive therapy following organ transplantation; silicosis; diabetes mellitus; chronic renal failure; leukemia; cancer of the head, neck, or lung; gastrectomy or jejunoileal bypass; low body mass index (BMI); cigarette smokers; drug and/or alcohol abusers; medically underserved, low-income populations.

Drug-resistant TB is caused by *MTB* microorganisms that are resistant to the drugs normally used to treat the disease. Drug-resistant TB is transmitted in the same way as drug-susceptible TB and is no more infectious than drug-susceptible TB. However, delay in the recognition of drug resistance or prolonged periods of infectiousness may facilitate increased transmission and further development of drug resistance.

Multidrug-resistant TB (MDR TB) is caused by microorganisms resistant to the most effective anti-TB drugs, namely isoniazid and rifampin. These drugs are considered first-line drugs.

Extensively drug-resistant TB (XDR TB) is a relatively rare type of drug-resistant TB. Extensively drug-resistant TB is resistant to isoniazid and rifampin, plus any fluoroquinolone and at least one of three injectable second-line drugs, i.e., amikacin, kanamycin, or capreomycin. Because XDR TB is resistant to first-line and second-line drugs, patients are left with treatment options that are more toxic, more expensive, and much less effective.

The major goals of the treatment for TB disease are: to cure the individual patient; to minimize risk of death and disability and to reduce the transmission of *MTB* to others. To ensure the achievement of these goals, TB disease must be treated for at least 6 months and in some cases even longer. Most of the bacteria are killed during the first 8 weeks of treatment. However, there are persistent organisms that require longer treatment. If treatment is not continued for a duration that is long enough, the surviving bacteria may cause the patient to become ill and infectious again, potentially with drug-resistant strains of MTB. There are several options for daily and intermittent therapy, but the goal of treatment for TB disease should be to provide the safest and most effective therapy in the shortest period of time. Given adequate treatment, almost all patients will recover and be cured. Regimens for the treatment of TB disease must contain multiple drugs to which the bacteria are susceptible. The standard of care for initiating treatment of TB disease is four-drug therapy. Treatment with a single drug can lead to the development of a bacterial population resistant to that drug. Likewise, the addition of a single drug to a failing anti-TB regimen can lead to additional resistance. When two or more drugs to which in vitro susceptibility has been demonstrated are given together, each helps prevent the emergence of tubercle bacilli resistant to others.

There are several TB drug products approved for treatment of TB disease in the United States and in Europe. These drug products are classified as first-line drugs, for example isoniazid, rifampin, pyrazinamide, ethambutol, rifabutin, rifapentine; or second-line drugs, for example streptomycin, cycloserine, capreomycin, 4-aminosalicylic acid, levofloxacin, moxifloxacin, gatifloxacin, amikacin, kanamycin, ethionamide. Isoniazid, rifampin, pyrazinamide and ethambutol form the core of the initial treatment regimen for TB. Recently two new drug products were approved for treating MDR TB: bedaquiline and delamanid.

Rifabutin, approved for preventing *Mycobacterium avium* complex disease in patients with HIV infection, but not approved for TB disease, is useful for treating TB disease in patients concurrently taking drugs that interact with rifampin (e.g., certain antiretroviral drugs).

Amikacin and kanamycin, are nearly identical aminoglycoside drugs used in treating patients with TB disease caused by drug-resistant organisms. Rifabutin and rifapentine may also be considered first-line drug products under certain circumstances. Streptomycin was formerly considered to be a first-line drug and, in some instances, is still used in the initial treatment regimen. However, increasing prevalence of resistance to streptomycin in many parts of the world has decreased its overall usefulness. The remaining drugs are reserved for special situations such as drug intolerance or resistance.

There are four basic treatment regimens recommended for treating individuals with TB disease caused by tubercle bacilli that are known or presumed to be susceptible to isoniazid, rifampin, pyrazinamide and ethambutol. Each treatment regimen consists of an initial 2-month treatment phase followed by a continuation phase of either 4 or 7 months. The 4-month continuation phase is used for majority of the patients. Although these regimens are broadly applicable, there are modifications that should be made under specified circumstances.

The initial phase of treatment is crucial for preventing the emergence of drug resistance and determine the outcome of the regimen. Four drugs - isoniazid, rifampin, pyrazinamide and ethambutol - should be included in the initial

treatment regimen until the results of drug-susceptibility tests are available. Each of the drugs in the initial regimen plays an important role. Isoniazid and rifampin allow for short-course regimens with high cure rates. Pyrazinamide has potent sterilizing activity which allows further shortening of the regimen from 9 to 6 months. Ethambutol helps to prevent the emergence of rifampin resistance when primary isoniazid resistance is present. If drug-susceptibility test results are known and the organisms are fully susceptible, ethambutol need not be included. For children, ethambutol is usually not recommended except when the risk of drug resistance is high or for children who have "adult-type" (upper lobe infiltration, cavity formation) TB disease.

The continuation phase of treatment is given for either 4 or 7 months. The 4-month continuation phase should be used in patients with uncomplicated, noncavitary, drug-susceptible TB, if there is documented sputum conversion within the first 2 months. The 7-month continuation phase is recommended for patients with cavitary or extensive pulmonary TB disease caused by drug-susceptible organisms and whose sputum culture obtained at the time of completion of 2 months of treatment is positive; and patients whose initial phase of treatment did not include pyrazinamide or patients being treated with once-weekly isoniazid and rifabutin and whose sputum culture at the time of completion of the initial phase (i.e., after 2 months) is positive.

In human immunodeficiency virus (HIV)-infected individuals, extrapulmonary TB disease is often accompanied by pulmonary TB. The treatment approach for patients co-infected with HIV and *MTB* is complex, and the clinical and public health consequences associated with the failure of treatment and other negative outcomes are serious. HIV-infected patients usually take several medicines and some of which interact with anti-TB drugs.

The treatment regimens mentioned above are effective for people living with HIV, with two exceptions due to increased risk of developing acquired drug resistance: once-weekly administration of isoniazid and rifabutin in the continuation phase should not be used in any HIV-infected patient; and patients with CD4+ lymphocytes counts < 100 should be treated with daily or three times weekly therapy in both the initial and continuation phase. Every effort should be made to use a rifamycin-based regimen for the entire course of therapy in co-infected patients. The key role of the rifamycins in the success of TB disease treatment mandates that the drug-drug interactions between the rifamycins and antiretroviral drugs be managed appropriately, rather than using TB treatment regimens that do not include a rifamycin or by withholding antiretroviral therapy until completion of anti-TB therapy.

Of particular concern is the interaction of rifamycins with antiretroviral agents and other anti-infective drugs. Rifampin can be used for the treatment of TB with certain combinations of antiretroviral agents. Rifabutin, which has fewer drug-drug interactions due to its decreased induction of the cytochrome P450 system, may also be used in place of rifampin and appears to be equally effective. Therefore, patients with HIV-related TB disease should be treated with a regimen including a rifamycin for the full course of TB disease treatment, unless the isolate is resistant to rifamycins or the patient has a severe side effect that is clearly due to rifamycins.

Six months should be considered the minimum duration of treatment for HIV-infected adults, even for patients with culture-negative TB disease. If there is evidence of a slow or suboptimal response (e.g., cultures are still positive after 2 months of therapy), the continuation phase should be prolonged to 7 months (a total of 9 months of treatment).

Drug-resistant TB disease can develop via two different mechanisms: primary and secondary drug resistance. Primary resistance occurs in persons who are initially exposed to and infected with resistant organisms. Secondary resistance, or acquired resistance, develops during TB therapy, either because the patient was treated with an inadequate regimen or because the patient did not take the prescribed regimen appropriately or because of other conditions such as drug malabsorption or drug-drug interactions leading to low serum levels.

Drug resistance in a patient with newly diagnosed TB disease is suspected on the basis of previous treatment, contact with a known drug-resistant case, or time spent in a region in which drug resistance is common. Drug resistance can be proven only by drug-susceptibility testing. Patients with strains of M. tuberculosis resistant to both isoniazid and rifampin (multidrug-resistant) are at high risk for treatment failure, relapse, further acquired resistance, or death.

Today's first-line antituberculosis therapy for drug-susceptible *MTB* infection is very effective in bacillary clearance, provided there is full compliance by the patient. It is important to note that despite having an effective treatment regimen, *MTB* still caused an estimated 10.4 million infections and 1.7 million deaths in 2017. Tuberculosis is one of the top 10 causes of death worldwide. About one-quarter of the world's population has latent TB. Those infected with TB bacteria have a 5-15% lifetime risk of falling ill with TB. However, patients with compromised immune systems, such as individuals living with HIV, malnutrition or diabetes, or tobacco users, have a much higher risk of falling ill (WHO, WHO Global Tuberculosis Report 2017, 2017).

Current front-line treatment consists of a 2 month 'intensive' period of a four-drug regimen containing rifampicin (RIF), isoniazid (INH), pyrazinamide (PZA), and ethambutol (EMB) and is followed by a longer "continuation" phase of RIF and INH to eradicate the remaining bacilli that have entered a dormant, slowly replicating latent phase (Hoagland DT et al., Adv Drug Deliv Rev. 2016 Jul 1, 102, 55-72).

The major concern of this epidemic is the emergence of resistant-bacteria; multidrug-resistant TB (MDR-TB) remains a public health crisis and a health security threat. WHO estimates that there were 600 000 new cases with resistance to rifampicin - the most effective first-line drug, of which 490 000 had MDR-TB (Hoagland DT et al., Adv Drug

Deliv Rev. 2016 Jul 1, 102, 55-72).

Quinolines are known in the art and have been used for tuberculosis. For example, document EP1527050B1 teaches the use of diarylquinoline in the treatment of mycobacterial diseases, in particular it discloses the first-in-class ATP synthase inhibitor Bedaquiline.

Document US2016113919A1 describes the use of Chloroquine stereoisomer for treating tuberculosis related diseases. Chloroquine belongs to the class of 4-aminoquinoline. 2-Aminoquinoline compounds are known in the art and have been used before in the pharmaceutical industry although for different purposes.

Document US7868022B2 describes 2-aminoquinoline compounds useful as inhibitor of beta-secretase (BACE) for the treatment of Alzheimer's disease (AD) and related diseases.

Document WO2012025237A9 describes 2-aminoquinoline compounds as modulators of KCNQ2/3 for the prophylaxis of pain.

Document US7989628B2 discloses 2-aminoquinolines derivatives as 5-HT5A receptor antagonists useful in the treatment of a range of CNS and PNS disorders.

Document WO2009001060A3 and journal article by Tantry, S. J. et al. (Med. Chem. Commun. 2016, 7, 1022-1032) disclose 2,4-diaminoquinolines as anti-tuberculosis agents. In particular they both describe examples of 4-amino-2-piperidinequinoline wherein the piperidine and the quinoline aryl rings are not further substituted.

Document WO2017001661A1 relates to compounds for treatment of tuberculosis, wherein the compounds have a 2-(4-(4-(trifluoromethoxy)phenyl)piperidin-1-yl)quinoline.
Document WO2019243971 discloses piperazine substituted quinoline derivatives for treatment of tuberculosis.

There is an urgent need for new and innovative treatment strategies to fight tuberculosis. Considerable progress has been made in TB drug discovery in recent past years. A number of drug candidates are at different stages of clinical development. Diaryl quinolone (TMC207 or Bedaquiline) received expedited approval from the FDA for the treatment of MDR-TB while Delamanid was approved by the European Union's European Medicines Agency (EMA). Both drugs have been conditionally approved because of adverse effects. The U.S. Food and Drug Agency (FDA) has approved Bedaquiline for MDR-TB and Delamanid as a compassionate care option for XDR-TB and TDR-TB infections, and the EMA approved both agents for MDR-TB. These are the two new drug therapies approved after 40 years. A lack of viable alternatives in late-stage clinical development is indicative of the state of drug discovery in this field, and innovation to drive new projects is desperately needed.

These facts are disclosed in order to illustrate the technical problem addressed by the present disclosure.

## General description

The present disclosure relates to the use of 5-(1-(quinolin-2-yl)piperidin-4-yl)-1,2,4-oxadiazole substituted with a 3-aryl or heteroaryl that can surprisingly be used for treating and/or preventing tuberculosis. Any references in the description to methods of treatment refer to the compounds, pharmaceutical compositions and medicaments of the present invention for use in a method for treatment of the human (or animal) body by therapy.

None of the prior art documents disclose the use of said compounds for treatment of tuberculosis. In fact, the prior art documents do not disclose compounds with a substituted 3-aryl or heteroaryl 5-(1-(quinolin-2-yl)piperidin-4-yl)-1,2,4-oxadiazole core. The prior art documents also do not teach or suggest that these compounds might be used in the treatment of tuberculosis.

In an embodiment, the compounds disclosed in the present disclosure are for use in the treatment of diseases caused by Mycobacterium tuberculosis (MTB), such as pulmonary TB, extrapulmonary TB or disseminated tuberculosis (TB).

An aspect of the present disclosure relates to compound of general formula I or a pharmaceutically acceptable salt, hydrate, solvate, N-oxide, stereoisomer, diastereoisomer, enantiomer, atropisomer, polymorph or ester thereof:

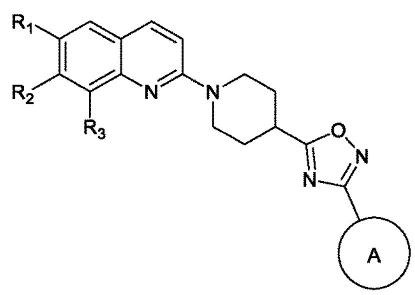

Formula I

wherein

$R_1$, $R_2$, $R_3$ and ring A are independently selected from each other;
Ring A is selected from: substituted or unsubstituted aryl, substituted or unsubstituted heteroaryl, or fused heteroaroaryl ring selected from pyridin-2-yl, pyridin-3-yl, pyridin-4-yl, pyrazin-2-yl, 1H-pyrrolo[2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Halogen, $C_1$-$C_6$ alkoxy, or unsubstituted heteroaryl;
$R_2$ is selected from H or OH;
$R_3$ is selected from H, Halogen, $C_1$-$C_6$ alkoxy, or unsubstituted heteroaryl.

Another aspect of the present disclosure relates to compound of general formula I or a pharmaceutically acceptable salt, hydrate, solvate, N-oxide, stereoisomer, diastereoisomer, enantiomer, atropisomer, polymorph or ester thereof:

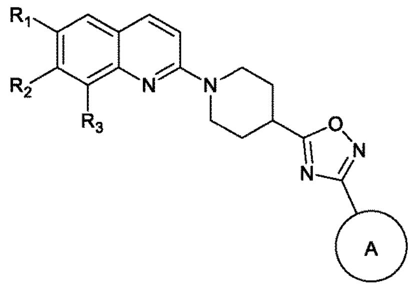
Formula I

wherein

$R_1$, $R_2$, $R_3$ and ring A are independently selected from each other;
Ring A is a substituted or unsubstituted ring selected from pyridin-2-yl, pyridin-3-yl, pyridin-4-yl, pyrazin-2-yl, 1H-pyrrolo[2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Halogen, $C_1$-$C_6$ alkoxy, or unsubstituted heteroaryl;
$R_2$ is selected from H or OH;
$R_3$ is selected from H, Halogen, $C_1$-$C_6$ alkoxy, or unsubstituted heteroaryl.

In an embodiment, the compound of general formula I or a pharmaceutically acceptable salt, hydrate, solvate, N-oxide, stereoisomer, diastereoisomer, enantiomer, atropisomer, polymorph or ester thereof:

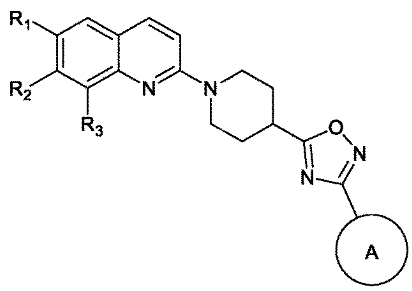
Formula I

wherein

$R_1$, $R_2$, $R_3$ and ring A are independently selected from each other;
Ring is a substituted or unsubstituted ring selected from pyridin-2-yl, pyridin-3-yl, pyridin-4-yl, pyrazin-2-yl, 1H-pyrrolo [2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Cl, $OCH_3$ or unsubstituted heteroaryl;
$R_2$ is selected from H or OH;
$R_3$ is selected from H, Cl, $OCH_3$ or unsubstituted heteroaryl.

In an embodiment for better results, the compound is represented by compound of formula I:

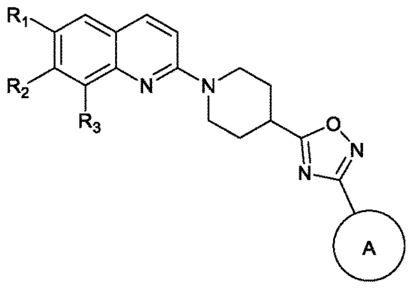

Formula I

wherein

Ring A is selected from pyridin-2-yl, pyridin-3-yl, pyridin-4-yl, pyrazin-2-yl, 1H-pyrrolo[2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Cl, $OCH_3$ or pyridine-4-yl;
$R_2$ is selected from H or OH;
$R_3$ is selected from H, Cl, $OCH_3$ or pyridine-4-yl.

In an embodiment, the compound is represented by compound of Formula II wherein:

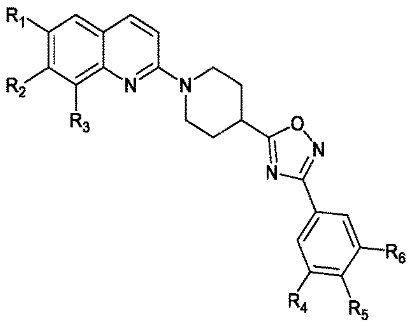

Formula II

wherein

$R_1$, $R_2$, $R_3$, $R_4$, $R_5$ and $R_6$ are independently selected from each other;
$R_1$ is selected from H, Cl, $OCH_3$ or pyridine-4-yl;
$R_2$ is selected from H or OH;
$R_3$ is selected from H, Cl, $OCH_3$ or pyridine-4-yl;
$R_4$ is selected from H, $OCH_3$, $CF_3$, $OCF_3$, $NO_2$ or $NH_2$;
$R_5$ is selected from H, $OCH_3$, $CF_3$, $OCF_3$, F, $NO_2$ or OH;
$R_6$ is selected from H, $OCH_3$, $CF_3$ or $OCF_3$.

In an embodiment for better results, for the compound of Formula II, $R_3$ is H or $OCH_3$.

In an embodiment for better results, for the compound of Formula II, $R_4$ is H, $OCH_3$ or $CF_3$.

In an embodiment for better results, for the compound of Formula II, $R_6$ is selected from H, $CF_3$ or OH.

In an embodiment for better results, for the compound of Formula II, $R_6$ is selected from H, $OCH_3$ or $CF_3$.

In an embodiment for better results, the compound is represented by compound of Formula III:

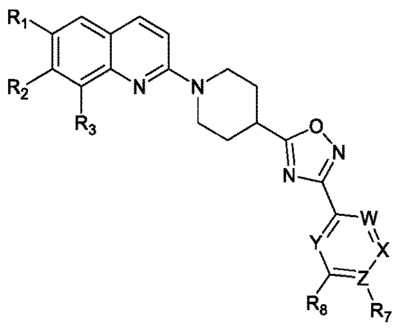

Formula III

wherein

$R_1$, $R_2$, $R_3$, $R_7$ and $R_8$ are independently selected from each other;
X, Y, Z and W are independently selected from C or N;
$R_1$ is selected from H, Cl, $OCH_3$ or pyridine-4-yl;
$R_2$ is selected from H or OH;
$R_3$ is selected from H, Cl, $OCH_3$ or pyridine-4-yl;
$R_7$ is selected from H, $OCH_3$, $CF_3$, $NHCH_3$, $NH_2$, Cl, piperidinyl, piperazinyl, morpholinyl, pyrrolidinyl, $NH(CH_2)OH$ or $CH_3$;
$R_8$ is selected from H, F or OH.

In an embodiment for better results, for the compound of Formula III, X is N;
In an embodiment for better results, for the compound of Formula III, $R_1$ is H, Cl, $OCH_3$ or pyridine-4-yl;
In an embodiment for better results, for the compound of Formula III, $R_3$ is H or $OCH_3$;
In an embodiment for better results, for the compound of Formula III, $R_7$ is H, $CF_3$, Cl, piperidinyl, piperazinyl, pyrrolidinyl or $NH(CH_2)OH$.
In an embodiment for better results, the compound is represented by compound of Formula IV:

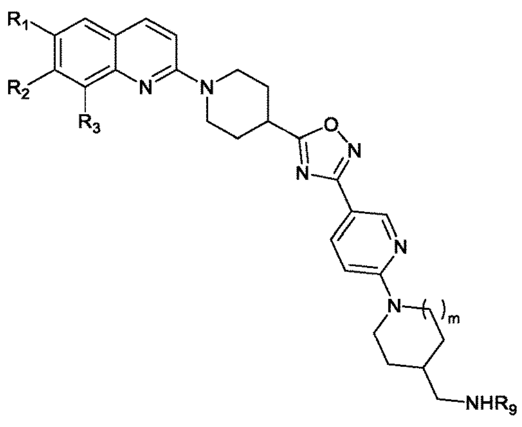

Formula IV

Wherein

$R_1$, $R_2$, $R_3$ and $R_9$ are independently selected from each other;
$R_1$ is selected from H, Cl, $OCH_3$ or pyridine-4-yl;
$R_2$ is selected from H or OH;
$R_3$ is selected from H, Cl, $OCH_3$ or pyridine-4-yl;
m is selected from 0 or 1;
$R_9$ is selected from H or tert-butyl carbamate.

In an embodiment for better results, the compound is represented by compound of Formula V:

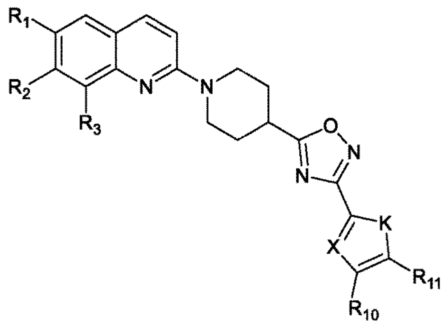

Formula V

wherein

$R_1$, $R_2$, $R_3$, $R_{10}$ and $R_{11}$ are independently selected from each other;
$R_1$ is selected from H, Cl, $OCH_3$ or pyridine-4-yl;
$R_2$ is selected from H or OH;
$R_3$ is selected from H, Cl, $OCH_3$ or pyridine-4-yl;
X is C or N;
K is S or O;
$R_{10}$ and $R_{11}$ are selected from H or attached together with carbons to form a 6-methoxybenzo[d]thiazol-2-yl.

In an embodiment for better results, in formula II $R_3$ is H or $OCH_3$.
In an embodiment for better results, in formula II. $R_4$ is H, $OCH_3$ or $CF_3$.
In an embodiment for better results, in formula II $R_5$ is selected from H, $CF_3$, $OCF_3$ or OH.
In an embodiment for better results, in formula II $R_6$ is selected from H, $OCH_3$ or $CF_3$.
In an embodiment for better results, in formula III X is N.
In an embodiment for better results, in formula III $R_1$ is H, Cl, $OCH_3$ or pyridine-4-yl.
In an embodiment for better results, in formula III $R_3$ is H, Cl, or $OCH_3$.
In an embodiment for better results, in formula III $R_7$ is H, $CF_3$, Cl, piperidinyl, piperazinyl, pyrrolidinyl or $NH(CH_2)OH$.
In an embodiment for better results, for compounds of Formula I, II, III, IV and V the salt is a hydrochloride or a trifluoroacetate.
In an embodiment for better results, the compound is selected from:

5-(1-(6-chloroquinolin-2-yl)piperidin-4-yl)-3-phenyl-1,2,4-oxadiazole;
5-(1-(6-chloroquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-(trifluoromethoxy)phenyl)-1,2,4-oxadiazole;
5-(1-(8-chloroquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-(trifluoromethyl)phenyl)-1,2,4-oxadiazole hydrochloride;
3-(3,5-dimethoxyphenyl)-5-(1-(8-methoxyquinolin-2-yl)piperidin-4-yl)-1,2,4-oxadiazole;
5-(1-(8-methoxyquinolin-2-yl)piperidin-4-yl)-3-(3-(trifluoromethyl)phenyl)-1,2,4-oxadiazole;
3-(3,5-bis(trifluoromethyl)phenyl)-5-(1-(8-methoxyquinolin-2-yl)piperidin-4-yl)-1,2,4-oxadiazole;
5-(1-(8-methoxyquinolin-2-yl)piperidin-4-yl)-3-(pyridin-4-yl)-1,2,4-oxadiazole;
5-(1-(8-methoxyquinolin-2-yl)piperidin-4-yl)-3-(pyridin-3-yl)-1,2,4-oxadiazole hydrochloride;
5-(1-(8-methoxyquinolin-2-yl)piperidin-4-yl)-3-(6-(trifluoromethyl)pyridin-3-yl)-1,2,4-oxadiazole;
3-(3-methoxyphenyl)-5-(1-(8-methoxyquinolin-2-yl)piperidin-4-yl)-1,2,4-oxadiazole hydrochloride;
5-(1-(8-methoxyquinolin-2-yl)piperidin-4-yl)-3-(3-(trifluoromethoxy)phenyl)-1,2,4-oxadiazole;
3-(4-methoxyphenyl)-5-(1-(8-methoxyquinolin-2-yl)piperidin-4-yl)-1,2,4-oxadiazole;
5-(1-(6-chloroquinolin-2-yl)piperidin-4-yl)-3-(pyridin-3-yl)-1,2,4-oxadiazole hydrochloride;
5-(1-(8-methoxyquinolin-2-yl)piperidin-4-yl)-3-(pyrazin-2-yl)-1,2,4-oxadiazole hydrochloride;
7-hydroxy-8-methoxy-2-(4-(3-(pyridin-3-yl)-1,2,4-oxadiazol-5-yl)piperidin-1-yl)quinoline 1-oxide;
5-(1-(8-chloroquinolin-2-yl)piperidin-4-yl)-3-(pyridin-3-yl)-1,2,4-oxadiazole hydrochloride;
5-(1-(8-chloroquinolin-2-yl)piperidin-4-yl)-3-(4-fluorophenyl)-1,2,4-oxadiazole;
3-(pyridin-3-yl)-5-(1-(8-(pyridin-4-yl)quinolin-2-yl)piperidin-4-yl)-1,2,4-oxadiazole hydrochloride;
5-(1-(6-methoxyquinolin-2-yl)piperidin-4-yl)-3-(pyridin-3-yl)-1,2,4-oxadiazole;
5-(1-(6-methoxyquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-nitrophenyl)-1,2,4-oxadiazole;

4-(5-(1-(8-methoxyquinolin-2-yl)piperidin-4-yl)-1,2,4-oxadiazol-3-yl)phenol;
5-(1-(8-methoxyquinolin-2-yl)piperidin-4-yl)-3-(pyridin-2-yl)-1,2,4-oxadiazole hydrochloride;
3-(5-(1-(8-methoxyquinolin-2-yl)piperidin-4-yl)-1,2,4-oxadiazol-3-yl)pyridine 1-oxide;
3-(pyridin-3-yl)-5-(1-(6-(pyridin-4-yl)quinolin-2-yl)piperidin-4-yl)-1,2,4-oxadiazole;
5-(5-(1-(8-methoxyquinolin-2-ylpiperidin-4-yl)-1,2,4-oxadiazol-3-yl)-N-methylpyridin-2-amine;
5-(1-(8-methoxyquinolin-2-yl)piperidin-4-yl)-3-(3-nitrophenyl)-1,2,4-oxadiazole;
5-(5-(1-(8-methoxyquinolin-2-yl)piperidin-4-yl)-1,2,4-oxadiazol-3-yl)pyridin-2-amine hydrochloride;
3-(6-chloropyridin-3-yl)-5-(1-(8-methoxyquinolin-2-yl)piperidin-4-yl)-1,2,4-oxadiazole hydrochloride;
5-(1-(8-methoxyquinolin-2-yl)piperidin-4-yl)-3-(pyrimidin-2-yl)-1,2,4-oxadiazole;
5-(1-(8-methoxyquinolin-2-yl)piperidin-4-yl)-3-(6-(piperidin-1-yl)pyridin-3-yl)-1,2,4-oxadiazole;
5-(1-(8-methoxyquinolin-2-yl)piperidin-4-yl)-3-(6-(piperazin-1-yl)pyridin-3-yl)-1,2,4-oxadiazole;
2-((5-(5-(1-(8-methoxyquinolin-2-yl)piperidin-4-yl)-1,2,4-oxadiazol-3-yl)pyridin-2-yl)amino)ethan-1-ol hydrochloride;
5-(5-(1-(8-methoxyquinolin-2-yl)piperidin-4-yl)-1,2,4-oxadiazol-3-yl)pyridin-3-ol;
5-(1-(8-methoxyquinolin-2-yl)piperidin-4-yl)-3-(1H-pyrrolo[2,3-b]pyridin-5-yl)-1,2,4-oxadiazole;
3-(furan-2-yl)-5-(1-(8-methoxyquinolin-2-yl)piperidin-4-yl)-1,2,4-oxadiazole;
5-(1-(8-methoxyquinolin-2-yl)piperidin-4-yl)-3-(6-methylpyridin-3-yl)-1,2,4-oxadiazole tert-butyl ((1-(5-(5-(1-(8-methoxyquinolin-2-yl)piperidin-4-yl)-1,2,4-oxadiazol-3-yl)pyridin-2-yl)piperidin-4-yl)methyl)carbamate;
tert-butyl ((1-(5-(5-(1-(8-methoxyquinolin-2-yl)piperidin-4-yl)-1,2,4-oxadiazol-3-yl)pyridin-2-yl)pyrrolidin-3-yl) methyl)carbamate;
(1-(5-(5-(1-(8-methoxyquinolin-2-yl)piperidin-4-yl)-1,2,4-oxadiazol-3-yl)pyridin-2-yl)pyrrolidin-3-yl)methanamine trifluoroacetate;
(1-(5-(5-(1-(8-methoxyquinolin-2-yl)piperidin-4-yl)-1,2,4-oxadiazol-3-yl)pyridin-2-yl)piperidin-4-yl)methanamine trifluoroacetate;
3-(6-methoxybenzo[d]thiazol-2-yl)-5-(1-(8-methoxyquinolin-2-yl)piperidin-4-yl)-1,2,4-oxadiazole;
5-(1-(8-methoxyquinolin-2-yl)piperidin-4-yl)-3-(thiophen-2-yl)-1,2,4-oxadiazole;
4-(5-(5-(1-(8-methoxyquinolin-2-yl)piperidin-4-yl)-1,2,4-oxadiazol-3-yl)pyridin-2-yl)morpholine;
3-(5-(1-(8-methoxyquinolin-2-yl)piperidin-4-yl)-1,2,4-oxadiazol-3-yl)aniline hydrochloride;
3-(6-methoxypyridin-3-yl)-5-(1-(8-methoxyquinolin-2-yl)piperidin-4-yl)-1,2,4-oxadiazole;
3-(5-fluoropyridin-3-yl)-5-(1-(8-methoxyquinolin-2-yl)piperidin-4-yl)-1,2,4-oxadiazole;
5-(1-(8-methoxyquinolin-2-yl)piperidin-4-yl)-3-(6-(pyrrolidin-1-yl)pyridin-3-yl)-1,2,4-oxadiazole hydrochloride.

In an embodiment, the compound is preferably selected from:

5-(1-(8-methoxyquinolin-2-yl)piperidin-4-yl)-3-(4-(trifluoromethoxy)phenyl)-1,2,4-oxadiazole;

5-(1-(8-methoxyquinolin-2-yl)piperidin-4-yl)-3-(4-(trifluoromethyl)phenyl)-1,2,4-oxadiazole hydrochloride;

3-(3,5-dimethoxyphenyl)-5-(1-(8-methoxyquinolin-2-yl)piperidin-4-yl)-1,2,4-oxadiazole;

5-(1-(8-methoxyquinolin-2-yl)piperidin-4-yl)-3-(3-(trifluoromethyl)phenyl)-1,2,4-oxadiazole;

5-(1-(8-methoxyquinolin-2-yl)piperidin-4-yl)-3-(pyridin-3-yl)-1,2,4-oxadiazole hydrochloride;

5-(1-(8-methoxyquinolin-2-yl)piperidin-4-yl)-3-(6-(trifluoromethyl)pyridin-3-yl)-1,2,4-oxadiazole;

5-(1-(8-chloroquinolin-2-yl)piperidin-4-yl)-3-(pyridin-3-yl)-1,2,4-oxadiazole hydrochloride;

5-(1-(6-methoxyquinolin-2-yl)piperidin-4-yl)-3-(pyridin-3-yl)-1,2,4-oxadiazole;

4-(5-(1-(8-methoxyquinolin-2-yl)piperidin-4-yl)-1,2,4-oxadiazol-3-yl)phenol;

3-(pyridin-3-yl)-5-(1-(6-(pyridin-4-yl)quinolin-2-yl)piperidin-4-yl)-1,2,4-oxadiazole;

3-(6-chloropyridin-3-yl)-5-(1-(8-methoxyquinolin-2-yl)piperidin-4-yl)-1,2,4-oxadiazole hydrochloride;

5-(1-(8-methoxyquinolin-2-yl)piperidin-4-yl)-3-(6-(piperidin-1-yl)pyridin-3-yl)-1,2,4-oxadiazole;

5-(1-(8-methoxyquinolin-2-yl)piperidin-4-yl)-3-(6-(piperazin-1-yl)pyridin-3-yl)-1,2,4-oxadiazole;

2-((5-(5-(1-(8-methoxyquinolin-2-yl)piperidin-4-yl)-1,2,4-oxadiazol-3-yl)pyridin-2-yl)amino)ethan-1-ol hydrochloride;

5-(1-(8-methoxyquinolin-2-yl)piperidin-4-yl)-3-(6-(pyrrolidin-1-yl)pyridin-3-yl)-1,2,4-oxadiazole hydrochloride.

Another aspect of the present disclosure relates to a pharmaceutical composition comprising the compounds of the present disclosure and a pharmaceutical acceptable excipient. Preferably, a therapeutically effective amount of the compounds described in the present disclosure or a pharmaceutically acceptable salt or solvate thereof, together with a pharmaceutically acceptable excipient.

Another aspect of the present disclosure relates to compound of general formula I or a pharmaceutically acceptable salt, hydrate, solvate, N-oxide, stereoisomer, diastereoisomer, enantiomer or atropisomer, polymorph or ester thereof:

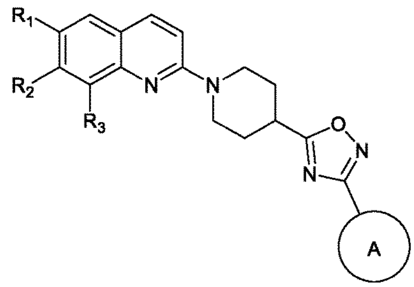

Formula I

wherein

Ring A is selected from: substituted or unsubstituted aryl, a substituted or unsubstituted heteroaryl, or fused heteroaroaryl ring selected from pyridin-2-yl, pyridin-3-yl, pyridin-4-yl, 1H-pyrrolo[2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Cl, $OCH_3$ or unsubstituted heteroaryl;
$R_2$ is selected from H or OH.

$R_3$ is selected from H, Cl, $OCH_3$ or unsubstituted heteroaryl.In an embodiment for better results, the compound is represented by compound of Formula I:

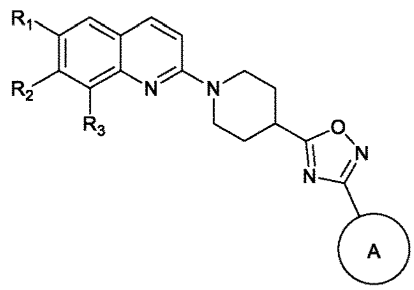

Formula I

wherein

$R_1$ is selected from H, $OCH_3$ or pyridine-4-yl;
$R_2$ is H;
$R_3$ is selected from H, Cl or $OCH_3$;
Ring A is selected from aryl substituted with $OCF_3$, $CF_3$, 3,5-$OCH_3$, OH, pyridin-3-yl, pyridin-3-yl substituted with $CF_3$, Cl, piperidine, piperazine, ethanolamine or pyrrolidine.

In an embodiment for better results, the compound is represented by compound of Formula II:

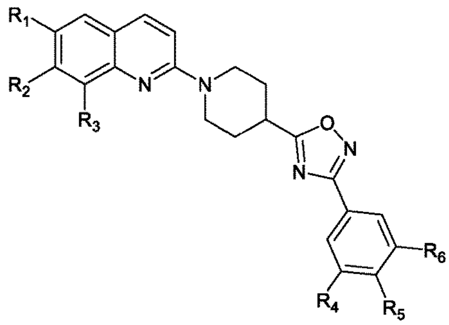

Formula II

wherein

$R_1$ is selected from H, Cl, $OCH_3$ or pyridine-4-yl;
$R_2$ is selected from H or OH;
$R_3$ is selected from H, Cl, $OCH_3$ or pyridine-4-yl;
$R_4$ is selected from H, $OCH_3$, $CF_3$, $OCF_3$, $NO_2$ or $NH_2$;
$R_5$ is selected from H, $OCH_3$, $CF_3$, $OCF_3$, F, $NO_2$ or OH;
$R_6$ is selected from H, $OCH_3$, $CF_3$ or $OCF_3$.

In an embodiment for better results, the compound is represented by compound of Formula IV:

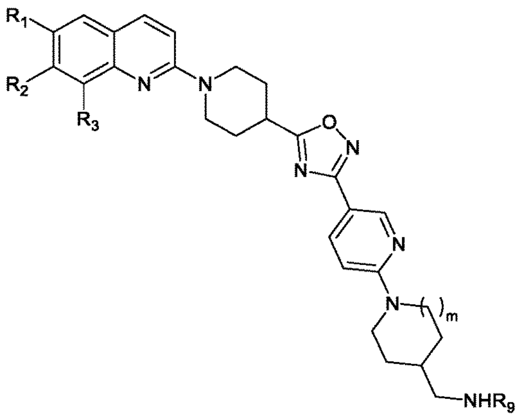

Formula IV

wherein

$R_1$ is selected from H, Cl, $OCH_3$ or pyridine-4-yl;
$R_2$ is selected from H or OH;
$R_3$ is selected from H, Cl, $OCH_3$ or pyridine-4-yl;
m is selected from 0 or 1;
$R_9$ is selected from H or tert-butyl carbamate.

In an embodiment, the compound is represented by compound of Formula V:

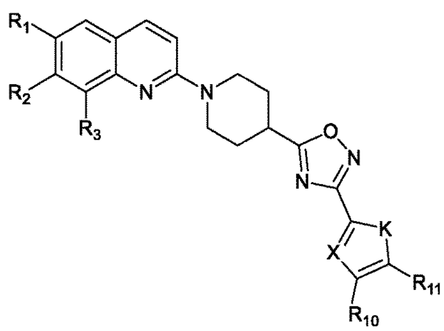

Formula V

Wherein

$R_1$ is selected from H, Cl, $OCH_3$ or pyridine-4-yl;
$R_2$ is selected from H or OH;
$R_3$ is selected from H, Cl, $OCH_3$ or pyridine-4-yl;
X is C or N;
K is S or O;
$R_{10}$ and $R_{11}$ are selected from H or attached together with carbons to form a substituted aryl ring; preferably wherein the aryl is substituted with a $OCH_3$.

In an embodiment, for the compounds of Formula I or Formula II or Formula III, Formula IV, or Formula V, the salt is hydrochloride or trifluoroacetate.

Another aspect of the present disclosure relates to the use of the compounds of the present disclosure in medicine or veterinary. Preferably, the compound is used in the treatment or prevention of tuberculosis.

In an embodiment, the compound now disclosed is used in combination with at least one anti-HIV agent. Preferably, the anti-HIV agent is selected from the following: HIV protease inhibitor (PI), HIV nucleoside reverse transcriptase inhibitor (NRTI), HIV non-nucleoside reverse transcriptase inhibitor (NNRTI), HIV integrase inhibitor.

In an embodiment, the compound is combined with at least another TB drug. Preferably, the TB drug is selected from the following: isoniazid, rifamycin and derivatives, pyrazinamide, ethambutol, cycloserine, ethionamide, streptomycin, amikacin, kanamycin, rifampin (rifampicin), aminoglycosides, capreomycin, p-aminosalicyclic acid, fluoroquinolones such as levofloxacin, moxafloxacin or gatifloxacin, or mixtures thereof and all other first and second-line drug products.

In an embodiment, the combination of the compound of the present disclosure together with an anti-HIV agent or a TB drug is administered simultaneously, separately or sequentially.

The present disclosure also relates to a pharmaceutical composition comprising the disclosed compound and a pharmaceutically acceptable excipient.

In an embodiment, said pharmaceutical composition may comprise (i) a therapeutically effective amount of the disclosed compound or a pharmaceutically acceptable salt or solvate thereof; and (ii) a pharmaceutically acceptable excipient.

In an embodiment, surprisingly the compounds of the present disclosure have been found to inhibit the growth of Mycobacterium tuberculosis. In particular, the compounds possess growth inhibition in vitro at concentration not more than 31.25 μM, in particular not more than 15.6 μM.

In an embodiment, the compounds of the present disclosure are non-cytotoxic for HepG2 cell line, metabolically stable in human microsomes, stable in plasma and relatively permeable under gastrointestinal tract parallel artificial membrane.

In the present disclosure 'Halogen' as used herein refers to fluoro (F), chloro (Cl), bromo (Br) or iodo (I), unless otherwise specified.

In the present disclosure 'Aryl' as used herein refers to a $C_6$-$C_{12}$ monocyclic or bicyclic hydrocarbon ring wherein at least one ring is aromatic. Examples of 'Aryl' group include phenyl, naphthalenyl, tetrahydronephthalenyl and indane.

In the present disclosure 'Heteroaryl' as used herein refers to a 5-6 membered monocyclic aromatic or a fused 8-10 membered bicyclic aromatic ring, which might be partially saturated, which monocyclic or bicyclic ring contains 1 to 4 heteroatoms selected from oxygen, nitrogen, and sulphur. Examples of monocyclic aromatic ring include furyl, furazanyl, imidazolyl, isothiazolyl, isoxazolyl, oxazolyl, oxadiazolyl, thiadiazolyl, pyrrolyl, pyranyl, pyrazolyl, pyrimidyl, pyridazinyl, pyrazinyl, pyridyl, thienyl, thiazolyl, triazolyl, tetrazolyl, triazinyl, tetrazinyl and the like. Examples of such bicyclic aromatic rings include azaindolyl, benzothienyl, benzoimidazolyl, benzoxazolyl, benzoisoxazolyl, benzothiazolyl, benzoisothiazolyl, benzoxadiazolyl, benzothiadiazolyl, benzofuranyl, cinnolinyl, furopyridyl, imidazopyridyl, indolyl, isoindolyl, isobenzofuranyl, indolizinyl, indazolyl, isoquinolinyl, naphthyridinyl, quinolinyl, quinazolinyl, quinoxalinyl, phthalazinyl, pteridinyl, purinyl, pyrrolopyridyl.

In the present disclosure 'Bicyclic ring' and 'Fused' in the context of a bicyclic ring refer to two rings which are joined together across a bond between two atoms (e.g. naphthalene), across a sequence of atoms to form a bridge (e.g. quinuclidine, adamantyl) or together at a single atom to form a spiro compound (e.g. 1,4-dioxa-8-aza-spiro[4.5]decane).

In the present disclosure 'Substituted aryl' or 'Substituted heteroaryl' used herein with reference to R group means that a particular R group (e.g. Ra, Rb, Rc) can be substituted with one or more groups selected from Rx, halogen, OH, ORx, SH, SRx, OCORx, SCORx, COOH, NH2, NO2, CN, NHRx, NRxRy, CORx, CSRx, COORx, OPhRxRy, CONH2, CONHRx, CONxNy, CONHOH, CONHNH2, CONHORx, CH2CH2NRxRx, CH2CH2NRxRy, NHCONH2, NRxCORy, NHCORx, CONHPhRx, CONRxRx, CONHNH2, COHRx, NHCORx, NHSO2Rx, wherein Rx and Ry are independently selected from $C_1$-$C_6$ alkyl, $C_1$-$C_6$-alkoxy, $C_1$-$C_6$ haloalkyl, $C_1$-$C_6$ haloalkoxy, aryl, heteroaryl, $C_3$-$C_{10}$ cycloalkyl, alkyl $C_1$-$C_6$ aryl and heterocyclyl, or Rx and Ry, together with the heteroatom to which they are joined, can form an heterocyclyl.

In the present disclosure 'Unsubstituted heteroaryl' as used herein refers to pyridin-2-yl, pyridin-3-yl, pyridin-4-yl, pyrazin-2-yl, pyrimidin-5-yl, furan-2-yl, thiophen-2-yl or indolyl.

In the present disclosure ' $C_1$-$C_6$ alkyl' refers to a linear or branched saturated hydrocarbon group containing from one to six carbon atoms. Examples of '$C_1$-$C_6$ alkyl' include methyl, ethyl, isopropyl, n-propyl, tert-butyl, sec-butyl, n-butyl, isobutyl, n-pentyl, isopentyl, n-hexyl. Preferably the hydrocarbon is linear.

In the present disclosure '$C_1$-$C_6$Haloalkyl' as used herein refers to a $C_1$-$C_6$ alkyl group as defined above substituted by one or more halogen atoms.

In the present disclosure ' $C_1$-$C_6$ alkoxy' as used herein refers to a -O($C_1$-$C_6$ alkyl) group wherein $C_1$-$C_6$ alkyl is as defined above. Examples of such groups include methoxy, ethoxy, isopropoxy, butoxy, pentoxy and hexyloxy.

In the present disclosure '$C_1$-$C_6$haloalkoxy' as used herein refers to a -O($C_1$-$C_6$alkyl) group as defined above substituted by one or more halogen atoms.

In the present disclosure 'Pharmaceutical acceptable' such as pharmaceutically acceptable carrier, excipient, etc. means pharmacologically acceptable and substantially non-toxic to the subject to which the particular compound is administrated.

In the present disclosure 'Pharmaceutically acceptable salts' of compounds of the present disclosure include salts with organic bases, salt with inorganic bases, salts with organic acids and salt with basic or acidic amino acids as well as quaternary ammonium salts. Salts with acids may, in particular, be employed in some instances. Examples of such salts include hydrochloric salt and trifluoroacetate salt.

In an embodiment, the compounds of the present disclosure are crystallised or recrystallized from solvents such as organic solvents. In such cases, solvate (e.g. hydrate) may form. When in a solvated form, the additional solvent is alcohols such as isopropanol (Isop) or ethanol (EtOH).

In the present disclosure, the described but not claimed prodrug form of the pharmacologically-active compounds disclosed herein are degraded in vivo to yield the compounds according to the present disclosure. Prodrugs are usually (but not always) of lower potency at the target receptor than the compounds to which they degrade into. Prodrugs are particularly useful when the desired compound has chemical or physical properties that make its administration difficult or inefficient. For example, the desired compound can only be poorly soluble, it is poorly transported across the mucosal epithelium, or it may have an undesirably short plasma half-life. Further discussion on prodrug can be found in Redasani, V.K. et al. 'Prodrug Design', Elsevier, UK, 2015.

In an embodiment, prodrug forms of pharmacologically-active compounds are compounds of the present disclosure according to Formula I, II, III, IV and V, the pharmaceutically acceptable acid or base addition salts, the stereochemically isomeric form, the tautomeric forms thereof and the N-oxide forms thereof, having an acid group which is esterified or aminated; or an amino group which forms a carbamate or an amide. Included in esterified acid groups are the 'Pharmaceutically acceptable esters'.

In an embodiment, the aminated groups include groups of the formula -CONRdRe, wherein Rd is H, $C_1$-$C_6$ alkyl, and Re is OH, H, $C_1$-$C_6$ alkyl.

In the present disclosure, carbamate include groups of the formula -NRfCOORg, wherein Rf is H or $C_1$-$C_6$ alkyl and Rg is $C_1$-$C_6$ alkyl or $C_1$-$C_6$ alkyl substituted with -OCO($C_1$-$C_6$ alkyl).

In the present disclosure, amine include groups of the formula -NRfRh, wherein Rf is as previously described and Rh is H or $C_1$-$C_6$alkyl.

In the present disclosure, 'Pharmaceutically acceptable esters' of compounds are compounds which one or more carboxyl group (e.g. -COOH) are modified by reaction with an alcoholic substituent A-OH so as to yield -COOA groups, wherein A is $C_1$-$C_6$alkyl.

In the present disclosure, N-oxide forms of the compounds according to Formula I, II, III, IV and V are meant to comprise those compounds wherein one or several Nitrogen atoms are oxidized to the so-called N-oxide, particularly those N-oxide wherein the Nitrogen of the Amine radical is oxidized.

The skilled person will appreciate that compounds of the present disclosure include those that are chemically stable. That is, compounds of the invention include those that are sufficiently robust to survive isolation from the reaction mixture to a useful degree of purity.

General methods for the preparation of salts and esters are well known to the person skilled in the art. Pharmaceutically acceptability of salts and esters will depend on a variety of factors, including formulation processing details, and specific in vivo criteria, and the person skilled in the art would be able to assess such details having the present disclosure in consideration.

If a chiral centre or another form of isomeric centre, such as geometric isomers are present in a compound of the present disclosure, all forms of isomers, including enantiomers and diastereomers, are intended to be enclosed herein. Compounds of the present disclosure containing a chiral centre are used as a mixture of isomers in all ratios (e.g. racemic mixture), an enantiomerically enriched mixture, or in a pure form. Individual isomers, or enantiomers are obtained by methods known in the art, such as optical resolution of products or intermediates (for example chiral chromatography (e.g. chiral HPLC) or supercritical fluid chromatography (SFC)). Wherein compounds of the disclosure may exist as alternative

tautomeric forms (e.g. keto/enol, imine/enamine), the disclosure relates to the individual tautomers in isolation, and to a mixture of the tautomers in all proportions.

Other aspects of the present disclosure are the provision of compounds according to the present disclosure, or a pharmaceutical composition comprising said compound for use in the treatment and/or prevention of tuberculosis.

In an embodiment, the compounds of general Formula I, II, III, IV and V are used in combination with other TB drug products such as isoniazid, rifampin, pyrazinamide, ethambutol, fluoroquinolones, aminoglycosides or other first-line and second-line drug products.

In an embodiment, the present disclosure describes the use of one of the compounds disclosed in combination with at least one TB drug or, a pharmaceutical composition comprising that compound in combination with at least one TB drug for use in the treatment or prevention of tuberculosis.

In an embodiment, the TB drug is selected from the following: isoniazid, rifamycin and derivatives, pyrazinamide, ethambutol, cycloserine, ethionamide, streptomycin, amikacin, kanamycin, rifampin (rifampicin), capreomycin, p-aminosalicyclic acid, aminoglycosides, fluoroquinolones such as levofloxacin, moxifloxacin or gatifloxacin, and all other first and second-line drug products.

In an embodiment, the compounds of general formula I, II, III, IV and V, whether or not in combination with another pharmacologically active compound, are administered by oral, nasal, rectal, bronchial (inhaled), topical (eye drops, buccal, and sublingual), vaginal, parenteral (subcutaneous, intramuscular, intravenous and intradermal) or via an implanted reservoir, and are prepared by any methods known in the art or pharmacy.

In an embodiment, the composition is prepared by bringing into association the above defined active ingredient with a carrier. In general, the formulations are prepared by uniformly and intimately bringing into association the active ingredient with liquid carriers or finely divided solid carriers, or both, and then if necessary, shaping the product.

In an embodiment, the present disclosure describes the use of a compound of general Formula I, II, III, IV and V as defined above in the preparation of an anti-mycobacterial agent, particularly as agent for the treatment or prevention of tuberculosis.

In an embodiment, the compounds of Formula I, II, III, IV and V or a pharmaceutically acceptable derivative thereof, are prepared from compound of general formula VI. In particular, the compounds according to Formula I, II, III, IV and V can be prepared by reacting compound of formula VI with compound of formula VII according to scheme 1 in the presence of a carboxyl activating agent, a suitable base and a suitable solvent.

Scheme 1

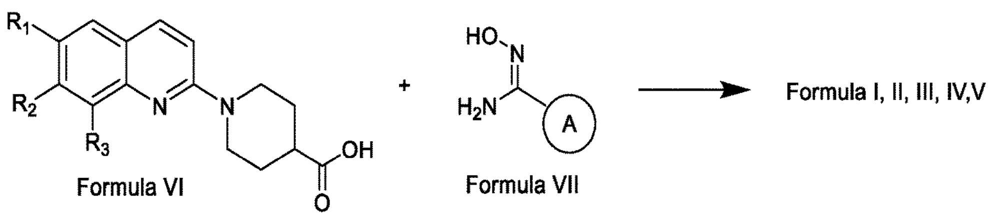

Formula VI

Formula VII

Formula I, II, III, IV,V

$R_1$, $R_2$, $R_3$ and ring A are as defined for compound of general formula I.

In an embodiment, the compound of formula VI is a carboxylic acid according to Formula I. The compound of formula VII is an amidoxime according to formula I.

In an embodiment, the carboxyl activating agent is an acid coupling agent selected from the following: Benzotriazol-1-ol (HOBt), 4-dimethylaminopyridine (DMAP), 1-hydroxy-7-aza-benzotriazole (HOAt), dicyclohexyl-carbodiimide (DCC), N,N'-Diisopropylcarbodiimide (DIC), 1,1'-carbonyldiimidazole (CDI), N-Cyclohexyl-N'-(2-morpholinoethyl)carbodiimide methyl-p-toluenesulfonate, 1,3-Di-p-tolylcarbodiimide, 1-ethyl-(3-(3-dimethylamino)propyl)-carbodiimide hydrochloride (EDCI), N-[(dimethylamino)-3-oxo-1H-1,2,3-triazolo[4,5-b]pyridin-1-yl-methylene]-N-methylmethanaminium hexafluorophosphate (HATU) or 2-(1H-Benzotriazole-1-yl)-1,1,3,3-tetramethyluronium hexafluorophosphate (HBTU), O--1,1,3,3-tetramethyluronium hexafluorophosphate (HCTU), more preferably the acid coupling agent is 1,1'-carbonyldiimidazole (CDI).

In an embodiment, the suitable base is an organic base or inorganic base. Suitable organic base comprises pyridine, triethylamine (TEA) and N-ethyl-N,N-diisopropylamine (DIPEA). Suitable inorganic base comprises sodium hydroxide, potassium hydroxide and sodium hydride. In a preferred embodiment, the base is selected from the group consisting of TEA and DIPEA. More preferably the base is N-ethyl-N,N-diisopropylamine (DIPEA).

In an embodiment, the suitable solvents comprise 1-butanol (1-BuOH), dimethylformamide (DMF), dimethylacetamide, 1,2-dimethoxyethane, dimethylsulfoxyde (DMSO), N-methylpyrrolidone, 1,4-dioxane, tetrahydrofuran (THF), benzene (Ph), toluene (PhMe), dichloromethane (DCM) or water ($H_2O$), and mixtures thereof. In a preferred embodiment, the solvent is toluene (PhMe).

In an embodiment the temperature is preferably from 60 °C to 160 °C, more preferably the temperature is 110 °C.

In an embodiment, compound of formula X is prepared by reacting compound of formula VIII with compound of formula IX, according to scheme 2, in the presence or absence of a base and in the presence or absence of a solvent:

Scheme 2

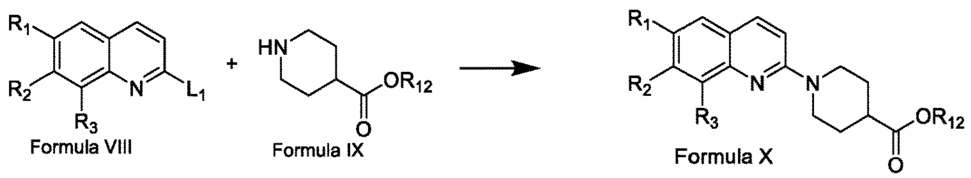


wherein $L_1$ is a leaving group by reaction with a compound of general formula IX,

preferably $L_1$ is a leaving group, preferably $L_1$ is a halogen selected from Br, Cl or F, more preferably, $L_1$ is Cl or Br; and

$R_1$, $R_2$ and $R_3$ are as defined for compound of general formula I;

$R_{12}$ is $CH_3$ or $CH_2CH_3$.

In an embodiment, compound of formula VIII and compound of formula IX are reacted together at elevated temperature in the absence of solvent (neat). The reaction is carried out in protic solvent such as ethanol, or in the presence of an aprotic solvent such as PhMe.

In an embodiment, the temperature is preferably from 80 °C to 150 °C, more preferably the temperature is 120 °C.

In an embodiment, compound of formula I is prepared by preferably reacting compound of formula VIII and compound of formula IX in the presence of a solvent such as 1-butanol and in the presence of an alkaline agent such as N,N-diisopropylethylamine in order to obtain compound of formula X.

In an embodiment, the temperature is preferably from 80 °C to 160 °C, more preferably the temperature is 130 °C.

In an embodiment, compound of formula I, II, III, IV and V are prepared by reacting compound of formula VIII with compound of formula XI, according to scheme 3, in the presence or absence of a base and in the presence or absence of a solvent:

Scheme 3

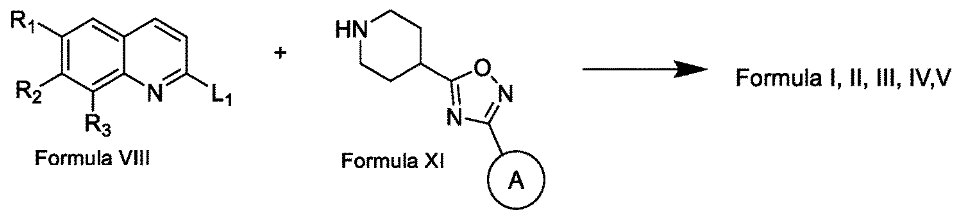


wherein $L_1$ is a leaving group by reaction with a compound of general formula XI,

preferably $L_1$ is a leaving group, preferably $L_1$ is a halogen selected from Br, Cl or F, more preferably, $L_1$ is Cl or Br; and

$R_1$, $R_2$, $R_3$ and Ring A are as defined for compound of general formula I.

In an embodiment, compound of formula VIII and compound of formula XI are reacted together at elevated temperature in the absence of solvent (neat). The reaction is carried out in protic solvent such as ethanol, or in the presence of an aprotic solvent such as PhMe.

In an embodiment, the temperature is preferably from 80 °C to 150 °C, more preferably the temperature is 120 °C.

In an embodiment, compound of formula I is prepared by preferably reacting compound of formula VIII and compound of formula XI in the presence of solvent such as 1-butanol and in the presence of an alkaline agent such as DIPEA in order to obtain compounds of formula I, II, III, IV or V.

In an embodiment, the temperature is preferably from 80 °C to 160 °C, more preferably the temperature is 130°C.

In an embodiment, compound of formula XIII is prepared by reacting compound of formula XII with compound of

formula VII, according to scheme 4, in the presence of a carboxyl activating agent, a suitable base and a suitable solvent:

Scheme 4

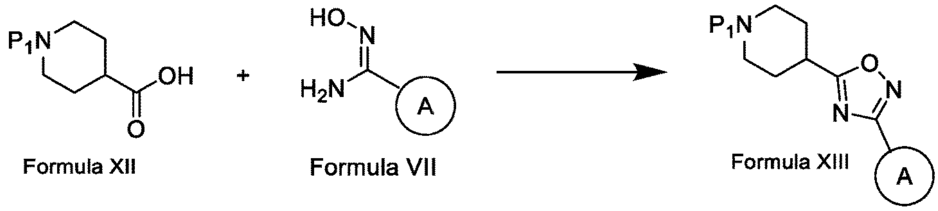

Formula XII Formula VII Formula XIII

$P_1$ is a amine protecting group according to Greener's Protecting Groups in Organic synthesis, Peter G. M. Wuts, Wiley, 2014. More preferably, the protecting group is tert-butylcarbonyl.

In an embodiment, the compound of formula XII is a carboxylic acid according to Formula I and the compound of formula VII is an amidoxime according to formula I.

In an embodiment, the carboxyl activating agent is an acid coupling agent selected from the following: Benzotriazol-1-ol (HOBt), 4-dimethylaminopyridine (DMAP), 1-hydroxy-7-aza-benzotriazole (HOAt), dicyclohexyl-carbodiimide (DCC), N,N'-Diisopropylcarbodiimide (DIC), 1,1'-carbonyldiimidazole (CDI), N-Cyclohexyl-N'-(2-morpholinoethyl)carbodiimide methyl-p-toluenesulfonate, 1,3-Di-p-tolylcarbodiimide, 1-ethyl-(3-(3-dimethylamino)propyl)-carbodiimide hydrochloride (EDCI), N-[(dimethylamino)-3-oxo-1H-1,2,3-triazolo[4,5-b]pyridin-1-yl-methylene]-N-methylmethanaminium hexafluorophosphate (HATU) or 2-(1H-Benzotriazole-1-yl)-1,1,3,3-tetramethyluronium hexafluorophosphate (HBTU), O-1,1,3,3-tetramethyluronium hexafluorophosphate (HCTU), more preferably the acid coupling agent is 1,1'-carbonyldiimidazole (CDI).

In an embodiment, the suitable base is an organic base or inorganic base. Suitable organic base comprises pyridine, TEA and N-ethyl-N,N-diisopropylamine (DIPEA). Suitable inorganic base comprises sodium hydroxide, potassium hydroxide and sodium hydride. In a preferred embodiment, the base is selected from the group comprising TEA and DIPEA. More preferably the base is N-ethyl-N,N-diisopropylamine (DIPEA).

In an embodiment, the suitable solvents comprise 1-BuOH, DMF, dimethylacetamide, 1,2-dimethoxyethane, DMSO, N-methylpyrrolidone, 1,4-dioxane, THF, Ph, PhMe, DCM or $H_2O$, and mixtures thereof. In a preferred embodiment, the solvent is PhMe.

In an embodiment, the temperature is preferably from 60 °C to 160 °C, more preferably, the temperature is 110 °C.

Compounds of general formula VIII are known and are commercially available or are prepared by methods known to those of skill in the art.

In an embodiment, for example, a compound of general formula VIII in which $L_1$ is Cl, is prepared from a compound of general formula XIV

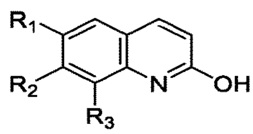

Formula XIV

by reacting with trichlorophosphate, phosphorous pentachloride, oxalyl chloride or thionyl chloride under heating in polar aprotic solvent such as DMF or acetonitrile (MeCN).

In an embodiment, compounds of general formula XIV are known and are commercially available or are prepared by methods known to those of skill in the art. For example, a compound of general formula XIV is prepared from a compound of general formula XV

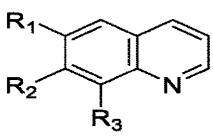

Formula XV

by reacting with dihydrogen peroxide under acidic conditions or 3-chloro-perbenzoic acid in halogenated solvents at room temperature to afford the respective N-oxide; followed by reaction with acetic anhydride under heating conditions to provide compound of formula XIV.

## DETAILED DESCRIPTION

The present disclosure relates to 5-(1-(quinolin-2-yl)piperidin-4-yl)-1,2,4-oxadiazole substituted with a 3-aryl or heteroaryl for use in the treatment and prevention of tuberculosis.

In an embodiment, the compounds of general formula I are prepared.

### Example 1

The compounds can generally be prepared by a succession of steps. The processes used for the synthesis of the compounds of the present disclosure are illustrated by a general scheme. Raw materials and reagents used to prepare compounds from the present disclosure are available from commercial suppliers or they can be prepared by methods known to those skilled in the art. The general schemes are merely illustrative of methods by which the compounds of this invention are synthesized, modifications to these schemes are suggested by those skilled in the art having referred to this disclosure.

In an embodiment, where the process refers to room temperature, the temperature is preferably from 20 °C to 25 °C, more preferably 20 °C.

In an embodiment, the compounds are characterized by their melting point, High Performance Liquid Chromatography Mass Spectrometry (HPLC-MS) and Nuclear Magnetic Resonance (NMR). HPLC-MS was performed on instrument HPLC Waters Alliance 2690 with detection performed by a Waters 2996 DAD detector (Diode Array Detector) and a QDA Waters mass spectrometer operating in electrospray (ES) ionization mode.

In an embodiment, MS data acquisition was performed with appropriate software. Compounds are described by their reported molecular ion which corresponds to the $[M+H]^+$ or $[M+2H]^{2+}$ (protonated molecules). For molecules with multiple isotropic patterns (Br, Cl...), the reported value is the one obtained for the lowest isotope mass. All results were obtained with experimental uncertainties that are commonly associated with the method used.

In an embodiment, the HPLC column used was a Sunfire C18, 5 μm (250 x 4.6 mm). The mobile phase was acetonitrile (A) and water with 0.1% formic acid (B) (75% A/25% B). The run time was 10 min, the flow rate was 1 mL/min and the temperature was 35 °C.

In an embodiment, melting point was recorded on a Buchi MP-M560 at 20 °C/min readings.

In an embodiment, 1H NMR spectra were recorded on a Bruker instrument operating at 400 or 500 MHz with solvent used as internal standard. Peak multiplicity abbreviations are s (singlet), d (doublet), t (triplet), m (multiplet), br (broad), dd (doublet of doublets), ddd (doublet of doublet of doublets), dtd (Doublet of triplet of doublets), ddt (Doublet of doublet of triplets), dt (doublet of triplets), td (triplet of doublets) and tt (Triplet of triplets). Coupling constant are reported in Hertz (Hz). 13C NMR spectra were recorded at 101 or 126 MHz.

In an embodiment, column chromatography was performed by flash chromatography on silica gel 60 (Merck), particle size 0.063-0.2 mm as stationary phase.

In an embodiment, purification of compounds via recrystallization was achieved by dissolving the crude compound in the minimum volume of a hot solvent of choice, generally a protic solvent, then covering the container and leaving it to cool down to room temperature and then at 0 °C gradually until crystal formation was observed.

In an embodiment, isolation of pure compound over column chromatography was achieved by collecting column fractions containing pure compound, evaporating the solvent in the rotavap and drying the residue under high vacuum at 40°C.

In an embodiment, the method of synthesizing Compound 1 (5-(1-(6-chloroquinolin-2-yl)piperidin-4-yl)-3-phenyl-1,2,4-oxadiazole) is as follows:

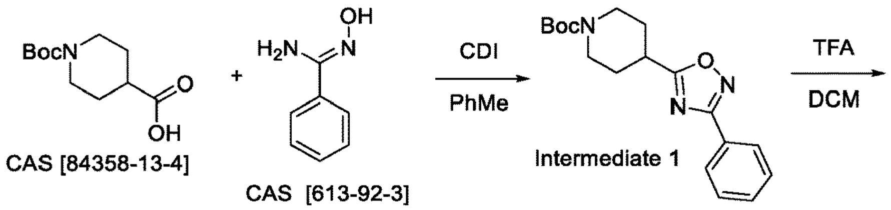


CAS [84358-13-4]

CAS [613-92-3]

Intermediate 1

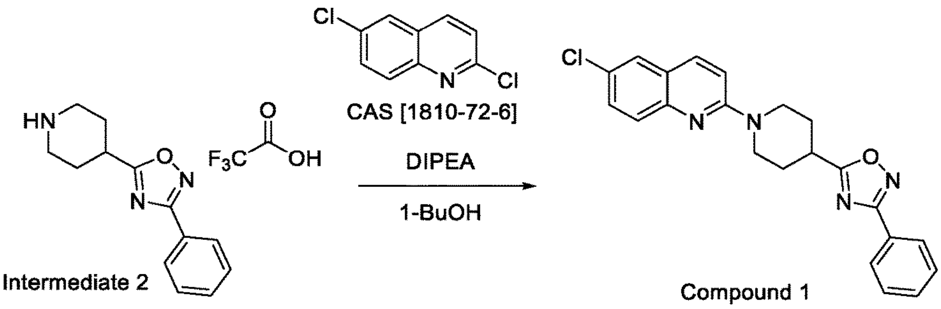


CAS [1810-72-6]

Intermediate 2

Compound 1

In an embodiment, preparation of intermediate 1 (tert-butyl 4-(3-phenyl-1,2,4-oxadiazol-5-yl)piperidine-1-carboxylate) was performed. Carbonyldiimidazole (CDI) (770 mg, 4.8 mmol) was added to a solution of 1-(tert-butoxycarbonyl)piperidine-4-carboxylic acid (1g, 4.3 mmol) in PhMe (10 ml) and the mixture was allowed to stir at room temperature for 2 hours. Benzamidoxime (650 mg, 4.8 mmol) was added and the resulting mixture was allowed to stir at room temperature and then at 110 °C over a duration of 1.5 hours. The solvent was evaporated, the resulting residue was diluted with ethyl acetate (EtOAc) and water, the organic layer was separated, dried over anhydrous sodium sulphate, filtered, evaporated in vacuum and the resulting residue was dried over high vacuum at 40 °C. The intermediate 1 was isolated as a colourless solid (900 mg, 62%).

In an embodiment, preparation of intermediate 2 (3-phenyl-5-(piperidin-4-yl)-1,2,4-oxadiazole) was performed. TFA (2.7 ml) was added to a solution of intermediate 1 (880 mg, 2.6 mmol) in DCM (6.8 ml). The mixture was allowed to stir at room temperature for 1hour. The solvent was removed in vacuum and the resulting residue was suspended and triturated in diethyl ether. The product was separated by filtration and was dried in high vacuum at 40 °C. The intermediate 2, was isolated as a colourless solid (850 mg, 70%).

In an embodiment, preparation of compound 1 (5-(1-(6-chloroquinolin-2-yl)piperidin-4-yl)-3-phenyl-1,2,4-oxadiazole) was performed. DIPEA (4.3 ml) was added to a suspension of 2,6-dichloroquinoline (243.6 mg, 1.23 mmol) and Intermediate 2 (400 mg, 1.23 mmol) in 1-BuOH (8.3 ml) and the mixture was allowed to stir at 130 °C over a duration of 20 hours. The solvent was evaporated, and the resulting residue was purified by column chromatography (silica gel, Petrol ether/EtOAc 4:1) in order to obtain compound 1 as an off-white solid (240 mg, 61%).

HPLC-MS $[M+H]^+$ 391.12; MP: 130-135 °C; $^1H$ NMR (400 MHz, $CDCl_3$) $\delta$ 8.12 - 8.04 (m, 2H), 7.81 (d, J = 9.2 Hz, 1H), 7.67 - 7.55 (m, 2H), 7.52-7.45 (m, 4H), 7.05 (d, J = 9.2 Hz, 1H), 4.55 (dt, J = 13.6, 4.0 Hz, 2H), 3.39 - 3.19 (m, 3H), 2.28 (dt, J = 12.4, 3.7 Hz, 2H), 2.14 - 1.98 (m, 2H); $^{13}C$ NMR (101 MHz, $CDCl_3$) $\delta$ 181.38, 168.28, 157.15, 146.35, 136.63, 131.16, 130.15, 128.84, 128.16, 127.54, 127.43, 126.81, 125.92, 12.55, 110.55, 44.65, 34.84, 29.05.

In an embodiment, the method of synthesizing Compound 2 (5-(1-(6-chloroquinolin-2-yl)piperidin-4-yl)-3-(4-(trifluoromethoxy)phenyl)-1,2,4-oxadiazole) is as follows:

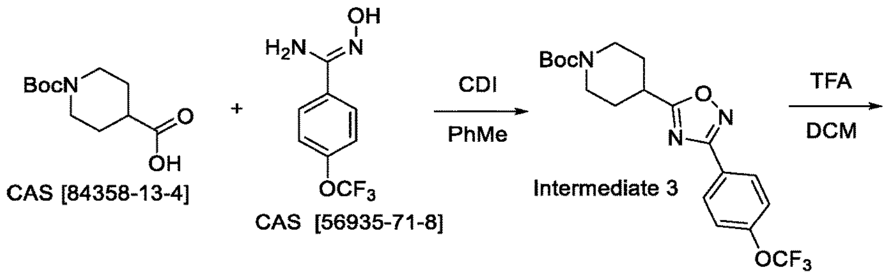


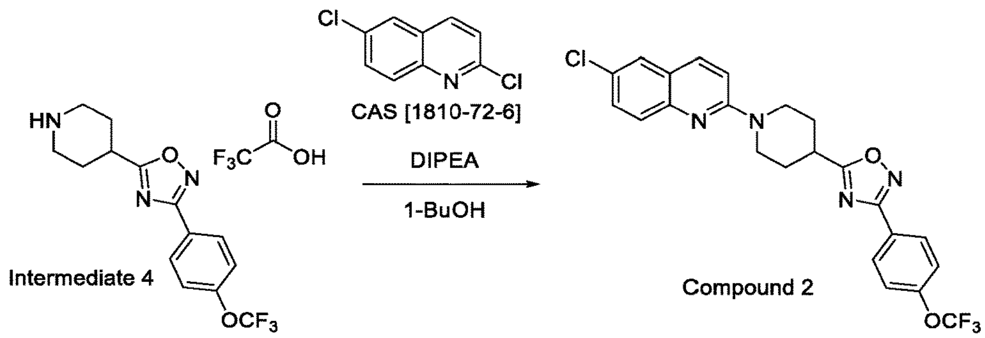


In an embodiment, preparation of intermediate 3 (tert-butyl 4-(3-phenyl-1,2,4-oxadiazol-5-yl)piperidine-1-carboxylate) was performed. Carbonyldiimidazole (CDI) (770 mg, 4.8 mmol) was added to a solution of 1-(tert-butoxycarbonyl)piperidine-4-carboxylic acid (1g, 4.3 mmol) in PhMe (10 ml) and the mixture was allowed to stir at room temperature for 2 hours. Benzamidoxime (650 mg, 4.8 mmol) was added and the resulting mixture was allowed to stir at room temperature for 1 hour and at 110 °C over a duration of 2 hours. The solvent was evaporated, the resulting residue was diluted with EtOAc and water, the organic layer was separated, dried over anhydrous sodium sulphate, filtered, evaporated in vacuum and the resulting residue was dried over high vacuum at 40 °C. The intermediate 3 was isolated as a brown solid (1 g, 57%).

In an embodiment, preparation of intermediate 4 (3-phenyl-5-(piperidin-4-yl)-1,2,4-oxadiazole trifluoroacetate) was performed. TFA (2.5 ml) was added to a solution of intermediate 3 (1 g, 2.4 mmol) in DCM (6.3 ml). The mixture was allowed to stir at room temperature for 1 hour. The solvent was removed in vacuum and the resulting residue was suspended and triturated in diethyl ether. The product was separated by filtration and was dried in high vacuum at 40 °C. The intermediate 4, was isolated as a colourless solid (920 mg, 90%).

In an embodiment, preparation of compound 2 (5-(1-(6-chloroquinolin-2-yl)piperidin-4-yl)-3-(4-(trifluoromethoxy)phenyl)-1,2,4-oxadiazole) was performed. DIPEA (2.3 ml) was added to a suspension of 2,6-dichloroquinoline (146 mg, 0.73 mmol) and Intermediate 4 (300 mg, 0.73 mmol) in 1-BuOH (4.4 ml) and the mixture was allowed to stir at 130 °C over a duration of 20 hours. The solvent was evaporated, and the resulting residue was purified by column chromatography (silica gel, Petrol ether/EtOAc 4:1) in order to obtain compound 2 as an off-white solid (190 mg, 55%).

HPLC-MS $[M+H]^+$ 475.06; MP: 122-126 °C; $^1H$ NMR (400 MHz, $CDCl_3$) δ 8.16 - 8.08 (m, 2H), 7.82 (d, J = 9.2 Hz, 1H), 7.67 - 7.55 (m, 2H), 7.47 (dd, J = 8.9, 2.4 Hz, 1H), 7.35 - 7.28 (m, 2H), 7.05 (d, J = 9.2 Hz, 1H), 4.55 (dt, J = 13.6, 3.9 Hz, 2H), 3.39 - 3.21 (m, 3H), 2.28 (dt, J = 12.5, 3.7 Hz, 2H), 2.14 - 1.99 (m, 2H); $^{13}C$ NMR (101 MHz, $CDCl_3$) δ 181.71, 167.26, 157.13, 151.24, 146.33, 136.65, 130.17, 129.16, 128.16, 127.59, 125.92, 125.43, 123.56, 121.63, 121.07, 110.53, 44.64, 34.83, 29.02.

In an embodiment, the method of synthesizing Compound 3 (5-(1-(8-methoxyquinolin-2-yl)piperidin-4-yl)-3-(4-(trifluoromethoxy)phenyl)-1,2,4-oxadiazole) is as follows:

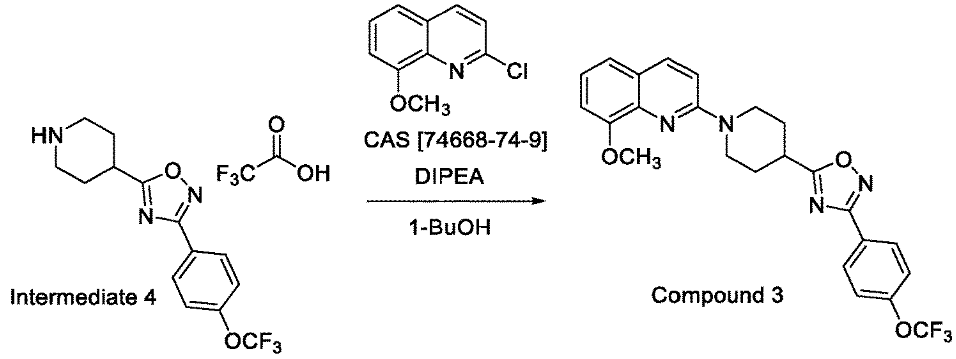

Intermediate 4
Compound 3

In an embodiment, preparation of compound 3 (5-(1-(8-Methoxyquinolin-2-yl)piperidin-4-yl)-3-(4-(trifluoromethoxy)phenyl)-1,2,4-oxadiazole) was performed. DIPEA (1.6 ml) was added to a suspension of 2-Chloro-8-methoxyquinoline (180 mg, 0.9 mmol) and intermediate 4 (300 mg, 0.9 mmol) in 1-BuOH (4.5 ml) and the mixture was allowed to stir at 130 °C for 48 hours. The solvent was evaporated and the resulting residue was purified by column chromatography (silica gel, Petrol ether/EtOAc 5:1). Compound 3 was isolated as an orange solid (260 mg, 55%).

HPLC-MS $[M+H]^+$471.17; MP: 124-125 °C; $^1H$ NMR (400 MHz, DMSO-$d_6$) δ 8.18 - 8.09 (m, 2H), 8.02 (d, $J$ = 9.2 Hz, 1H), 7.59 - 7.52 (m, 2H), 7.34 - 7.24 (m, 2H), 7.15 (t, $J$ = 7.8 Hz, 1H), 7.04 (dd, $J$ = 7.8, 1.4 Hz, 1H), 4.57 (dt, $J$ = 13.6, 3.9 Hz, 2H), 3.91 (s, 3H), 3.50 (tt, $J$ = 11.1, 3.9 Hz, 1H), 3.20 (ddd, $J$ = 13.8, 11.5, 2.7 Hz, 2H), 2.22 (dd, $J$ = 13.6, 3.6 Hz, 2H), 1.92 - 1.77 (m, 2H); $^{13}C$ NMR (101 MHz, DMSO-$d_6$) δ 182.89, 166.95, 156.50, 153.72, 150.81, 139.21, 137.93, 129.74, 125.88, 123.92, 122.40, 122.07, 121.69, 119.79, 110.70, 109.77, 56.12, 44.43, 34.34, 29.00.

In an embodiment, the method of synthesizing Compound 4 (5-(1-(8-chloroquinolin-2-yl)piperidin-4-yl)-3-(4-(trifluoromethoxy)phenyl)-1,2,4-oxadiazole) is as follows:

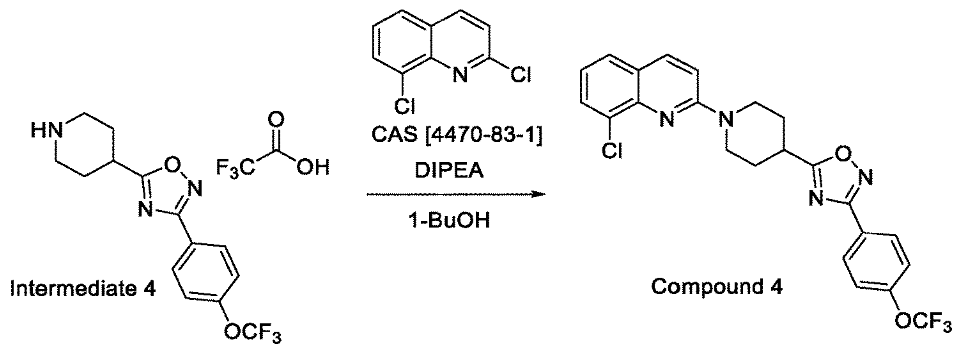

Intermediate 4
Compound 4

In an embodiment, preparation of compound 4 (5-(1-(8-chloroquinolin-2-yl)piperidin-4-yl)-3-(4-(trifluoromethoxy)phenyl)-1,2,4-oxadiazole) was performed. DIPEA (2 ml) was added to a suspension of 2,8-dichloroquinoline (146 mg, 0.74 mmol) and Intermediate 4 (300 mg, 0.74 mmol) in 1-BuOH (5 ml) and the mixture was allowed to stir at 130 °C over a duration of 48 hours. The solvent was evaporated, and the resulting residue was purified by column chromatography (silica gel, Petrol ether/EtOAc 7:1) in order to obtain compound 4 as a colourless solid (260 mg, 73%).

$[M+H]^+$ 475.13; MP: 109-110 °C; $^1H$ NMR (400 MHz, DMSO-$d_6$) δ 8.25 - 8.07 (m, 3H), 7.83 - 7.70 (m, 2H), 7.67 - 7.56 (m, 2H), 7.46 (d, $J$ = 9.2 Hz, 1H), 7.25 (t, $J$ = 7.7 Hz, 1H), 4.70 (dt, $J$ = 13.5, 3.9 Hz, 2H), 3.60 (tt, $J$ = 11.0, 3.9 Hz, 1H), 3.38 - 3.25 (m, 2H), 2.42 - 2.23 (m, 2H), 1.99 - 1.64 (m, 2H); $^{13}C$ NMR (101 MHz, DMSO-$d_6$) δ 182.77, 166.94, 157.31, 150.80, 143.68, 138.46, 129.81, 129.73, 129.38, 127.12, 125.87, 124.23, 122.33, 122.07, 119.13, 111.40, 44.19, 34.25, 28.95.

In an embodiment, the method of synthesizing Compound 5 (5-(1-(8-methoxyquinolin-2-yl)piperidin-4-yl)-3-(4-(trifluoromethyl)phenyl)-1,2,4-oxadiazole hydrochloride) is as follows:

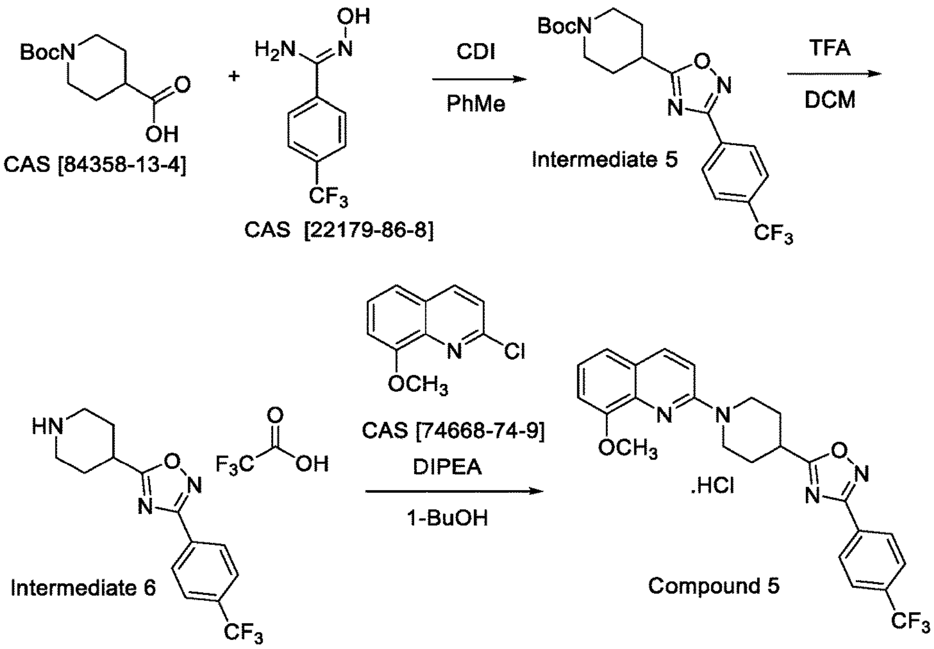


In an embodiment, preparation of intermediate 5 (*tert*-Butyl 4-(3-(4-(trifluoromethyl)phenyl)-1,2,4-oxadiazol-5-yl)piperidine-1-carboxylate) was performed. Carbonyldiimidazole (CDI) (779 mg, 4.8 mmol) was added to a solution of 1-(*tert*-butoxycarbonyl)piperidine-4-carboxylic acid (1g, 4.37 mmol) in PhMe (10 ml) and the mixture was allowed to stir at room temperature for 2 hours. 4-(trifluoromethyl)benzamidoxime (980 mg, 4.8 mmol) was added and the resulting mixture was allowed to stir at 110°C over a duration of 2.5 hours. The solvent was evaporated in high vacuum to give intermediate 5 as a clear oil (1.2 g). Intermediate 5 was taken crude to the next step.

In an embodiment, preparation of intermediate 6 (5-(Piperidin-4-yl)-3-(4-(trifluoromethyl)phenyl)-1,2,4-oxadiazole trifluoroacetate) was performed. TFA (3 ml) was added to a solution of Intermediate 5 (1.2 g, 3 mmol) in DCM (5 ml). The mixture was allowed to stir at room temperature for 1 hour. The solvent was evaporated in high vacuum to give intermediate 6 a colourless solid (1.33 g). Intermediate 6 was taken crude to the next step.

In an embodiment, preparation of compound 5 (5-(1-(8-Methoxyquinolin-2-yl)piperidin-4-yl)-3-(4-(trifluoromethyl)phenyl)-1,2,4-oxadiazole) was performed. DIPEA (5 ml) was added to a suspension of 2-chloro-8-methoxyquinoline (656 mg, 3.39 mmol) and Intermediate 6 (1.33 g, 3.39 mmol) in 1-BuOH (15 ml) and the mixture was allowed to stir at 130°C over a duration of 20 hours. The solvent was evaporated, and the resulting residue was purified by column chromatography (silica gel, Petrol ether/EtOAc 4:1). The product was dissolved in diethyl ether (5 mL) and a 2M solution of hydrochloric acid in diethyl ether (4 eq.) was added dropwise at 0 °C. The precipitate was separated by filtration and was washed with cold diethylether. Compound 5 was isolated as an off-white solid (90 mg, 5%).

HPLC-MS $[M+H]^+$ 455.17; MP: 110-113 °C; $^1H$ NMR (500 MHz, DMSO-$d_6$) $\delta$ 8.22 (d, $J$ = 8.1 Hz, 2H), 8.11 (d, $J$ = 9.2 Hz, 1H), 7.93 (d, $J$ = 8.1 Hz, 2H), 7.35 (dd, $J$ = 17.1, 8.6 Hz, 2H), 7.22 (t, $J$ = 7.8 Hz, 1H), 7.13 (d, $J$ = 7.9 Hz, 1H), 4.52 (dt, $J$ = 13.7, 4.0 Hz, 2H), 3.95 (s, 3H), 3.59 - 3.53 (m, 3H), 2.26 (dd, $J$ = 13.7, 3.9 Hz, 2H), 1.98 - 1.87 (m, 2H). $^{13}C$ NMR (126 MHz, DMSO-$d_6$) $\delta$ 182.98, 128.36, 126.67, 119.97, 56.48, 44.98, 34.08, 28.95

In an embodiment, the method of synthesizing compound 6 (3-(3,5-dimethoxyphenyl)-5-(1-(8-methoxyquinolin-2-yl)piperidin-4-yl)-1,2,4-oxadiazole) is as follows:

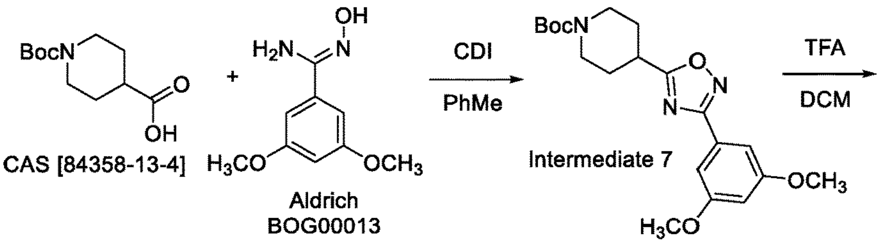


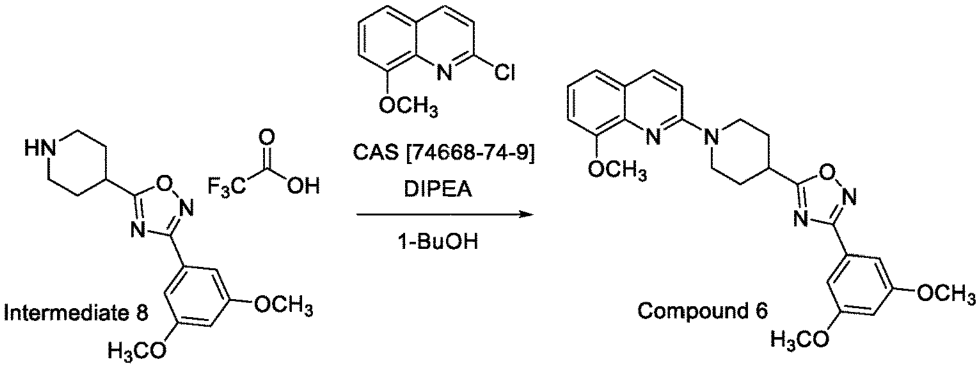


In an embodiment, preparation of intermediate 7 (*tert*-butyl 4-(3-(3,5-dimethoxyphenyl)-1,2,4-oxadiazol-5-yl) piperidine-1-carboxylate) was performed. Carbonyldiimidazole (CDI) (779 mg, 4.8 mmol) was added to a solution of 1-(tert-butoxycarbonyl)piperidine-4-carboxylic acid (1g, 4.37 mmol) in PhMe (10 ml) and the mixture was allowed to stir at room temperature for 2 hours. 3,5-Dimethoxy-benzamidoxime (980 mg, 4.8 mmol) was added and the resulting mixture was allowed to stir at 110 °C over a duration of 2 hours. The solvent was evaporated in high vacuum and the product was purified by column chromatography (silica gel, Petrol ether/EtOAc 4:1). Intermediate 7 was isolated as a colourless solid (1.19 g, 70%).

In an embodiment, preparation of intermediate 8 (3-(3,5-dimethoxyphenyl)-5-(piperidin-4-yl)-1,2,4-oxadiazole trifluoroacetate) was performed. TFA (3 ml) was added to a solution of Intermediate 7 (1.19 g, 3.06 mmol) in DCM (5 ml). The mixture was allowed to stir at room temperature for 1 hour. The solvent was evaporated in high vacuum to give intermediate 8 (1 g, 70%) as a clear oil. Intermediate 8 was taken crude to the next step.

In an embodiment, preparation of compound 6 (3-(3,5-dimethoxyphenyl)-5-(1-(8-methoxyquinolin-2-yl)piperidin-4-yl)-1,2,4-oxadiazole) was performed. DIPEA (5 ml) was added to a suspension of 2-chloro-8-methoxyquinoline (400 mg, 2.06 mmol) and Intermediate 8 (1.00 g, 2.48 mmol) in 1-BuOH (15 ml) and the mixture was allowed to stir at 130 °C over a duration of 20 hours. The solvent was evaporated, and the resulting residue was purified by column chromatography (silica gel, Petrol ether/EtOAc 4:1) in order to obtain compound 6 as an off-white solid (1.05 g, 95%).

HPLC-MS $[M+H]^+$ 447.19; MP: 124-127 °C; $^1H$ NMR (400 MHz, $CDCl_3$) δ 7.93 (d, *J* = 9.2 Hz, 1H), 7.31 - 7.14 (m, 4H), 7.08 (d, *J* = 9.2 Hz, 1H), 6.99 (dd, *J* = 7.6, 1.4 Hz, 1H), 6.62 (t, *J* = 2.4 Hz, 1H), 4.61 (dt, *J* = 13.1, 3.5 Hz, 2H), 4.06 (s, 3H), 3.88 (s, 6H), 3.38 - 3.22 (m, 3H), 2.32 dd, *J* = 13.5, 3.8 Hz, 2H), 2.15 - 2.07 (m 2H). $^{13}C$ NMR (101 MHz, $CDCl_3$) δ 181.50, 168.24, 161.05, 156.74, 153.53, 139.18, 137.72, 128.52, 123.97, 122.33, 119.45, 110.05, 108.78, 105.10, 104.01, 56.15, 55.59, 44.78, 34.91, 29.24.

In an embodiment, the method of synthesizing compound 7 (5-(1-(8-methoxyquinolin-2-yl)piperidin-4-yl)-3-(3-(trifluoromethyl)phenyl)-1,2,4-oxadiazole) is as follows:

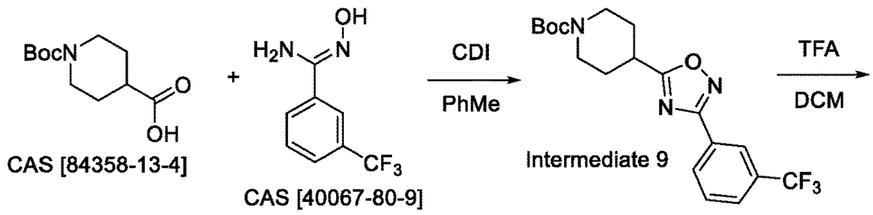


CAS [84358-13-4]
CAS [40067-80-9]
Intermediate 9

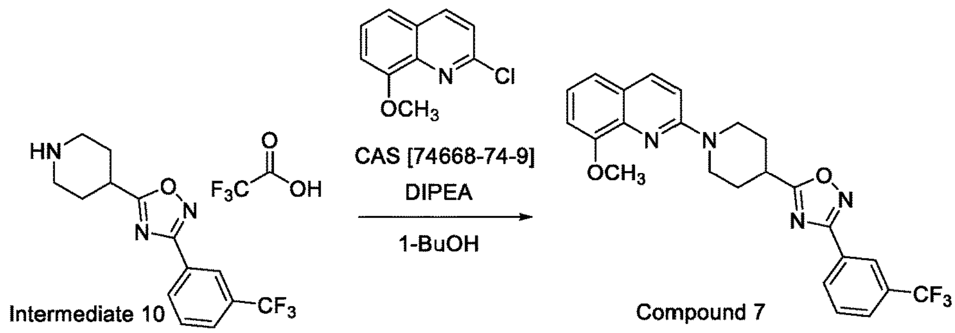


Intermediate 10
Compound 7

In an embodiment, preparation of intermediate 9 (*tert*-butyl 4-(3-(3-(trifluoromethyl)phenyl)-1,2,4-oxadiazol-5-yl) piperidine-1-carboxylate) was performed. Carbonyldiimidazole (CDI) (779 mg, 4.8 mmol) was added to a solution of 1-(*tert*-butoxycarbonyl)piperidine-4-carboxylic acid (1g, 4.37 mmol) in PhMe (10 ml) and the mixture was allowed to stir at room temperature for 2 hours. 3-(Trifluoromethyl)benzamidoxime (980 mg, 4.8 mmol) was added and the resulting mixture was allowed to stir at 110°C over a duration of 2 hours. The solvent was evaporated in high vacuum and the product was purified by column chromatography (silica gel, Petrol ether/EtOAc 4:1). Intermediate 9 was isolated as an oil (1.15 g, 66%).

In an embodiment, preparation of intermediate 10 (5-(piperidin-4-yl)-3-(3-(trifluoromethyl)phenyl)-1,2,4-oxadiazole trifluoroacetate) was performed. TFA (3 ml) was added to a solution of Intermediate 9 (1.15 g, 2.9 mmol) in DCM (5 ml). The mixture was allowed to stir at room temperature for 1 hour. The solvent was evaporated in high vacuum to give intermediate 10 (1 g, 84%) as a clear oil. Intermediate 10 was taken crude to the next step.

In an embodiment, preparation of compound 7 (5-(1-(8-methoxyquinolin-2-yl)piperidin-4-yl)-3-(3-(trifluoromethyl)phenyl)-1,2,4-oxadiazole) was performed. DIPEA (5 ml) was added to a suspension of 2-chloro-8-methoxyquinoline (393 mg, 2.03 mmol) and Intermediate 10 (1.00 g, 2.43 mmol) in 1-BuOH (15 ml) and the mixture was allowed to stir at 130 °C over a duration of 20 hours. The solvent was evaporated, and the resulting residue was purified by column chromatography (silica gel, Petrol ether/EtOAc 4:1) in order to obtain compound 7 as an off-white solid (968 mg, 88%).

HPLC-MS $[M+H]^+$ 455.17; MP: 100-105 °C; $^1$H NMR (400 MHz, $CDCl_3$) δ 8.38 (bs, 1H), 8.29 (d, *J* = 7.8 Hz, 1H), 7.94 (d, *J* = 9.1 Hz, 1H), 7.78 (d, *J* = 7.8 Hz, 1H), 7.63 (t, *J* = 7.8 Hz, 1H), 7.27 - 7.15 (m, 2H), 7.09 (d, *J* = 9.1 Hz, 1H), 7.00 (dd, *J* = 7.5, 1.4 Hz, 1H), 4.61 (dt, *J* = 13.7, 4.0 Hz, 2H), 4.05 (s, 3H), 3.31 (m, 3H), 2.34 (dt, *J* = 13.7, 3.6 Hz, 2H), 2.20 - 2.05 (m, 2H); $^{13}$C NMR (101 MHz, $CDCl_3$) δ 182.04, 171.57, 167.28, 156.63, 153.43, 137.89, 133.25, 130.55, 129.42, 127.78, 127.70, 124.44, 123.96, 122.48, 119.45, 111.98, 110.10, 108.89, 56.15, 44.84, 34.86, 29.20.

In an embodiment, the method of synthesizing compound 8 (3-(3,5-bis(trifluoromethyl)phenyl)-5-(1-(8-methoxyquinolin-2-yl)piperidin-4-yl)-1,2,4-oxadiazole) is as follows:

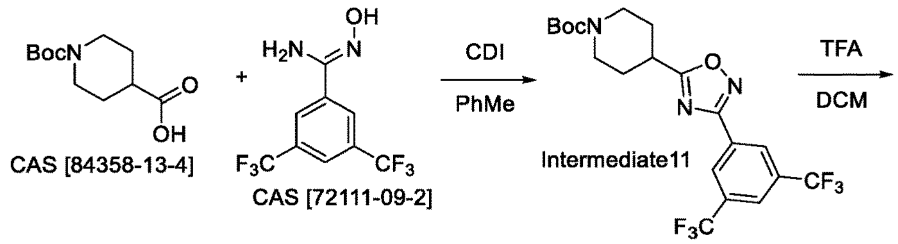


CAS [84358-13-4]
CAS [72111-09-2]
Intermediate11

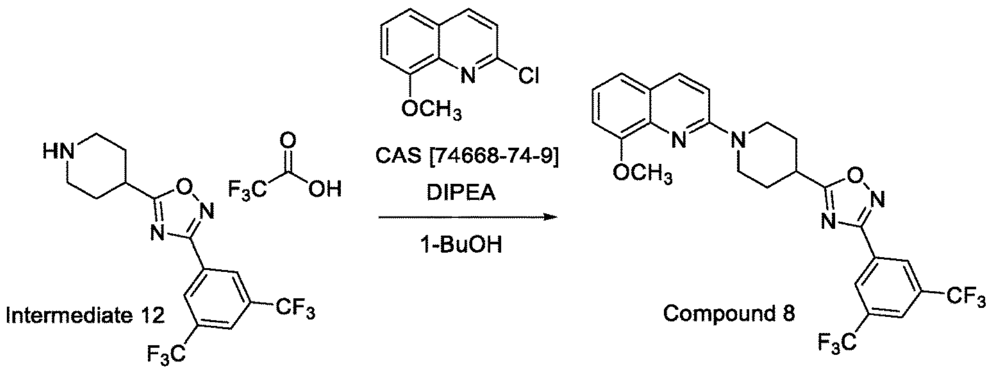


Intermediate 12
Compound 8

In an embodiment, preparation of intermediate 11 (*tert*-butyl 4-(3-(3,5-bis(trifluoromethyl)phenyl)-1,2,4-oxadiazol-5-yl)piperidine-1-carboxylate) was performed. Carbonyldiimidazole (CDI) (779 mg, 4.8 mmol) was added to a solution of 1-(*tert*-butoxycarbonyl)piperidine-4-carboxylic acid (1g, 4.37 mmol) in PhMe (10 ml) and the mixture was allowed to stir at room temperature for 2 hours. 3,5-Bis(trifluoromethyl)benzamidoxime (1.3 g, 4.8 mmol) was added and the resulting mixture was allowed to stir at 110 °C over a duration of 2 hours. The solvent was evaporated in high vacuum and the product was purified by column chromatography (silica gel, Petrol ether/EtOAc 4:1). Intermediate 11 was isolated as a clear oil (1.18 g, 59%).

In an embodiment, preparation of intermediate 12 (3-(3,5-bis(trifluoromethyl)phenyl)-5-(piperidin-4-yl)-1,2,4-oxadiazole trifluoroacetate) was performed. TFA (3 ml) was added to a solution of Intermediate 11 (1.18 g, 2.54 mmol) in DCM (5 ml). The mixture was allowed to stir at room temperature for 1 hour. The solvent was evaporated in high vacuum to give intermediate 12 (1 g, 70%) as a clear oil. Intermediate 12 was taken crude to the next step.

In an embodiment, preparation of compound 8 (3-(3,5-bis(trifluoromethyl)phenyl)-5-(1-(8-methoxyquinolin-2-yl) piperidin-4-yl)-1,2,4-oxadiazole) was performed. DIPEA (5 ml) was added to a suspension of 2-chloro-8-methoxyquinoline (440 mg, 2.28 mmol) and Intermediate 12 (1.00 g, 2.74 mmol) in 1-BuOH (15 ml) and the mixture was allowed to stir at 130 °C over a duration of 20 hours. The solvent was evaporated, and the resulting residue was purified by column chromatography (silica gel, Petrol ether/EtOAc 4:1) in order to obtain compound 8 as an off-white solid (397 mg, 50%).

HPLC-MS $[M+H]^+$ 523.13; MP: 121-125 °C; $^1$H NMR (400 MHz, $CDCl_3$) δ 8.58 (bs, 2H), 8.04 (d, *J* = 7.6 Hz, 1H), 7.94 (d, *J* = 9.2 Hz, 1H), 7.30 - 7.16 (m, 2H), 7.09 (d, *J* = 9.2 Hz, 1H), 7.00 (dd, *J* = 7.5, 1.5 Hz, 1H), 4.62 (dt, *J* = 13.6, 3.9 Hz, 2H), 4.06 (s, 3H), 3.43 - 3.25 (m, 3H), 2.34 (dt, *J* = 12.9, 4.0 Hz, 2H), 2.21 - 2.06 (m, 2H); $^{13}$C NMR (101 MHz, $CDCl_3$) δ 182.68, 166.29, 156.70, 153.55, 137.78, 132.66, 132.32, 129.13, 124.58, 124.29, 124.02, 122.44, 119.46, 116.73, 110.04, 108.80, 56.14, 44.75, 34.93, 29.17.

In an embodiment, the method of synthesizing compound 9 (5-(1-(8-methoxyquinolin-2-yl)piperidin-4-yl)-3-(pyridin-4-yl)-1,2,4-oxadiazole) is as follows:

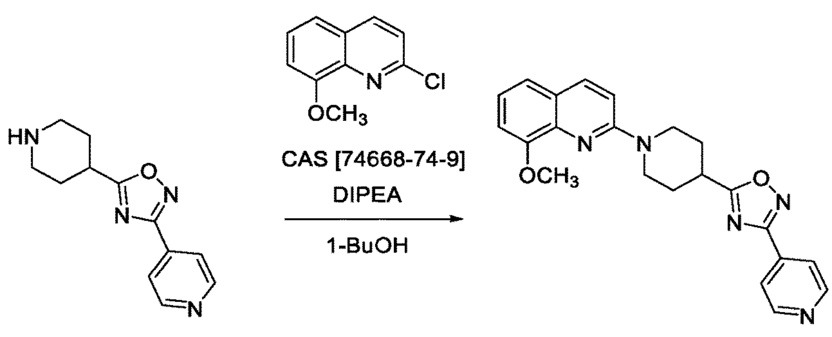


CAS [276237-03-7]
Compound 9

In an embodiment, preparation of compound 9 (5-(1-(8-methoxyquinolin-2-yl)piperidin-4-yl)-3-(pyridin-4-yl)-1,2,4-oxadiazole) was performed. DIPEA (5 ml) was added to a suspension of 2-chloro-8-methoxyquinoline (380 mg, 1.97 mmol) and 4-[5-(piperidin-4-yl)-1,2,4-oxadiazol-3-yl]pyridine (500 mg, 2.17 mmol) in 1-BuOH (15 ml) and the mixture was allowed to stir at 130 °C over a duration of 20 hours. The solvent was evaporated, and the resulting residue was purified by column chromatography (silica gel, DCM/MeOH 10%) in order to obtain compound 9 as a brown solid (250 mg, 34%).

HPLC-MS $[M+H]^+$ 388.21; MP: not aplicable (oil); $^1H$ NMR (400 MHz, MeOD-$d_4$) δ 9.09 (bs, 2H), 8.74 - 8.69 (m, 2H), 8.45 (d, *J* = 9.4 Hz, 1H), 7.66 (d, *J* = 9.2 Hz, 1H), 7.55 - 7.43 (m, 3H), 4.50 - 4.45 (m, 2H), 4.14 (s, 3H), 3.83 - 3.75 (m, 3H), 2.55 - 2.50 (m, 2H), 2.27 - 2.21 (m, 2H). $^{13}C$ NMR (101 MHz, MeOD-$d_4$) δ 143.75, 142.96, 126.07, 124.70, 119.59, 112.64, 55.83, 46.38, 32.97, 28.22.

In an embodiment, the method of synthesizing compound 10 (5-(1-(8-methoxyquinolin-2-yl)piperidin-4-yl)-3-(pyridin-3-yl)-1,2,4-oxadiazole hydrochloride) is as follows:

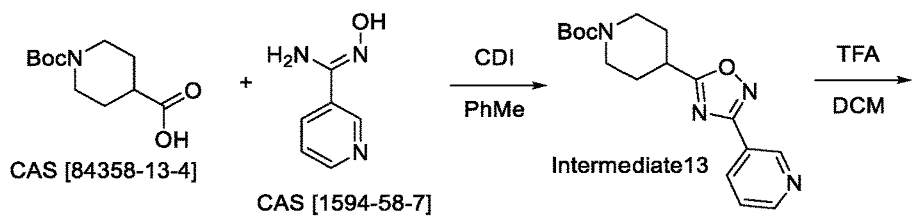


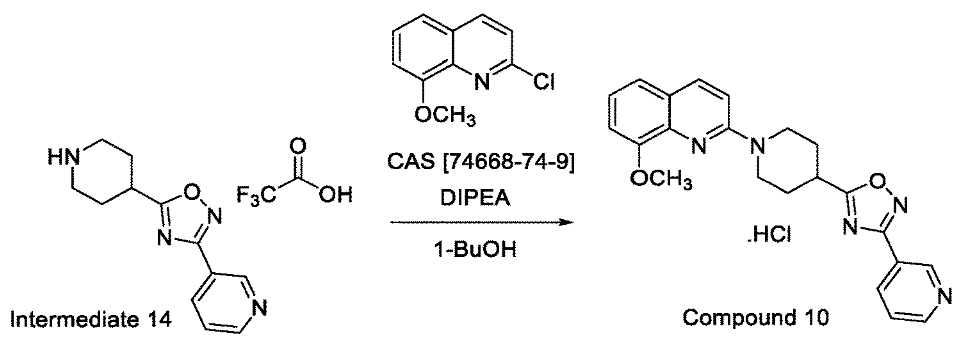


In an embodiment, preparation of intermediate 13 (*tert*-butyl 4-(3-(pyridin-3-yl)-1,2,4-oxadiazol-5-yl)piperidine-1-carboxylate) was performed. Carbonyldiimidazole (CDI) (779 mg, 4.8 mmol) was added to a solution of 1-(*tert*-butoxycarbonyl)piperidine-4-carboxylic acid (1g, 4.37 mmol) in PhMe (10 ml) and the mixture was allowed to stir at room temperature for 2 hours. (Z)-N'-hydroxynicotinimidamide (660 mg, 4.8 mmol) was added and the resulting mixture was allowed to stir at 110 °C over a duration of 2 hours. The solvent was evaporated in high vacuum and the product was purified by column chromatography (silica gel, EtOAc). Intermediate 13 was isolated as a clear oil (788 mg, 58%).

In an embodiment, preparation of intermediate 14 (5-(piperidin-4-yl)-3-(pyridin-3-yl)-1,2,4-oxadiazole trifluoroacetate) was performed. TFA (3 ml) was added to a solution of Intermediate 13 (787 mg, 2.39 mmol) in DCM (5 ml). The mixture was allowed to stir at room temperature for 1 hour. The solvent was evaporated in high vacuum to give intermediate 14 (1.78 g) as a clear oil. Intermediate 14 was taken crude to the next step.

In an embodiment, preparation of compound 10 (5-(1-(8-methoxyquinolin-2-yl)piperidin-4-yl)-3-(pyridin-3-yl)-1,2,4-oxadiazole hydrochloride) was performed. DIPEA (5 ml) was added to a suspension of 2-chloro-8-methoxyquinoline (500 mg, 2.58 mmol) and Intermediate 14 (900 mg, 2.84 mmol) in 1-BuOH (15 ml) and the mixture was allowed to stir at 130 °C over a duration of 20 hours. The solvent was evaporated, and the resulting residue was purified by column chromatography (silica gel, Petrol ether/EtOAc 3:1). The product was dissolved in EtOAc (5 mL) and a 2M solution of hydrochloric acid in diethyl ether (4 eq.) was added dropwise at 0 °C. The precipitate was separated by filtration and was washed with cold EtOAc. Compound 10 was isolated as a brown solid (128 mg, 13%).

HPLC-MS $[M+H]^+$ 388.22; MP: not aplicable (oil); $^1H$ NMR (400 MHz, MeOD-$d_4$) δ 9.54 (bs, 1H), 9.28 (dt, *J* = 8.3, 1.7 Hz, 1H), 9.13 - 9.06 (m, 1H), 8.48 (d, *J* = 9.8 Hz, 1H), 8.34 (ddd, *J* = 8.3, 5.8, 0.8 Hz, 1H), 7.67 (d, *J* = 9.7 Hz, 1H), 7.60 - 7.50 (m, 2H), 7.52 - 7.42 (m, 1H), 4.50 (dt, *J* = 14.2, 4.0 Hz, 2H), 4.17 (s, 3H), 3.85 - 3.74 (m 3H), 2.59 - 2.48 (m, 2H), 2.34 - 2.17 (m, 2H); $^{13}C$ NMR (101 MHz, MeOD-$d_4$) δ 182.70, 163.93, 151.63, 144.22, 143.73, 143.53, 140.37, 127.97, 127.30, 126.39, 126.05, 121.87, 119.58, 112.63, 112.55, 55.84, 32.92, 28.26.

In an embodiment, the method of synthesizing compound 11 (5-(1-(8-methoxyquinolin-2-yl)piperidin-4-yl)-3-(6-(trifluoromethyl)pyridin-3-yl)-1,2,4-oxadiazole) is as follows:

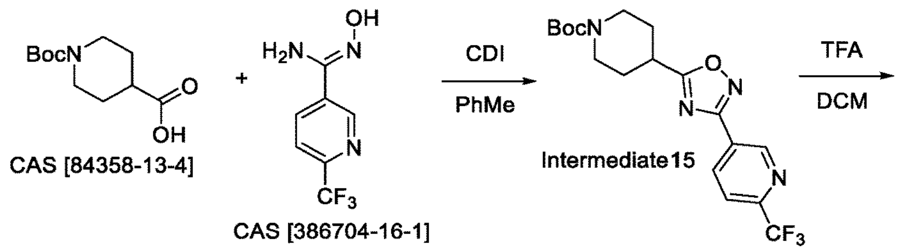


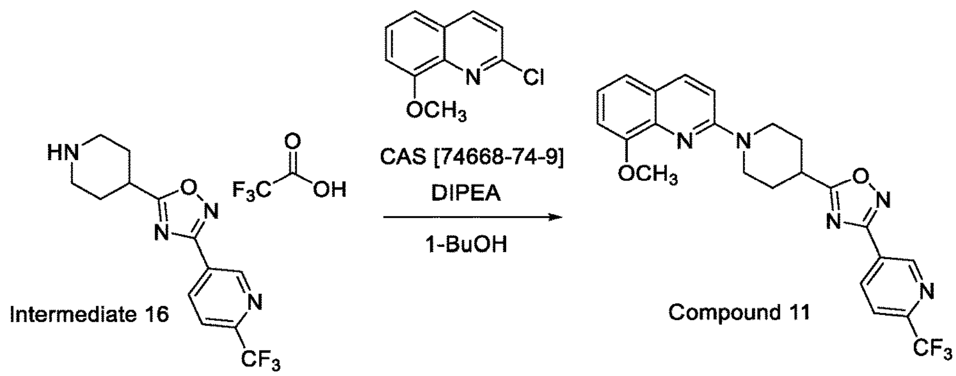


In an embodiment, preparation of intermediate 15 (*tert*-butyl 4-(3-(6-(trifluoromethyl)pyridin-3-yl)-1,2,4-oxadiazol-5-yl)piperidine-1-carboxylate) was performed. Carbonyldiimidazole (CDI) (779 mg, 4.8 mmol) was added to a solution of 1-(tert-butoxycarbonyl)piperidine-4-carboxylic acid (1g, 4.37 mmol) in PhMe (10 ml) and the mixture was allowed to stir at room temperature for 2 hours. 6-(Trifluoromethyl)nicotinamidoxime (1 g, 4.8 mmol) was added and the resulting mixture was allowed to stir at 110°C over a duration of 2 hours. The solvent was evaporated in high vacuum and the product was purified by column chromatography (silica gel, Petrol ether/EtOAc 4:1). Intermediate 15 was isolated as a clear oil (1.1 g, 68%).

In an embodiment, preparation of intermediate 16 (5-(piperidin-4-yl)-3-(6-(trifluoromethyl)pyridin-3-yl)-1,2,4-oxadiazole trifluoroacetate) was performed. TFA (3 ml) was added to a solution of Intermediate 15 (1.1 g, 2.77 mmol) in DCM (5 ml). The mixture was allowed to stir at room temperature for 1 hour. The solvent was evaporated in high vacuum to give intermediate 16 (1.09 g, 100%) as a clear oil. Intermediate 16 was taken crude to the next step.

In an embodiment, preparation of compound 11 (5-(1-(8-methoxyquinolin-2-yl)piperidin-4-yl)-3-(6-(trifluoromethyl)pyridin-3-yl)-1,2,4-oxadiazole was performed. DIPEA (10 ml) was added to a suspension of 2-chloro-8-methoxyquinoline (700 mg, 3.18 mmol) and Intermediate 16 (1.09 g, 2.65 mmol) in 1-BuOH (20 ml) and the mixture was allowed to stir at 130 °C over a duration of 20 hours. The solvent was evaporated, and the resulting residue was purified by column chromatography (silica gel, Petrol ether/EtOAc 4:1) in order to obtain compound 11 as a yellow solid (288 mg, 24%).

HPLC-MS $[M+H]^+$ 456.17; MP: 106-109 °C; $^1$H NMR (400 MHz, $CDCl_3$) δ 9.43 (bs, 1H), 8.56 (dd, *J* = 8.3, 2.0 Hz, 1H), 7.94 (d, *J* = 9.1 Hz, 1H), 7.83 (dd, *J* = 8.3, 0.8 Hz, 1H), 7.27 - 7.18 (m, 2H), 7.08 (d, *J* = 9.2 Hz, 1H), 7.00 (dd, *J* = 7.5, 1.5 Hz, 1H), 4.61 (dt, *J* = 13.6, 3.9 Hz, 2H), 4.05 (s, 3H), 3.42 - 3.25 (m, 3H), 2.34 (dt, *J* = 12.1, 3.6 Hz, 2H), 2.19 - 2.03 (m, 2H); $^{13}$C NMR (101 MHz, $CDCl_3$) δ 182.58, 165.41, 156.54, 153.38, 150.18, 149.83, 148.75, 138.76, 138.00, 136.18, 125.87, 123.96, 122.58, 120.54, 119.45, 110.09, 108.94, 56.14, 44.85, 34.85, 29.17.

In an embodiment, the method of synthesizing compound 12 (3-(3-methoxyphenyl)-5-(1-(8-methoxyquinolin-2-yl)piperidin-4-yl)-1,2,4-oxadiazole hydrochloride) is as follows:

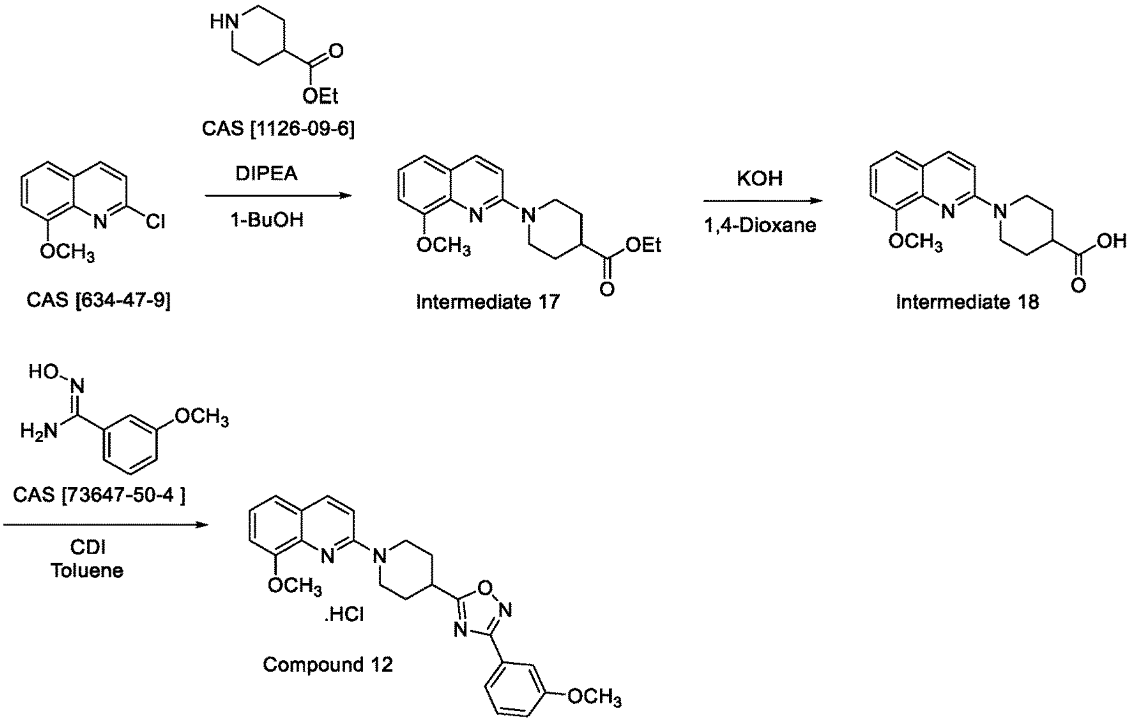


CAS [634-47-9]

Intermediate 17

Intermediate 18

Compound 12

In an embodiment, preparation of Intermediate 17 (Ethyl 1-(8-Methoxyquinolin-2-yl)piperidine-4-carboxylate) was performed. DIPEA (10 ml) was added to a suspension of 2-Chloro-8-methoxyquinoline (2 g, 10.3 mmol) and ethyl isonicotinate (1.78 g, 11.36 mmol) in 1-BuOH (20 ml) and the mixture was allowed to stir at 130°C for 20 hours. The solvent was evaporated and the resulting residue was purified by column chromatography (silica gel, Petrol ether/EtOAc 4:1) and Intermediate 17 was isolated as a clear oil (1.62 g, 51%).

In an embodiment, preparation of intermediate 18 (1-(8-Methoxyquinolin-2-yl)piperidine-4-carboxylic acid) was performed. A 5N aqueous solution of potassium hydroxide (10 ml) was added to a solution of Intermediate 17 (1.62 g, 5.16 mmol) in 1,4-dioxane (10 ml). The solution was allowed to stir at 60 °C over a duration of 5 hours. When the reaction was complete, water was added and the pH was reduced to pH2 with acetic acid. The product was extracted with EtOAc, the organic layer was separated, dried with anhydrous sodium sulphate, was filtered and evaporated in high vacuum to give Intermediate 18 as a clear oil (1.17 g, 80%).

In an embodiment, preparation of compound 12 (3-(3-methoxyphenyl)-5-(1-(8-methoxyquinolin-2-yl)piperidin-4-yl)-1,2,4-oxadiazole hydrochloride) was performed. CDI (273 mg, 1.68 mmol) was added to a solution of Intermediate 18 (438 mg, 1.53 mmol) in PhMe (5 mL) and the reaction mixture was allowed to stir at room temperature for 2 hours. Then 3-methoxy-benzamidoxime (279 mg, 1.68 mmol) was added and the reaction mixture was allowed to stir for 1 hour at room temperature and for 2 hours at 110 °C. The solvent was evaporated in high vacuum and the resulting residue was purified by column chromatography (silica gel, Petrol ether/EtOAc 4:1). The product was dissolved in EtOAc (5 mL) and a 2M solution of hydrochloric acid in diethyl ether (1 mL) was added dropwise at 0 °C. The precipitate was separated by filtration and was washed with cold diethyl ether. Compound 12 was isolated as a colourless solid (181 mg, 26%).

HPLC-MS $[M+H]^+$ 417.24; MP: 77-78 °C; $^1H$ NMR (500 MHz, MeOD-$d_4$) δ 8.43 (d, *J* = 9.7 Hz, 1H), 7.66 - 7.55 (m, 3H), 7.55 7.47 (m, 2H), 7.50 - 7.39 (m, 2H), 7.11 (ddd, *J* = 8.4, 2.7, 1.0 Hz, 1H), 4.44 (dt, *J* = 14.4, 4.1 Hz, 2H), 4.13 (s, 3H), 3.86 (s, 3H), 3.75 (ddd, *J* = 14.0, 11.1, 3.1 Hz, 2H), 3.64 (tt, *J* = 10.5, 4.2 Hz, 1H), 2.46 (dt, *J* = 12.8, 4.0 Hz, 2H), 2.26 - 2.17 (m, 2H); $^{13}C$ NMR (126 MHz, MeOD-$d_4$) δ 180.98, 168.06, 164.10, 160.11, 151.76, 147.62, 143.52, 129.88, 127.69, 125.93, 121.91, 119.58, 119.22, 116.84, 112.51, 112.41, 112.09, 55.82, 54.52, 46.36, 32.88, 28.37.

In an embodiment, the method of synthesizing compound 13 (5-(1-(8-methoxyquinolin-2-yl)piperidin-4-yl)-3-(3-(trifluoromethoxy)phenyl)-1,2,4-oxadiazole) is as follows:

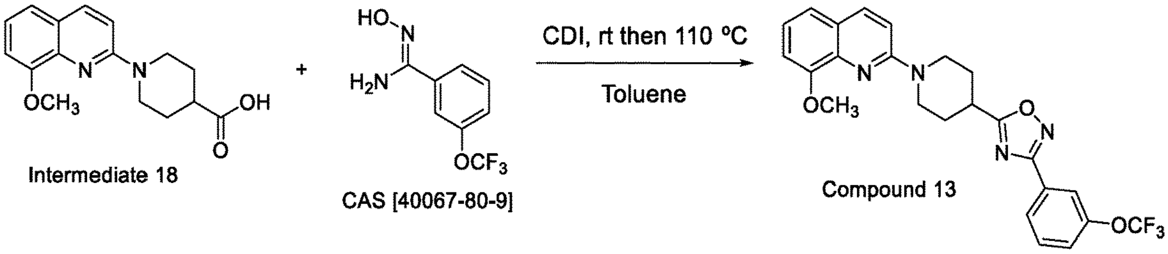


In an embodiment, preparation of compound 13 (5-(1-(8-methoxyquinolin-2-yl)piperidin-4-yl)-3-(3-(trifluoromethoxy)phenyl)-1,2,4-oxadiazole) was performed. CDI (311 mg, 2.63 mmol) was added to a solution of Intermediate 18 (500 mg, 1.75 mmol) in PhMe (10 mL) and the reaction mixture was allowed to stir at room temperature for 2 hours. Then N'-Hydroxy-3-(trifluoromethoxy)benzimidamide (423 mg, 2.63 mmol) was added and the reaction mixture was allowed to stir for 1 hour at room temperature and for 2 hours at 110 °C. The solvent was evaporated in high vacuum and the resulting residue was purified by column chromatography (silica gel, Petrol ether/EtOAc 4:1) in order to obtain compound 13 as an off-white solid (234 mg, 28%).

HPLC-MS $[M+H]^+$ 471.17; MP: 140-143 °C; $^1H$ NMR (400 MHz, MeOD-$d_4$) δ 8.45 (d, *J* = 9.7 Hz, 1H), 8.08 (dt, *J* = 7.8, 1.2 Hz, 1H), 7.94 (bs, 1H), 7.69 - 7.59 (m, 2H), 7.56 - 7.39 (m, 4H), 4.45 (dt, *J* = 14.2, 4.2 Hz, 2H), 4.14 (s, 3H), 3.78 (ddd, *J* = 14.0, 11.0, 3.1 Hz, 2H), 3.67 (tt, *J* = 10.4, 4.3 Hz, 1H), 2.54 - 2.44 (m, 2H), 2.30 - 2.15 (m, 2H); $^{13}C$ NMR (101 MHz, MeOD-$d_4$) δ 181.42, 174.23, 167.02, 151.61, 149.47, 147.44, 143.67, 130.76, 128.81, 126.39, 126.02, 125.60, 123.52, 121.86, 119.57, 119.30, 112.60, 112.52, 55.82, 46.39, 32.81, 28.31.

In an embodiment, the method of synthesizing compound 14 (3-(4-methoxyphenyl)-5-(1-(8-methoxyquinolin-2-yl)piperidin-4-yl)-1,2,4-oxadiazole) is as follows:

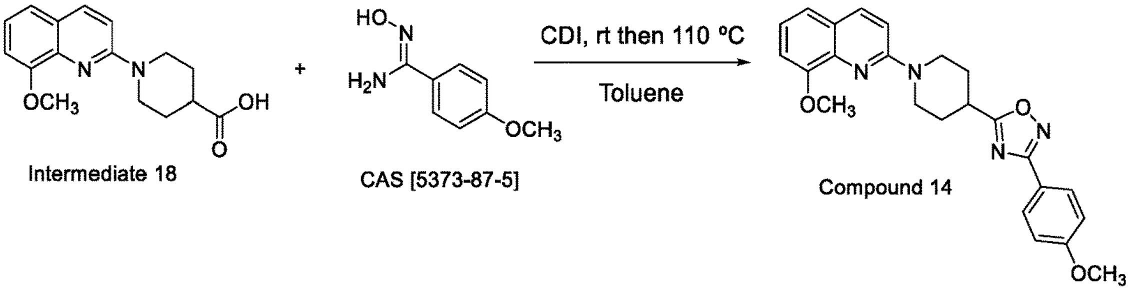


In an embodiment, preparation of compound 14 (3-(4-methoxyphenyl)-5-(1-(8-methoxyquinolin-2-yl)piperidin-4-yl)-1,2,4-oxadiazole) was performed. CDI (311 mg, 2.53 mmol) was added to a solution of Intermediate 18 (500 mg, 1.75 mmol) in PhMe (10 mL) and the reaction mixture was allowed to stir at room temperature for 2 hours. Then N'-Hydroxy-4-methoxybenzimidamide (329 mg, 2.53 mmol) was added and the reaction mixture was allowed to stir for 1 hour at room temperature and 2 hours at 110 °C. The solvent was evaporated in high vacuum and the resulting residue was purified by column chromatography (silica gel, Petrol ether/EtOAc 4:1) in order to obtain compound 14 as an off-white solid (378 mg, 52%).

HPLC-MS $[M+H]^+$ 417.25; MP: 90-100 °C; $^1H$ NMR (500 MHz, $CDCl_3$) δ 8.06 - 7.99 (m, 2H), 7.92 (d, *J = 9.2* Hz, 1H), 7.24 (dd, *J* = 8.1, 1.4 Hz, 1H), 7.19 (t, *J* = 7.8 Hz, 1H), 7.07 (d, *J* = 9.1 Hz, 1H), 7.02 - 6.95 (m, 3H), 4.58 (dt, *J* = 13.6, 4.1 Hz, 2H), 4.05 (s, 3H), 3.87 (s, 3H), 3.36 - 3.22 (m, 3H), 2.31 (dt, *J* = 12.1, 3.7 Hz, 2H), 2.16 - 2.03 (m, 2H); $^{13}C$ NMR (126 MHz, $CDCl_3$) δ 181.14, 167.97, 161.88, 156.52, 138.05, 129.04, 123.87, 122.56, 119.44, 119.33, 114.23, 110.19, 109.02, 60.40, 56.19, 55.38, 45.02, 34.76, 29.24, 14.21.

In an embodiment, the method of synthesizing compound 15 (5-(1-(6-chloroquinolin-2-yl)piperidin-4-yl)-3-(pyridin-3-yl)-1,2,4-oxadiazole hydrochloride) is as follows:

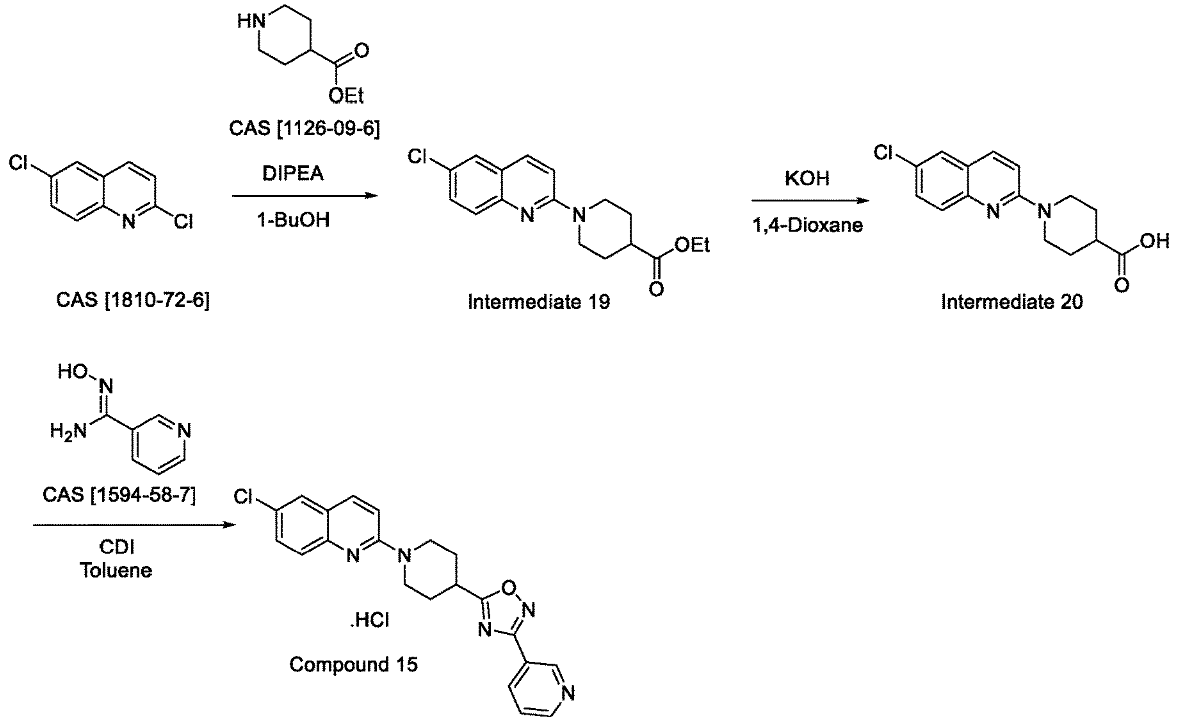


CAS [1810-72-6]

Intermediate 19

Intermediate 20

.HCl

Compound 15

In an embodiment, preparation of Intermediate 19 (ethyl 1-(6-chloroquinolin-2-yl)piperidine-4-carboxylate) was performed. DIPEA (5 ml) was added to a suspension of 2,6-dichloroquinoline (1 g, 5.1 mmol) and ethyl isonicotinate (873 mg, 5.6 mmol) in 1-BuOH (20 ml) and the mixture was allowed to stir at 130 °C for a duration of 20 hours. The solvent was evaporated and the resulting residue was purified by column chromatography (silica gel, Petrol ether/EtOAc 4:1) and Intermediate 19 was isolated as a clear oil (1.1 g, 68%).

In an embodiment, preparation of intermediate 20 (1-(6-chloroquinolin-2-yl)piperidine-4-carboxylic acid) was performed. A 5N aqueous solution of potassium hydroxide (20 ml) was added to a solution of Intermediate 19 (1.1 g, 3.46 mmol) in 1,4-dioxane (20 ml). The solution was allowed to stir at 80 °C over a duration of 2 hours. Some of the solvent was evaporated in vacuum and the precipitated starting material was removed by filtration. Water was added and the mixture was acidified to pH 1 with hydrochloric acid at 0 °C . The precipitate was separated by filtration and was washed with water. The intermediate 20 was isolated as a colourless solid (700 mg, 70%).

In an embodiment, preparation of compound 15 (5-(1-(6-chloroquinolin-2-yl)piperidin-4-yl)-3-(pyridin-3-yl)-1,2,4-oxadiazole hydrochloride) was performed. CDI (326 mg, 2.01 mmol) was added to a solution of Intermediate 20 (390 mg, 1.34 mmol) in PhMe (10 mL) and the reaction mixture was allowed to stir at room temperature for 2 hours. Then 3-pyridylamidoxime (276 mg, 2.01 mmol) was added and the reaction mixture was allowed to stir for 1 hour at room temperature and for 20 hours at 110 °C. The solvent was evaporated in high vacuum and the resulting residue was purified by column chromatography (silica gel, Petrol ether/EtOAc 4:1). The product was dissolved in EtOAc (5 mL) and a 2M solution of hydrochloric acid in diethyl ether (1 mL) was added dropwise at 0 °C . The precipitate was separated by filtration and was washed with cold diethyl ether. Compound 15 was isolated as pale yellow solid (420 mg, 80%).

HPLC-MS $[M+H]^+$ 392.19; MP: 271-274 °C; $^1H$ NMR (400 MHz, DMSO-$d_6$) δ 9.23 (bs, 1H), 8.88 (dd, *J* = 5.1, 1.6 Hz, 1H), 8.56 (m, 2H), 8.40 (d, *J* = 9.8 Hz, 1H), 8.07 (d, *J* = 2.4 Hz, 1H), 7.88 - 7.76 (m, 2H), 7.74 (d, *J* = 9.8 Hz, 1H), 4.77 - 4.68 (m, 2H), 3.80 - 3.66 (m, 3H), 2.34 (dt, *J* = 11.9, 3.7 Hz, 2H), 2.15 - 2.00 (m, 2H); $^{13}C$ NMR (101 MHz, DMSO-$d_6$) δ 182.47, 165.71, 151.92, 150.51, 146.07, 141.83, 137.56, 136.48, 132.58, 129.47, 127.54, 125.88, 123.83, 122.42, 120.82, 114.54, 47.17, 33.09, 28.94.

In an embodiment, the method of synthesizing compound 16 (5-(1-(8-methoxyquinolin-2-yl)piperidin-4-yl)-3-(pyrazin-2-yl)-1,2,4-oxadiazole hydrochloride) is as follows:

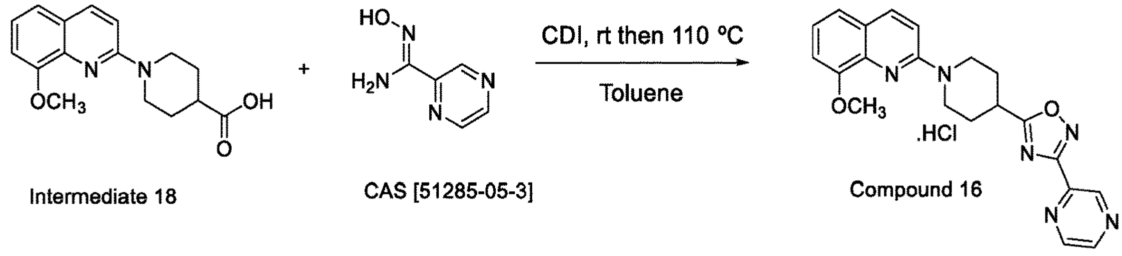


Intermediate 18 CAS [51285-05-3] Compound 16

In an embodiment, the method of synthesizing compound 16 (5-(1-(8-methoxyquinolin-2-yl)piperidin-4-yl)-3-(pyrazin-2-yl)-1,2,4-oxadiazole hydrochloride) was performed. CDI (426 mg, 2.63 mmol) was added to a solution of Intermediate 18 (500 mg, 1.75 mmol) in PhMe (10 mL) and the reaction mixture was allowed to stir at room temperature for 2 hours. Then N'-hydroxypyrazine-2-carboximidamide (363 mg, 2.63 mmol) was added and the reaction mixture was allowed to stir for 1 hour at room temperature and for 17 hours at 110 °C. The solvent was evaporated in high vacuum and the resulting residue was purified by column chromatography (silica gel, Petrol ether/EtOAc 4:1). The product was dissolved in EtOAc (5 mL) and a 2M solution of hydrochloric acid in diethyl ether (1 mL) was added dropwise at 0 °C. The precipitate was separated by filtration and was washed with cold diethyl ether. Compound 16 was isolated as pale yellow solid (212 mg, 29%).

HPLC-MS $[M+H]^+$ 389.22; MP: 125-129 °C; $^1H$ NMR (400 MHz, DMSO-$d_6$) δ 9.26 (bs, 1H), 8.91 - 8.84 (m, 2H), 8.41 (d, $J$ = 9.6 Hz, 1H), 7.66 (d, $J$ = 9.7 Hz, 1H), 7.50 (dd, $J$ = 7.7, 1.5 Hz, 1H), 7.48 - 7.35 (m, 2H), 4.45 (dt, $J$ = 13.8, 3.9 Hz, 2H), 4.07 - 4.02 (m, 5H), 3.76 - 3.62 (m, 1H), 2.34 (dd, $J$ = 13.9, 3.8 Hz, 2H), 2.12 - 1.97 (m, 2H); $^{13}C$ NMR (101 MHz, DMSO-$d_6$) δ 182.72, 166.41, 147.58, 145.72, 144.22, 142.08, 122.48, 120.21, 56.88, 46.69, 33.16, 28.86.

In an embodiment, the method of synthesizing compound 17 (7-hydroxy-8-methoxy-2-(4-(3-(pyridin-3-yl)-1,2,4-oxadiazol-5-yl)piperidin-1-yl)quinoline 1-oxide) is as follows:

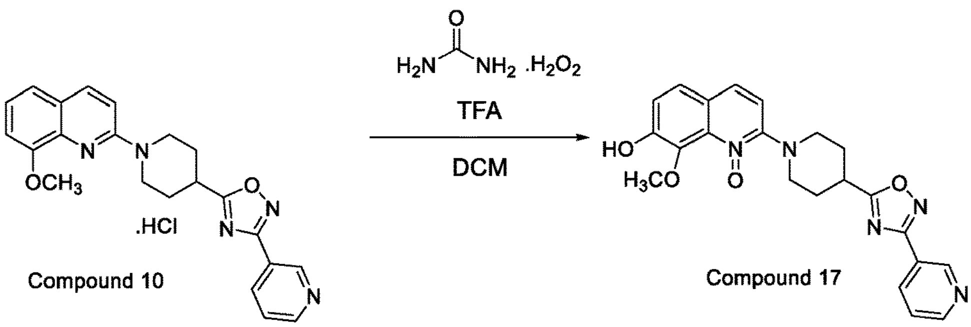


Compound 10 Compound 17

In an embodiment, preparation of compound 17 (7-hydroxy-8-methoxy-2-(4-(3-(pyridin-3-yl)-1,2,4-oxadiazol-5-yl)piperidin-1-yl)quinoline 1-oxide) was performed. Urea hydrogen peroxide (304 mg, 3.23 mmol) was added to a solution of Compound 10 (270 mg, 0.70 mmol) in DCM (10 mL). The reaction mixture was cooled down to 0 °C and trifluoroacetic anhydride (0.4 mL, 2.6 mmol) was added dropwise. The mixture was allowed to stir at room temperature until completion. Fresh hydrogen peroxide and trifluoroacetic anhydride were added once a day for 6 days. The reaction mixture was diluted with DCM and was quenched with a saturated aqueous solution of sodium thiosulfate. The organic layer was separated, re-washed with a saturated solution of sodium thiosulfate, water followed by brine. The organic layer was dried over anhydrous sodium sulphate and evaporated under reduced pressure. Compound 17 was isolated as a brown solid (108 mg, 37%).

HPLC-MS $[M+H]^+$ 422.16; MP: 75-80 °C; $^1H$ NMR (500 MHz, DMSO-$d_6$) δ 9.16 (bs, 1H), 8.81 - 8.75 (m, 1H), 8.37 (dt, $J$ = 8.0, 2.0 Hz, 1H), 8.18 (d, $J$ = 9.4 Hz, 1H), 7.62 (dd, $J$ = 8.0, 4.9 Hz, 1H), 7.44 (d, $J$ = 9.4 Hz, 1H), 7.26 (d, $J$ = 8.3 Hz, 1H), 7.03 (d, $J$ = 8.4 Hz, 1H), 4.57 (dt, $J$ = 13.7, 4.2 Hz, 2H), 3.90 (s, 3H), 3.34 - 3.21 m, 3H), 2.23 (dt, $J$ = 12.7, 3.9 Hz, 2H), 1.92 - 1.78 (m, 2H); $^{13}C$ NMR (126 MHz, DMSO-$d_6$) δ 182.91, 166.14, 156.43, 152.80, 152.44, 147.87, 135.44, 134.40, 124.94, 123.12, 122.18, 121.40, 120.64, 111.82, 110.00, 56.39, 44.38, 34.20, 28.98.

In an embodiment, the method of synthesizing compound 18 (5-(1-(8-chloroquinolin-2-yl)piperidin-4-yl)-3-(pyridin-3-yl)-1,2,4-oxadiazole hydrochloride) is as follows:

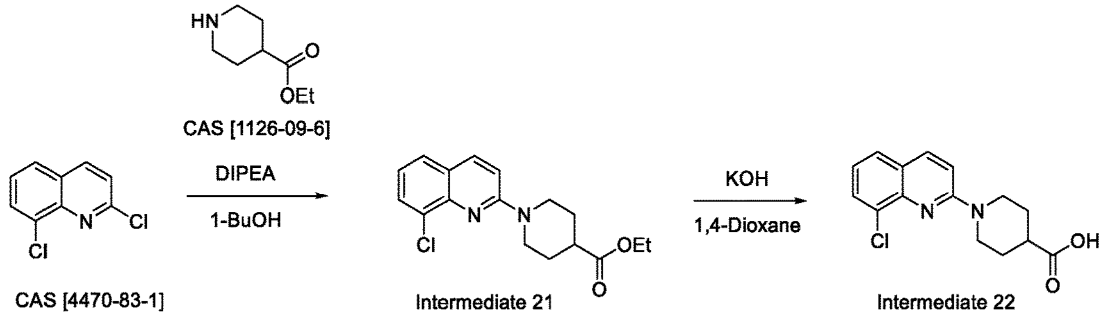

CAS [1126-09-6]
CAS [4470-83-1]
Intermediate 21
Intermediate 22

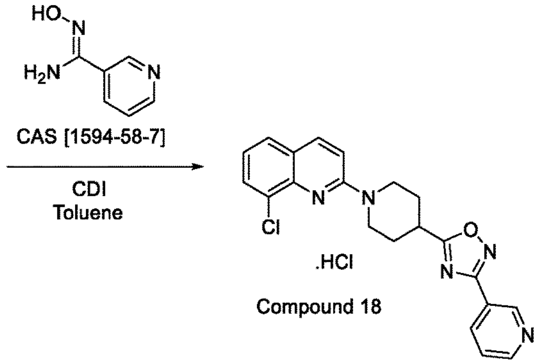

CAS [1594-58-7]
Compound 18

In an embodiment, preparation of Intermediate 21 (ethyl 1-(8-chloroquinolin-2-yl)piperidine-4-carboxylate) was performed. DIPEA (10 ml) was added to a suspension of 2,8-dichloroquinoline (2 g, 10 mmol) and ethyl isonicotinate (1.7 g, 11 mmol) in 1-BuOH (40 ml) and the mixture was allowed to stir at 130 °C for a duration of 20 hours. The solvent was evaporated and the resulting residue was purified by column chromatography (silica gel, Petrol ether/EtOAc 4:1) and Intermediate 21 was isolated as a clear oil (1.8 g, 57%).

In an embodiment, preparation of intermediate 22 (1-(8-chloroquinolin-2-yl)piperidine-4-carboxylic acid) was performed. A 5N aqueous solution of potassium hydroxide (20 ml) was added to a solution of Intermediate 21 (1.8 g, 5.65 mmol) in 1,4-dioxane (20 ml). The solution was allowed to stir at 80°C over a duration of 3 hours. The solvent was evaporated in vacuum and the mixture was acidified to pH 1 with hydrochloric acid at 0 °C. The precipitate was separated by filtration and was washed with water. The intermediate 22 was isolated as a yellow solid (1.9 g).

In an embodiment, preparation of compound 18 (5-(1-(8-chloroquinolin-2-yl)piperidin-4-yl)-3-(pyridin-3-yl)-1,2,4-oxadiazole hydrochloride) was performed. CDI (470 mg, 2.90 mmol) was added to a solution of Intermediate 22 (560 mg, 1.93 mmol) in PhMe (10 mL) and the reaction mixture was allowed to stir at room temperature for 2 hours. Then N'-Hydroxynicotinimidamide (397 mg, 2.90 mmol) was added and the reaction mixture was allowed to stir for 1 hour at room temperature and 23 hours at 110 °C. The solvent was evaporated in high vacuum and the resulting residue was purified by column chromatography (silica gel, Petrol ether/EtOAc 1:1). The product was dissolved in EtOAc (5 mL) and a 2M solution of hydrochloric acid in diethyl ether (1 mL) was added dropwise at 0 °C. The precipitate was separated by filtration and was washed with cold diethyl ether. Compound 18 was isolated as pale yellow solid (504 mg, 62%).

HPLC-MS $[M+H]^+$ 392.22; MP: 223-228 °C; $^1H$ NMR (400 MHz, DMSO-$d_6$) $\delta$ 9.24 (bs, 1H), 8.91 - 8.84 (m, 1H), 8.57 (dt, $J$ = 8.0, 1.9 Hz, 1H), 8.13 (d, $J$ = 9.2 Hz, 1H), 7.79 (dd, $J$ = 8.0, 5.0 Hz, 1H), 7.75 - 7.67 (m, 2H), 7.41 (d, $J$ = 9.2 Hz, 1H), 7.20 (t, $J$ = 7.7 Hz, 1H), 4.65 (dt, $J$ = 13.6, 3.8 Hz, 2H), 3.59 (tt, $J$ = 11.0, 4.0 Hz, 1H), 3.32 (ddd, $J$ = 13.8, 11.4, 2.7 Hz, 2H), 2.25 (dd, $J$ = 13.5, 3.7 Hz, 2H), 1.96 - 1.81 (m, 2H); $^{13}C$ NMR (101 MHz, DMSO-$d_6$) $\delta$ 183.12, 165.67, 157.23, 150.41, 146.06, 143.50, 138.56, 137.65, 129.88, 129.24, 127.15, 125.85, 124.22, 123.92, 122.41, 111.46, 44.23, 34.23, 28.92.

In an embodiment, the method of synthesizing compound 19 (5-(1-(8-chloroquinolin-2-yl)piperidin-4-yl)-3-(4-fluorophenyl)-1,2,4-oxadiazole) is as follows:

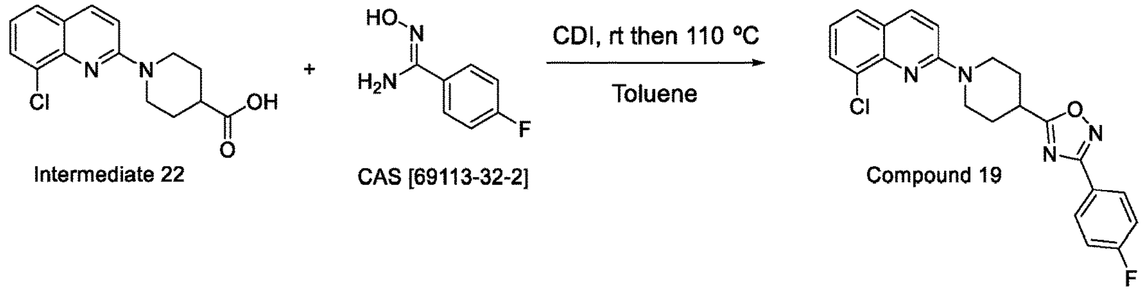


Intermediate 22 CAS [69113-32-2] Compound 19

In an embodiment, preparation of compound 19 (5-(1-(8-chloroquinolin-2-yl)piperidin-4-yl)-3-(4-fluorophenyl)-1,2,4-oxadiazole) was performed. CDI (470 mg, 2.90 mmol) was added to a solution of Intermediate 22 (560 mg, 1.93 mmol) in PhMe (10 mL) and the reaction mixture was allowed to stir at room temperature for 2 hours. Then 4-fluoro-N'-Hydroxybenzimidamide (447 mg, 2.90 mmol) was added and the reaction mixture was allowed to stir for 1 hour at room temperature and for 17 hours at 110 °C. The solvent was evaporated in high vacuum and the resulting residue was purified by column chromatography (silica gel, Petrol ether/EtOAc 3:1) in order to obtain compound 19 as an off-white solid (327 mg, 42%).

HPLC-MS $[M+H]^+$ 409.21; MP: 145-147 °C; $^1H$ NMR (400 MHz, $CDCl_3$) δ 8.13 - 8.03 (m, 2H), 7.90 (d, *J* = 9.2 Hz, 1H), 7.67 (dd, *J* = 7.5, 1.4 Hz, 1H), 7.52 (dd, *J* = 8.0, 1.4 Hz, 1H), 7.21 - 7.09 (m, 3H), 7.06 (d, *J* = 9.1 Hz, 1H), 4.65 (dt, *J* = 13.6, 3.9 Hz, 2H), 3.40 - 3.26 (m, 3H), 2.30 (dt, *J* = 13.5, 3.7 Hz, 2H), 2.16 - 2.01 (m, 2H); $^{13}C$ NMR (101 MHz, $CDCl_3$) δ 181.52, 167.46, 165.78, 163.29, 157.02, 143.99, 137.95, 130.50, 129.64, 126.14, 124.04, 122.10, 116.13, 115.91, 110.12, 44.48, 34.83, 29.02.

In an embodiment, the method of synthesizing compound 20 (3-(pyridin-3-yl)-5-(1-(8-(pyridin-4-yl)quinolin-2-yl)piperidin-4-yl)-1,2,4-oxadiazole hydrochloride) is as follows:

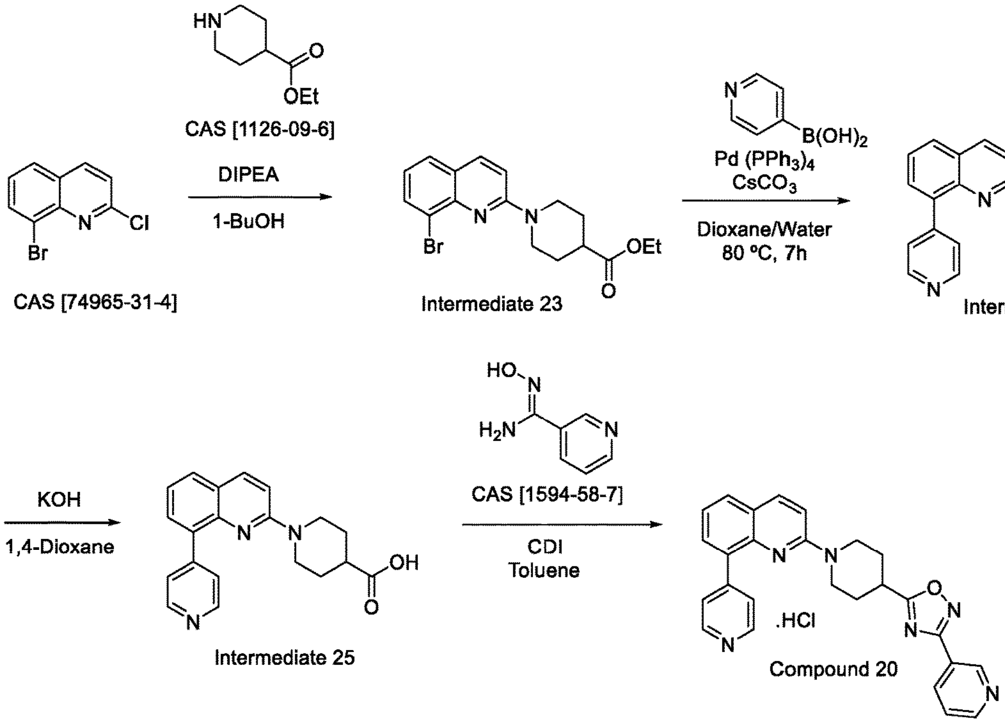


CAS [1126-09-6] CAS [74965-31-4] Intermediate 23 Intermediate 24 Intermediate 25 CAS [1594-58-7] .HCl Compound 20

In an embodiment, preparation of intermediate 23 (Ethyl 1-(8-bromoquinolin-2-yl)piperidine-4-carboxylate) was performed. DIPEA (5 ml) was added to a suspension of 8-bromo-2-chloroquinoline (1 g, 6.2 mmol) and ethyl isonipecotate (1.9 g, 12.3 mmol) in 1-BuOH (20 ml) and the mixture was allowed to stir at 130 °C over a duration of 20 hours. The solvent was evaporated, and the resulting residue was purified by column chromatography (silica gel, Petrol ether/EtOAc 4:1) in order to obtain the title Intermediate 23 as a yellow solid (1.2 g, 54%).

In an embodiment, preparation of intermediate 24 (ethyl 1-(8-(pyridin-4-yl)quinolin-2-yl)piperidine-4-carboxylate) was performed. Tetrakis(triphenylphosphine)palladium(0) (192 mg, 0.15 mmol) was added to a stirring suspension of intermediate 23 (1.2 g, 3.3 mmol), pyridine-4-ylboronic acid (450 mg, 3.7 mmol) and caesium carbonate (6 ml, 3M aqueous

solution) in a mixture of 1,4-dioxane (30 ml) and water (5 ml). The suspension was allowed to stir at 80 °C for a duration of 20 hours. The solvent was evaporated in high vacuum and the residue was purified by column chromatography (silica gel, Petrol ether/EtOAc 4:1). Intermediate 24 was isolated as a yellow solid (1.2 g, 89%).

In an embodiment, preparation of intermediate 25 (1-(8-(pyridin-4-yl)quinolin-2-yl)piperidine-4-carboxylic acid) was performed. Potassium hydroxide (60 mL, 5N aqueous solution) was added to a solution of Intermediate 24 (1.2 g, 3.3 mmol) in 1,4-dioxane (60 ml). The mixture was allowed to stir at 80 °C over a duration of 3.5 hours. The organic solvent was evaporated and the resulting aqueous mixture was acidified with acetic acid to pH1. The precipitate was separated by filtration and Intermediate 25 was obtained as a dark yellow solid (698 mg, 64%).

In an embodiment, preparation of compound 20 (3-(pyridin-3-yl)-5-(1-(8-(pyridin-4-yl)quinolin-2-yl)piperidin-4-yl)-1,2,4-oxadiazole hydrochloride) was performed. CDI (511 mg, 3.15 mmol) was added to a solution of Intermediate 25 (698 mg, 2.1 mmol) in PhMe (10 mL) and the reaction mixture was allowed to stir at room temperature for 2 hours. Then (Z)-N'-hydroxynicotinimidamide (432 mg, 3.15 mmol) was added and the reaction mixture was allowed to stir for 1 hour at room temperature and for 20 hours at 110 °C. The solvent was evaporated in high vacuum and the resulting residue was purified by column chromatography (silica gel, DCM/MeOH 2%). The product was dissolved in EtOAc (5 mL) and a 2M solution of hydrochloric acid in diethyl ether (1 mL) was added dropwise at 0 °C. The precipitate was separated by filtration and was washed with cold EtOAc. Compound 20 was isolated as a yellow solid (456 mg, 46%).

HPLC-MS $[M+H]^+$ 435.27; MP: 273-277 °C; $^1H$ NMR (400 MHz, DMSO-$d_6$) δ 9.23 (bs, 1H), 9.03 - 8.96 (m, 2H), 8.90 (dd, *J* = 5.1, 1.6 Hz, 1H), 8.59 (dt, *J* = 8.1, 1.9 Hz, 1H), 8.55 - 8.48 (m, 2H), 8.22 (d, *J* = 9.3 Hz, 1H), 7.97 (dd, *J* = 8.0, 1.5 Hz, 2H), 7.92 (dd, *J* = 7.4, 1.5 Hz, 1H), 7.83 (dd, *J* = 8.1, 5.1 Hz, 1H), 7.49 - 7.39 (m, 2H), 4.44 (dt, *J* = 13.7, 3.8 Hz, 2H), 3.60 (tt, *J* = 10.9, 4.0 Hz, 1H), 3.30 (ddd, *J* = 13.8, 11.3, 2.7 Hz, 2H), 2.22 (dd, *J* = 13.7, 3.8 Hz, 2H), 1.94 - 1.79 (m, 2H); $^{13}C$ NMR (101 MHz, DMSO-$d_6$) δ 183.13, 165.55, 157.44, 157.30, 149.93, 145.56, 144.54, 140.72, 138.82, 138.11, 132.18, 131.63, 130.44, 128.31, 126.10, 124.08, 123.54, 122.46, 111.42, 44.38, 34.18, 28.88.

In an embodiment, the method of synthesizing compound 21 (5-(1-(6-methoxyquinolin-2-yl)piperidin-4-yl)-3-(pyridin-3-yl)-1,2,4-oxadiazole) is as follows:

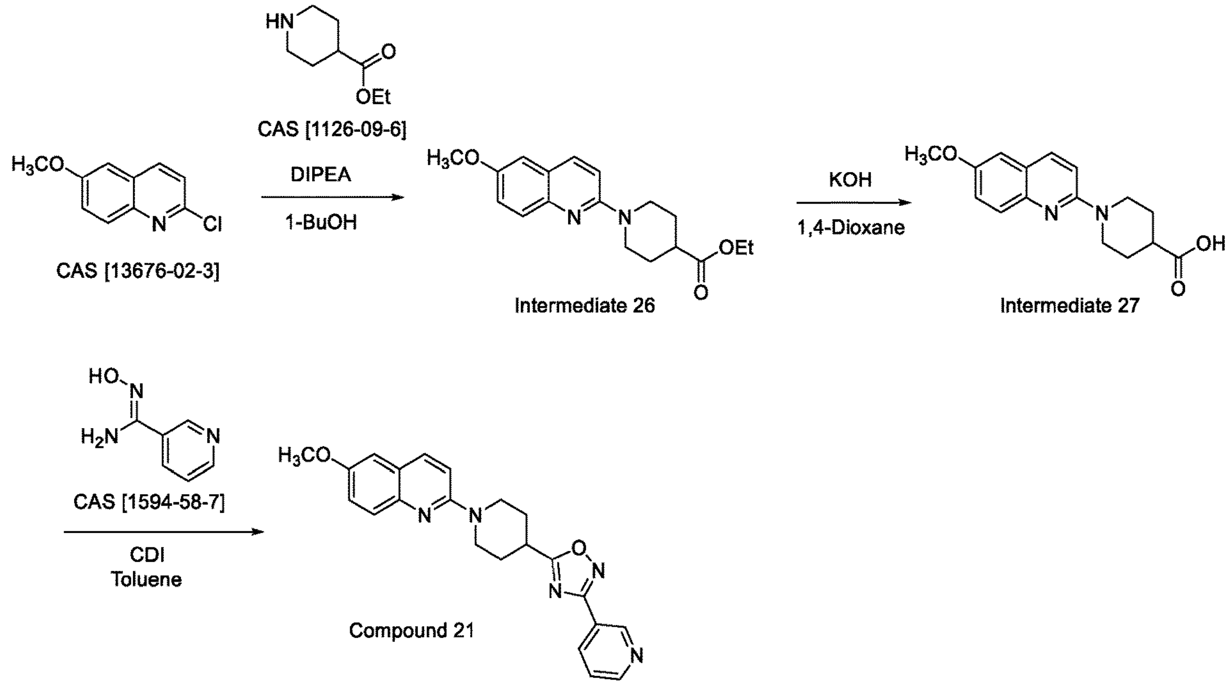


In an embodiment, preparation of Intermediate 26 (ethyl 1-(6-methoxyquinolin-2-yl)piperidine-4-carboxylate) was performed. DIPEA (5 ml) was added to a suspension of 2-Chloro-6-methoxyquinoline (1g, 5.2 mmol) and ethyl isonicotinate (972 mg, 6.2 mmol) in 1-BuOH (20 ml) and the mixture was allowed to stir at 130 °C for 20 hours. The solvent was evaporated and the resulting residue was purified by column chromatography (silica gel, Petrol ether/EtOAc 4:1) and intermediate 26 was isolated as an off-white solid (900 mg, 55%).

In an embodiment, preparation of intermediate 27 (1-(6-methoxyquinolin -2-yl)piperidine-4-carboxylic acid) was performed. A 5N aqueous solution of potassium hydroxide (20 ml) was added to a solution of Intermediate 26 (900 mg, 2.86 mmol) in 1,4-dioxane (20 ml). The solution was allowed to stir at 80 °C over a duration of 2 hours. The solvent was evaporated in vacuum and the mixture was acidified to pH 1 with hydrochloric acid at 0 °C. The precipitate was separated by filtration and was washed with water. The intermediate 27 was isolated as an off-white solid (1.02 g).

In an embodiment, preparation of compound 21 (5-(1-(6-methoxyquinolin-2-yl)piperidin-4-yl)-3-(pyridin-3-yl)-1,2,4-oxadiazole) was performed. CDI (356 mg, 2.97 mmol) was added to a solution of Intermediate 27 (567 mg, 1.98 mmol) in PhMe (9 mL) and the reaction mixture was allowed to stir at room temperature for 2 hours. Then N'-Hydroxynicotinimidamide (410 mg, 2.97 mmol) was added and the reaction mixture was allowed to stir for 1 hour at room temperature and for 23 hours at 110 °C. The solvent was evaporated in high vacuum and the resulting residue was purified by column chromatography (silica gel, Petrol ether/EtOAc 1:2) and compound 21 was isolated as an off-white solid (199 mg, 26%).

HPLC-MS $[M+H]^+$ 388.24; MP: 185-186 °C; $^1H$ NMR (400 MHz, DMSO-$d_6$) δ 9.17 (bs, 1H), 8.82 - 8.76 (m, 1H), 8.36 (dt, *J* = 8.0, 1.9 Hz, 1H), 8.27 (d, *J* = 9.3 Hz, 1H), 7.80 (d, *J* = 8.6 Hz, 1H), 7.62 (ddd, J = 8.0, 4.8, 0.9 Hz, 1H), 7.54 (d, *J* = 9.6 Hz, 1H), 7.41 - 7.34 (m, 2H), 4.47 (dt, *J* = 13.7, 3.8 Hz, 2H), 3.86 (s, 3H), 3.66 - 3.57 (m, 1H), 3.54 - 3.44 (m, 2H), 2.30 (dd, *J* = 13.5, 3.8 Hz, 2H), 2.06 - 1.92 (m, 2H); $^{13}C$ NMR (101 MHz, DMSO-$d_6$) δ 182.51, 166.22, 156.09, 152.76, 148.11, 135.09, 124.83, 122.95, 122.48, 108.21, 55.99, 45.77, 33.54, 28.78.

In an embodiment, the method of synthesizing compound 22 (5-(1-(6-methoxyquinolin-2-yl)piperidin-4-yl)-3-(4-(trifluoromethoxy)phenyl)-1,2,4-oxadiazole) is as follows:

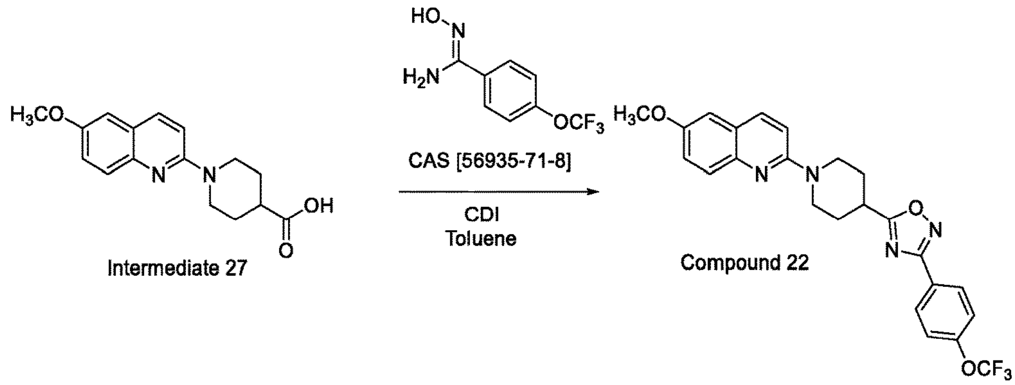

Intermediate 27
Compound 22

In an embodiment, preparation of compound 22 (5-(1-(6-methoxyquinolin-2-yl)piperidin-4-yl)-3-(4-(trifluoromethoxy)phenyl)-1,2,4-oxadiazole) was performed. CDI (290 mg, 2.4 mmol) was added to a solution of Intermediate 27 (454 mg, 1.6 mmol) in PhMe (7 mL) and the reaction mixture was allowed to stir at room temperature for 2 hours. Then 4-(Trifluoromethoxy)benzamidoxime (340 mg, 2.4 mmol) was added and the reaction mixture was allowed to stir for 1 hour at room temperature and for 23 hours at 110 °C. The solvent was evaporated in high vacuum and the resulting residue was purified by column chromatography (silica gel, Petrol ether/EtOAc 4:1) and compound 22 was isolated as a pale brown solid (100 mg, 13%).

HPLC-MS $[M+H]^+$ 471.18; MP: 151-153 °C; $^1H$ NMR (400 MHz, $CDCl_3$) δ 8.12 (d, *J* = 8.6 Hz, 2H), 7.96 - 7.86 (m, 2H), 7.31 (d, *J* = 8.4 Hz, 3H), 7.05 (d, *J* = 9.3 Hz, 1H), 7.01 - 6.96 (m, 1H), 4.49 (dt, *J* = 13.8, 4.4 Hz, 2H), 3.88 (s, 3H), 3.45 - 3.31 (m, 3H), 2.35 - 2.31 (m, 2H), 2.16 - 2.09 (m, 2H); $^{13}C$ NMR (101 MHz, $CDCl_3$) δ 181.29, 167.29, 161.91, 156.10, 151.27, 130.80, 129.20, 125.36, 123.00, 122.37, 122.33, 121.09, 121.00, 119.72, 110.62, 106.54, 55.60, 45.81, 34.25, 28.94.

In an embodiment, the method of synthesizing compound 23 (5-(1-(8-methoxyquinolin-2-yl)piperidin-4-yl)-3-(4-nitrophenyl)-1,2,4-oxadiazole) is as follows:

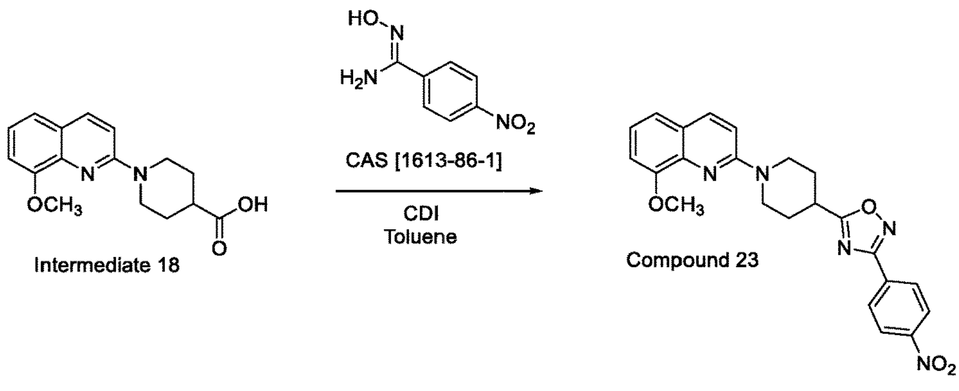

Intermediate 18
Compound 23

In an embodiment, preparation of compound 23 (5-(1-(8-methoxyquinolin-2-yl)piperidin-4-yl)-3-(4-nitrophenyl)-1,2,4-oxadiazole) was performed. CDI (440 mg, 2.7 mmol) was added to a solution of Intermediate 18 (700 mg,

2.44 mmol) in PhMe (10 mL) and the reaction mixture was allowed to stir at room temperature for 2 hours. Then 4-nitrobenzamidoxime (580 mg, 3.2 mmol) was added and the reaction mixture was allowed to stir for 1 hour at room temperature and for 23 hours at 110 °C. The solvent was evaporated in high vacuum and the resulting residue was purified by column chromatography (silica gel, DCM/MeOH 5%) and compound 23 was isolated as an orange solid (513 mg, 49%).

HPLC-MS $[M+H]^+$432.18; MP: 145-147 °C; $^1H$ NMR (400 MHz, DMSO-$d_6$) δ 8.43 - 8.35 (m, 2H), 8.31 - 8.22 (m, 2H), 8.02 (d, *J* = 9.2 Hz, 1H), 7.34 - 7.24 (m, 2H), 7.15 (t, *J* = 7.8 Hz, 1H), 7.04 (dd, *J* = 7.8, 1.3 Hz, 1H), 4.57 (dt, *J* = 13.3, 3.8 Hz, 2H), 3.91 (s, 3H), 3.53 (tt, *J* = 11.0, 3.9 Hz, 1H), 3.27 - 3.15 (m, 2H), 2.28 - 2.19 (m, 2H), 1.94 - 1.79 (m, 2H); $^{13}C$ NMR (101 MHz, DMSO-$d_6$) δ 183.33, 166.67, 156.50, 153.71, 149.59, 139.20, 137.93, 132.51, 128.88, 124.91, 123.92, 122.41, 119.79, 110.70, 109.76, 56.12, 44.42, 34.35, 28.95.

In an embodiment, the method of synthesizing compound 24 (4-(5-(1-(8-methoxyquinolin-2-yl)piperidin-4-yl)-1,2,4-oxadiazol-3-yl)phenol) is as follows:

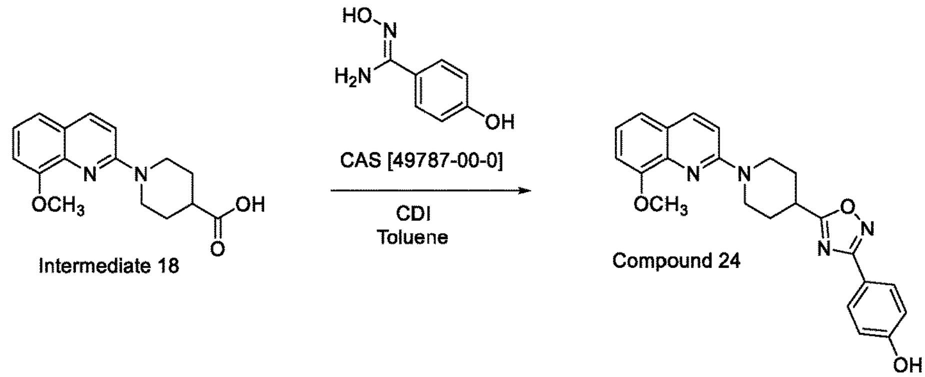


In an embodiment, preparation of compound 24 (4-(5-(1-(8-methoxyquinolin-2-yl)piperidin-4-yl)-1,2,4-oxadiazol-3-yl)phenol) was performed. CDI (400 mg, 2.39 mmol) was added to a solution of Intermediate 18 (500 mg, 1.75 mmol) in PhMe (10 mL) and the reaction mixture was allowed to stir at room temperature for 2 hours. Then 4-hydroxybenzamidine oxime (242 mg, 1.59 mmol) was added and the reaction mixture was allowed to stir for 1 hour at room temperature and for 4 hours at 110 °C. Water was added to the reaction mixture and the product was extracted with EtOAc. The organic layer was separated, dried over anhydrous sodium sulphate, was filtered and evaporated in high vacuum. The resulting residue was purified by column chromatography (silica gel, DCM/MeOH 5%) and compound 24 was isolated as an off-white solid (146 mg, 23%).

HPLC-MS $[M+H]^+$ 403.19; MP: 200-203 °C; $^1H$ NMR (400 MHz, DMSO-$d_6$) δ 10.12 (bs, 1H), 8.02 (d, *J* = 9.2 Hz, 1H), 7.87 - 7.79 (m, 2H), 7.31 - 7.26 (m, 2H), 7.15 (*t*, J = 7.9 Hz, 1H), 7.04 (dd, *J* = 7.8, 1.3 Hz, 1H), 6.95 - 6.86 (m, 2H), 4.56 (dt, *J* = 13.2, 3.9 Hz, 2H), 3.90 (s, 3H), 3.43 (tt, *J* = 12.5, 4.6 Hz, 1H), 3.19 (ddd, *J* = 13.9, 11.9, 2.7 Hz, 2H), 2.19 (dd, *J* = 13.6, 3.7 Hz, 2H), 1.89 - 1.76 (m, 2H); $^{13}C$ NMR (101 MHz, DMSO-$d_6$) δ 181.98, 167.79, 160.71, 156.51, 153.72, 139.22, 137.92, 129.23, 123.91, 122.38, 119.79, 117.39, 116.38, 110.69, 109.77, 56.12, 44.47, 34.31, 29.08.

In an embodiment, the method of synthesizing compound 25 (5-(1-(8-methoxyquinolin-2-yl)piperidin-4-yl)-3-(pyridin-2-yl)-1,2,4-oxadiazole hydrochloride) is as follows:

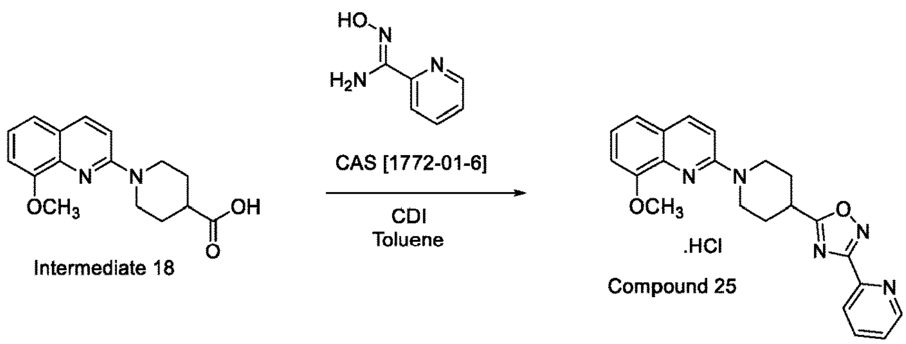


In an embodiment, preparation of compound 25 (5-(1-(8-methoxyquinolin-2-yl)piperidin-4-yl)-3-(pyridin-2-yl)-1,2,4-oxadiazole hydrochloride) was performed. CDI (640 mg, 3.85 mmol) was added to a solution of Intermediate 18 (1g, 3.5 mmol) in PhMe (15 mL) and the reaction mixture was allowed to stir at room temperature for 2 hours. Then Pyridine-2-carboxamide oxime (630 mg, 4.55 mmol) was added and the reaction mixture was allowed to stir for 1 hour at room temperature and for 7 hours at 110 °C. The solvent was evaporated in high vacuum and the resulting residue was

purified by column chromatography (silica gel, Petrol ether/EtOAc 2:1-1:2). The product was dissolved in EtOAc (5 mL) and a 2M solution of hydrochloric acid in diethyl ether (1 mL) was added dropwise at 0 °C. The precipitate was separated by filtration and was washed with cold EtOAc. Compound 25 was isolated as an off-white solid (179 mg, 12%).

HPLC-MS $[M+H]^+$ 388.2; MP: 60-64 °C; $^1$H NMR (400 MHz, MeOD-$d_4$) δ 8.77 - 8.70 (m, 1H), 8.46 (d, *J* = 9.7 Hz, 1H), 8.24 - 8.16 (m, 1H), 8.05 (td, *J* = 7.8, 1.7 Hz, 1H), 7.68 - 7.58 (m, 2H), 7.56 - 7.49 (m, 2H), 7.46 (dd, *J* = 6.1, 3.1 Hz, 1H), 4.48 (dt, *J* = 14.1, 4.2 Hz, 2H), 4.16 (s, 3H), 3.85 - 3.63 (m, 3H), 2.50 (dt, *J* = 13.0, 4.0 Hz, 2H), 2.32 - 2.18 (m, 2H); $^{13}$C NMR (101 MHz, MeOD-$d_4$) δ 181.74, 167.67, 151.79, 149.69, 147.64, 145.75, 143.45, 137.87, 123.26, 121.92, 119.57, 117.36, 112.45, 55.79, 46.30, 32.92, 28.33.

In an embodiment, the method of synthesizing compound 26 (3-(5-(1-(8-methoxyquinolin-2-yl)piperidin-4-yl)-1,2,4-oxadiazol-3-yl)pyridine 1-oxide) is as follows:

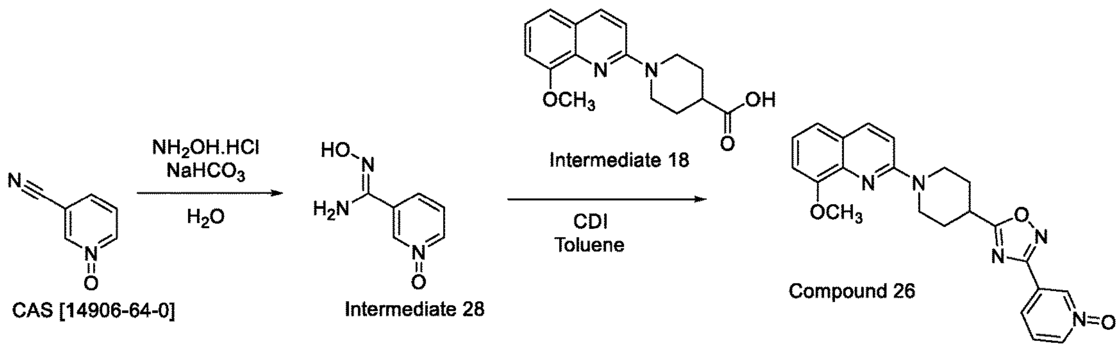


In an embodiment, preparation of Intermediate 28 (2 (Z)-3-(N'-hydroxycarbamimidoyl)pyridine 1-oxide) was performed. Hydroxylamine hydrochloride (0.4 mL, 9.96 mmol) was added dropwise to a solution of nitrile starting material (1g, 8.3 mmol) in water (14 mL). Sodium hydrogencarbonate (840 mg, 9.96 mmol) was added and the mixture was allowed to stir at room temperature over a duration of 18 hours. The solid was separated by filtration and was washed with water to give crude Intermediate 28 as a colourless solid (1.36 g, 94%).

In an embodiment, preparation of compound 26 (3-(5-(1-(8-methoxyquinolin-2-yl)piperidin-4-yl)-1,2,4-oxadiazol-3-yl)pyridine 1-oxide) was performed. CDI (640 mg, 3.85 mmol) was added to a solution of Intermediate 18 (1 g, 3.5 mmol) in PhMe (15 mL) and the reaction mixture was allowed to stir at room temperature for 2 hours. Then Intermediate 28 (700 mg, 4.55 mmol) was added and the reaction mixture was allowed to stir for 1 hour at room temperature and for 7 hours at 110 °C. The solvent was evaporated in high vacuum and the resulting residue was purified by column chromatography (silica gel, DCM/MeOH 5%) and the title compound 26 was isolated as an off-white solid (157 mg, 11%).

HPLC-MS $[M+H]^+$ 404.17; MP: 189-192 °C; $^1$H NMR (400 MHz, DMSO-$d_6$) δ 9.44 (bs, 1H), 9.23 (ddd, *J* = 6.5, 1.9, 0.9 Hz, 1H), 8.83 (d, *J* = 9.2 Hz, 1H), 8.68 (dt, *J* = 8.0, 1.2 Hz, 1H), 8.41 (dd, *J* = 8.0, 6.5 Hz, 1H), 8.14- 8.05 (m, 2H), 7.95 (t, *J* = 7.8 Hz, 1H), 7.84 (dd, *J* = 7.8, 1.3 Hz, 1H), 5.37 (dt, *J* = 13.5, 3.8 Hz, 2H), 4.72 (s, 3H), 4.33 (tt, *J* = 11.1, 3.9 Hz, 1H), 4.08 - 3.96 (m, 2H), 3.03 (dd, *J* = 13.4, 3.7 Hz, 2H), 2.75 - 2.58 (m, 2H); $^{13}$C NMR (101 MHz, DMSO-$d_6$) δ 183.39, 164.72, 156.48, 153.71, 141.57, 139.19, 137.94, 137.01, 127.91, 126.47, 123.92, 123.65, 122.41, 119.79, 110.69, 109.77, 56.12, 44.37, 34.33, 28.92.

In an embodiment, the method of synthesizing compound 27 (3-(pyridin-3-yl)-5-(1-(6-(pyridin-4-yl)quinolin-2-yl)piperidin-4-yl)-1,2,4-oxadiazole) is as follows:

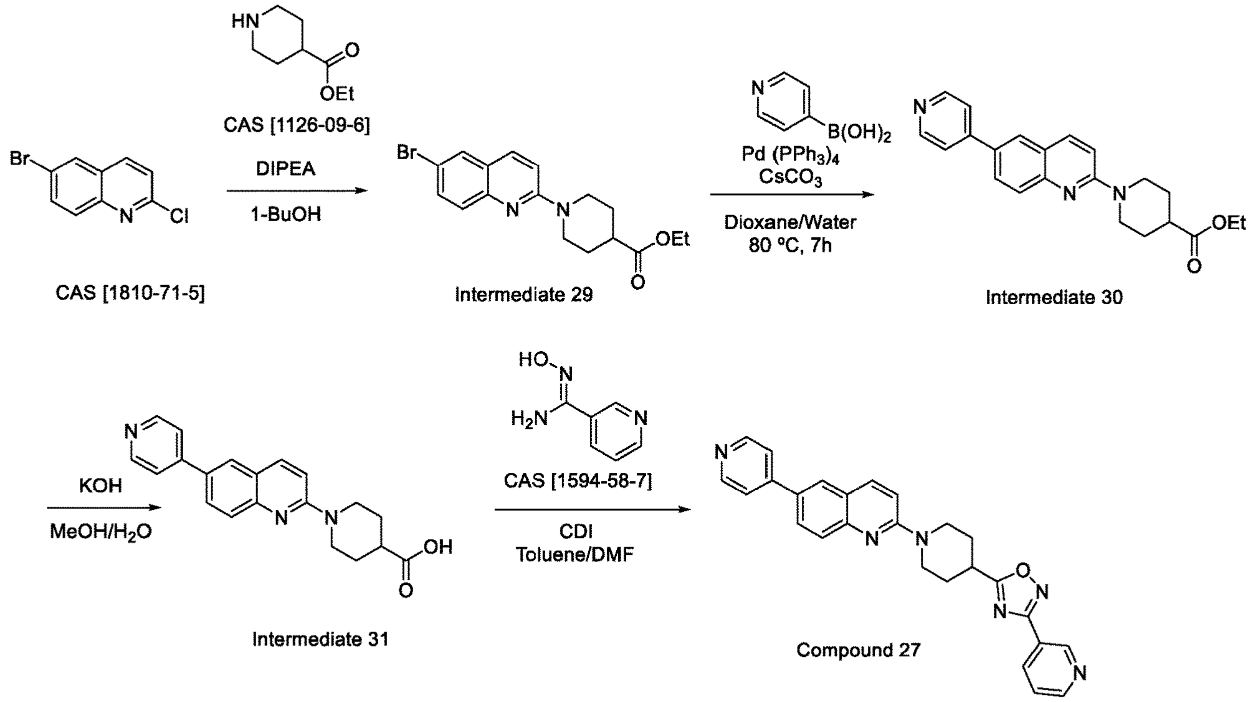


In an embodiment, preparation of intermediate 29 (Ethyl 1-(6-bromoquinolin-2-yl)piperidine-4-carboxylate) was performed. DIPEA (28 ml) was added to a suspension of 6-bromo-2-chloroquinoline (10 g, 41.2 mmol) and ethyl isonipecotate (7.12 g, 45.3 mmol) in 1-BuOH (100 ml) and the mixture was allowed to stir at 130 °C over a duration of 18 hours. The solvent was evaporated, and the resulting residue was purified by column chromatography (silica gel, Petrol ether/EtOAc 8:1-4:1) in order to obtain the title Intermediate 29 as a yellow solid (8.09 g, 55%).

In an embodiment, preparation of intermediate 30 (ethyl 1-(6-(pyridin-4-yl)quinolin-2-yl)piperidine-4-carboxylate) was performed. Tetrakis(triphenylphosphine)palladium(0) (414 mg, 0.36 mmol) was added to a stirring suspension of intermediate 29 (2.6 g, 7.16 mmol), pyridine-4-ylboronic acid (970 mg, 7.87 mmol) and caesium carbonate (12 ml, 3M aqueous solution) in a mixture of 1,4-dioxane (40 ml) and water (9 ml). The suspension was allowed to stir at 80 °C for a duration of 18 hours. The solvent was evaporated in high vacuum and the residue was purified by column chromatography (silica gel, Petrol ether/EtOAc 2:1-1:2) and the title intermediate 30 was isolated as a colourless solid (1.7 g, 71%).

In an embodiment, preparation of intermediate 31 (1-(6-(pyridin-4-yl)quinolin-2-yl)piperidine-4-carboxylic acid) was performed. Potassium hydroxide (1.4 g, 23.5 mmol) was added to a solution of Intermediate 30 (1.7 g, 4.7 mmol) in a mixture of MeOH (34 mL) and water (3.4 ml). The mixture was allowed to stir at room temperature over a duration of 5 hours. The organic solvent was evaporated, water was added and the resulting aqueous mixture was acidified with acetic acid to pH4. No precipitate formed. The aqueous solution was extracted with EtOAc, the organic layer was dried with anhydrous sodium sulphate, was subsequently filtered and evaporated in high vacuum in order to obtain Intermediate 31 as a yellow solid (1.1 g, 70%).

In an embodiment, preparation of Compound 27 (3-(pyridin-3-yl)-5-(1-(6-(pyridin-4-yl)quinolin-2-yl)piperidin-4-yl)-1,2,4-oxadiazole) was performed. CDI (1.3 g, 7.8 mmol) was added to a solution of Intermediate 31 (1.9 g, 5.7 mmol) in a mixture of PhMe (10 mL) and DMF (10 mL) and the reaction mixture was allowed to stir at 50 °C for 15 minutes. Then N'-Hydroxynicotinimidamide (713 mg, 5.2 mmol) was added and the reaction mixture was allowed to stir at 100 °C over a duration of 3 hours. The solvent was evaporated in high vacuum and the resulting residue was purified by column chromatography (silica gel, DCM/MeOH 5%) in order to obtain compound 27 as a yellow solid (570 mg, 23%).

HPLC-MS $[M+2H]^{2+}$ 218.40; MP: 188-190 °C; $^{1}$H NMR (400 MHz, $CDCl_3$) δ 9.30 (bs, 1H), 8.74 (d, *J* = 6.5 Hz, 1H), 8.69 - 8.63 (m, 2H), 8.34 (dt, *J* = 8.0, 2.0 Hz, 1H), 7.98 (d, *J* = 9.2 Hz, 1H), 7.90 (d, *J*= 2.0 Hz, 1H), 7.85 - 7.76 (m, 2H), 7.62 - 7.57 (m, 2H), 7.46 - 7.37 (m, 1H), 7.09 (d, *J* = 9.2 Hz, 1H), 4.62 (dt, *J* = 13.7, 3.9 Hz, 2H), 3.43 - 3.26 (m, 3H), 2.31 (dd, *J* = 13.5, 3.8 Hz, 2H), 2.16 - 2.01 (m, 2H); $^{13}$C NMR (101 MHz, $CDCl_3$) δ 177.15, 161.63, 152.69, 147.28, 145.46, 143.91, 143.59, 143.24, 133.21, 129.92, 127.00, 123.40, 122.73, 120.92, 118.88, 118.37, 118.27, 116.62, 105.57, 39.85, 30.10, 24.30.

In an embodiment, the method of synthesizing compound 28 (5-(5-(1-(8-methoxyquinolin-2-yl)piperidin-4-yl)-1,2,4-oxadiazol-3-yl)-N-methylpyridin-2-amine) is as follows:

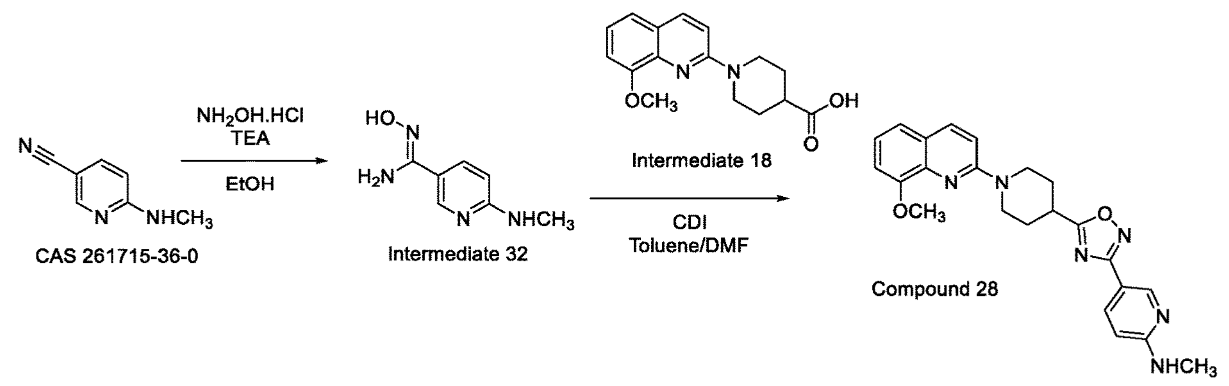


In an embodiment, preparation of Intermediate 32 ((Z)-N'-hydroxy-6-(methylamino)nicotinimidamide) was performed. Hydroxylamine hydrochloride (785 mg, 11.3 mmol) was slowly added to a solution of nitrile starting material (1g, 7.5 mmol) in ethanol (8 mL). Triethylamine (2.1 mL, 15 mmol) was added and the mixture was allowed to stir at room temperature over a duration of 23 hours. The solvent was removed under reduced pressure, the solids were washed with water and crude product was isolated as a colourless solid (2.06g). The crude intermediate 32 was used for the next step without further purification.

In an embodiment, preparation of compound 28 (5-(5-(1-(8-methoxyquinolin-2-yl)piperidin-4-yl)-1,2,4-oxadiazol-3-yl)-N-methylpyridin-2-amine) was performed. CDI (600 mg, 7.8 mmol) was added to a solution of Intermediate 18 (700 mg, 2.44 mmol) in a mixture of PhMe (10 mL) and DMF (5 mL) and the reaction mixture was allowed to stir at room temperature over a duration of 1 hour. Then intermediate 32 (610 mg, 3.67 mmol) was added and the reaction mixture was allowed to stir at 90 °C over a duration of 5 hours. The solvent was evaporated in high vacuum and the resulting residue was purified by column chromatography (silica gel, Petrol ether/EtOAc 1:1) in order to obtain compound 28 as an off-white solid (317 mg, 35%).

HPLC-MS $[M+H]^+$ 417.20; MP: 240-243 °C; $^1$H NMR (400 MHz, DMSO-$d_6$) $\delta$ 8.93 (bs, 1H), 8.38 (d, $J$ = 10.8 Hz, 2H), 8.22 (bs, 1H), 7.63 (d, $J$ = 9.6 Hz, 1H), 7.48 (d, $J$ = 7.9 Hz, 1H), 7.45 - 7.33 (m, 2H), 7.22 (d, $J$ = 9.3 Hz, 1H), 4.48 - 4.42 (m, 2H), 4.03 (s, 3H), 3.56 - 3.50 (m, 3H), 3.06 (s, 3H), 2.34 - 2.27 (m, 2H), 2.06 - 1.96 (m, 2H); $^{13}$C NMR (101 MHz, DMSO-$d_6$) $\delta$ 182.28, 164.84, 155.09, 125.20, 122.57, 120.19, 113.30, 112.60, 111.12, 56.85, 46.58, 34.48, 33.19, 28.90.

In an embodiment, the method of synthesizing compound 29 (5-(1-(8-methoxyquinolin-2-yl)piperidin-4-yl)-3-(3-nitrophenyl)-1,2,4-oxadiazole) is as follows:

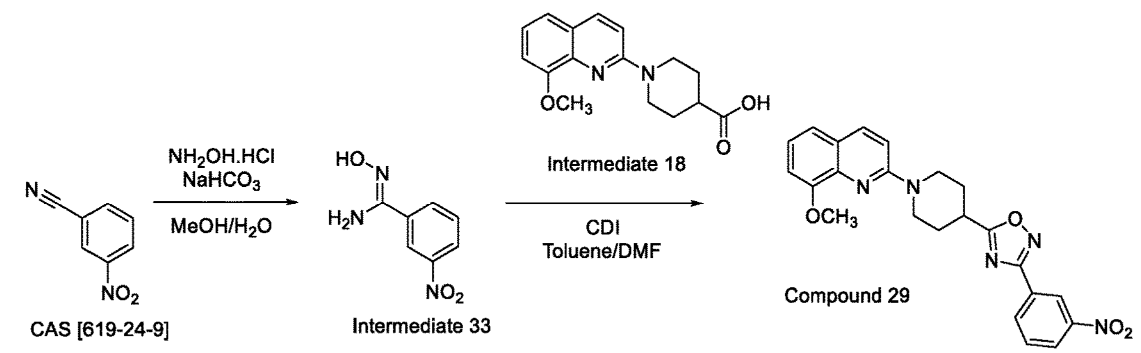


In an embodiment, preparation of Intermediate 33 ((Z)-N'-hydroxy-3-nitrobenzimidamide) was performed. Hydroxylamine hydrochloride (1.1 g, 16.2 mmol) was slowly added to a solution of nitrile starting material (2 g, 13.5 mmol) in a mixture of methanol (MeOH) (10 mL) and water (2 mL). Sodium hydrogencarbonate (1.4 g, 16.2 mmol) was added and the mixture was allowed to stir at 85 °C over a duration of 5 hours. The reaction mixture was cooled down to 0 °C, the precipitate was separated by filtration and was washed with water. Intermediate 33 was isolated as a yellow solid (2.42 g, 98%).

In an embodiment, preparation of compound 29 (5-(1-(8-methoxyquinolin-2-yl)piperidin-4-yl)-3-(3-nitrophenyl)-1,2,4-oxadiazole) was performed. CDI (600 mg, 3.7 mmol) was added to a solution of Intermediate 18 (700 mg, 2.44 mmol) in a mixture of PhMe (10 mL) and DMF (5 mL) and the reaction mixture was allowed to stir at room temperature over a duration of 1 hour. Then intermediate 33 (560 mg, 3.68 mmol) was added and the reaction mixture was allowed to stir at 90 °C over a duration of 5 hours. The solvent was evaporated in high vacuum and the resulting residue was purified by column chromatography (silica gel, Petrol ether/EtOAc 2:1) in order to obtain compound 29 as a yellow solid (189 mg, 18%).

HPLC-MS $[M+H]^+$ 417.20; MP: 240-243 °C; $^1H$ NMR (400 MHz, DMSO-$d_6$) δ 8.69 (bs, 1H), 8.50 - 8.38 (m, 2H), 8.02 (d, $J$ = 9.1 Hz, 1H), 7.87 (t, $J$ = 8.0 Hz, 1H), 7.34 - 7.20 (m, 2H), 7.15 (t, $J$ = 7.8 Hz, 1H), 7.04 (dd, $J$ = 7.8, 1.3 Hz, 1H), 4.57 (dt, $J$ = 13.6, 3.8 Hz, 2H), 3.91 (s, 3H), 3.53 (tt, $J$ = 11.1, 3.9 Hz, 1H), 3.21 (ddd, $J$ = 13.8, 11.6, 2.7 Hz, 2H), 2.24 (dd, $J$ = 13.6, 3.6 Hz, 2H), 1.95 - 1.80 (m, 2H); $^{13}C$ NMR (101 MHz, DMSO-$d_6$) δ 183.29, 166.55, 156.50, 153.73, 148.69, 139.23, 137.92, 133.52, 1310.65, 128.21, 126.53, 123.93, 122.40, 121.94, 119.79, 110.69, 109.81, 56.15, 44.42, 34.38, 28.97.

In an embodiment, the method of synthesizing compound 30 (5-(5-(1-(8-methoxyquinolin-2-yl)piperidin-4-yl)-1,2,4-oxadiazol-3-yl)pyridin-2-amine hydrochloride) is as follows:

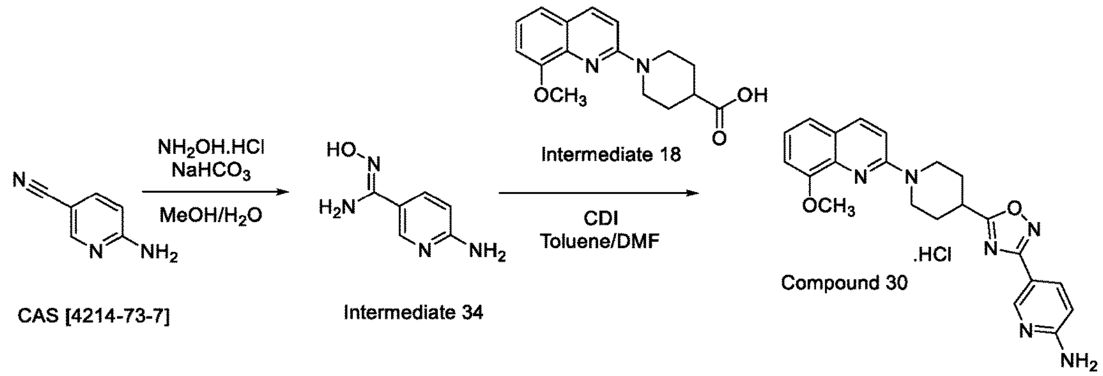

CAS [4214-73-7]
Intermediate 34
Compound 30

In an embodiment, preparation of Intermediate 34 ((Z)-6-amino-N'-hydroxynicotinimidamide) was performed. Hydroxylamine hydrochloride (830 mg, 12.6 mmol) was slowly added to a solution of nitrile starting material (1g, 8.4 mmol) in MeOH (10 mL). A solution of sodium hydrogencarbonate (1.8 g, 16.8 mmol) in water (5.5 mL) was added and the mixture was allowed to stir at 85 °C over a duration of 24 hours. The reaction mixture was cooled down to 0 °C, the precipitate was separated by filtration and was washed with water. Intermediate 34 was isolated as a colourless solid (2.1 g).

In an embodiment, preparation of compound 30 (5-(5-(1-(8-methoxyquinolin-2-yl)piperidin-4-yl)-1,2,4-oxadiazol-3-yl)pyridin-2-amine hydrochloride) was performed. CDI (595 mg, 3.66 mmol) was added to a solution of Intermediate 18 (700 mg, 2.44 mmol) in a mixture of PhMe (10 mL) and DMF (5 mL) and the reaction mixture was allowed to stir at room temperature over a duration of 1 hour. Then intermediate 34 (560 mg, 3.66 mmol) was added and the reaction mixture was allowed to stir at 90 °C over a duration of 5 hours. The solvent was evaporated in high vacuum and the resulting residue was purified by column chromatography (silica gel, Petrol ether/EtOAc, 1:2) in order to obtain compound 30 as a clear oil. The product was dissolved in EtOAc (5 mL) and a 2M solution of hydrochloric acid in diethyl ether (2.5 mL) was added dropwise at 0 °C. The precipitate was separated by filtration and was washed with cold diethyl ether. Compound 30 was isolated as an off-white solid (39.5 mg, 4%).

HPLC-MS $[M+H]^+$ 403.20; MP: not applicable (oil); $^1H$ NMR (400 MHz, DMSO-$d_6$) δ 8.78 (bs, 2H), 8.52 (bs, 1H), 8.31 (dt, $J$ = 9.3, 2.4 Hz, 2H), 7.57 (d, $J$ = 9.6 Hz, 1H), 7.45 (dd, $J$ = 7.9, 1.4 Hz, 1H), 7.41 - 7.27 (m, 2H), 7.15 (d, $J$ = 9.3 Hz, 1H), 4.50 - 4.42 (m, 2H), 4.01 (s, 3H), 3.64 - 3.57 (s, 1H), 3.12 - 3.00 (m, 2H), 2.27 (dd, $J$ = 13.7, 3.9 Hz, 2H), 2.04 1.89 (m, 2H), $^{13}C$ NMR (101 MHz, DMSO-$d_6$) δ 182.41, 164.78, 155.78, 140.55, 136.99, 122.80, 120.11, 114.70, 111.55, 56.71, 46.16, 45.79, 34.53, 33.37, 28.88, 8.88.

In an embodiment, the method of synthesizing compound 31 (3-(6-chloropyridin-3-yl)-5-(1-(8-methoxyquinolin-2-yl)piperidin-4-yl)-1,2,4-oxadiazole hydrochloride) is as follows:

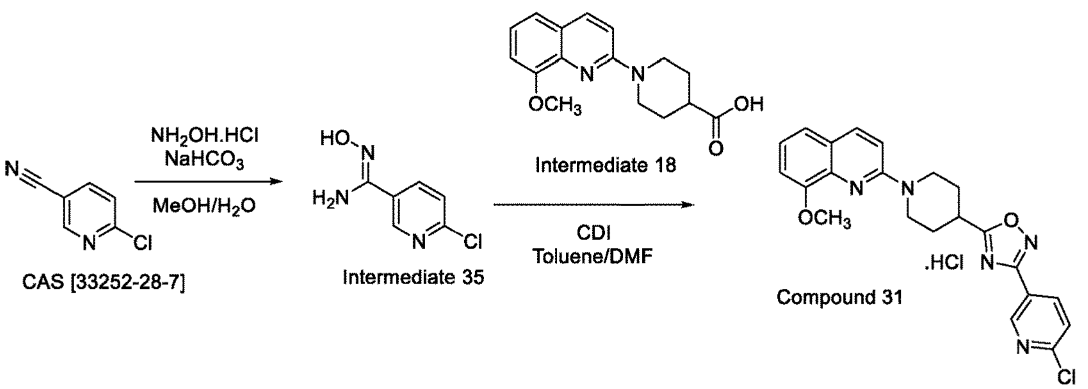

CAS [33252-28-7]
Intermediate 35
Compound 31

In an embodiment, preparation of Intermediate 35 ((Z)-6-chloro-N'-hydroxynicotinimidamide) was performed. Hydroxylamine hydrochloride (1.2 g, 17 mmol) was slowly added to a solution of nitrile starting material (2 g, 14 mmol) in a

mixture of MeOH (20 mL) and water (4 mL). Sodium hydrogencarbonate (1.4 g, 17 mmol) was added and the mixture was allowed to stir at 85 °C over a duration of 2 hours. The reaction mixture was cooled down to 0 °C, the precipitate was separated by filtration and was washed with water. Intermediate 35 was isolated as a colourless solid (1.97 g, 82%).

In an embodiment, preparation of compound 31 (3-(6-chloropyridin-3-yl)-5-(1-(8-methoxyquinolin-2-yl)piperidin-4-yl)-1,2,4-oxadiazole hydrochloride) was performed. CDI (852 mg, 5.24 mmol) was added to a solution of Intermediate 18 (1.4 g, 4.4 mmol) in a mixture of PhMe (20 mL) and DMF (1 mL) and the reaction mixture was allowed to stir at room temperature over a duration of 1 hour. Then intermediate 35 (900 mg, 5.24 mmol) was added and the reaction mixture was allowed to stir at 80 °C over a duration of 18 hours. The solvent was evaporated in high vacuum and the resulting residue was purified by column chromatography (silica gel, Petrol ether/EtOAc 2:1) in order to obtain compound 31 as a clear oil. The product was dissolved in EtOAc (10 mL) and a 2M solution of hydrochloric acid in diethyl ether (2 mL) was added dropwise at 0 °C. The precipitate was separated by filtration and was washed with cold diethyl ether. Compound 31 was isolated as a colourless solid (430 mg, 23%).

HPLC-MS $[M+H]^+$ 422.25; MP: 217-218 °C; $^1H$ NMR (400 MHz, DMSO-$d_6$) δ 8.99 (bs, 1H), 8.40 (dd, *J* = 8.4, 2.4 Hz, 1H), 8.36 (d, *J* = 9.1 Hz, 1H), 7.75 (dd, *J* = 8.3, 0.7 Hz, 1H), 7.61 (d, *J* = 9.6 Hz, 1H), 7.47 (d, *J* = 7.7 Hz, 1H), 7.44 - 7.33 (m, 2H), 4.46 (dt, *J* = 14.2, 3.8 Hz, 2H), 4.03 (s, 3H), 3.70 - 3.65 (m, 1H), 3.58 - 3.47 (m, 2H), 2.36 - 2.26 (m, 2H), 2.08 - 1.94 (m, 2H); $^{13}C$ NMR (101 MHz, DMSO-$d_6$) δ 182.68, 165.47, 153.35, 148.54, 138.45, 125.63, 122.68, 122.45, 120.16, 56.79, 46.37, 33.31, 28.88.

In an embodiment, the method of synthesizing compound 32 (5-(1-(8-methoxyquinolin-2-yl)piperidin-4-yl)-3-(pyrimidin-2-yl)-1,2,4-oxadiazole) is as follows:

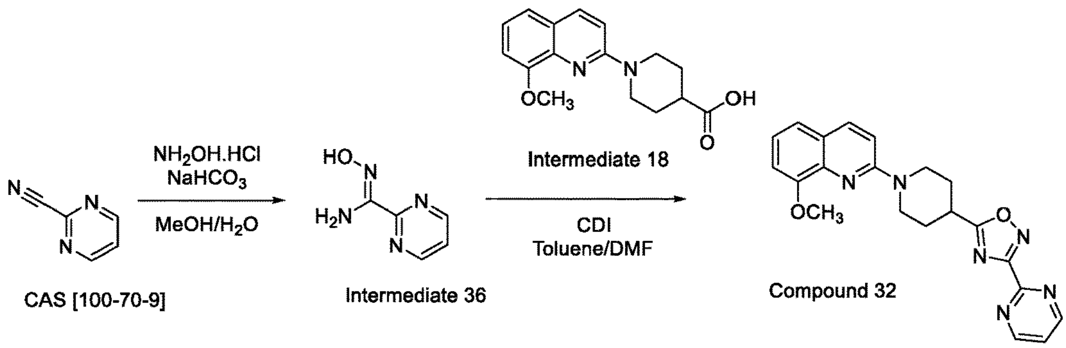

CAS [100-70-9] Intermediate 36 Compound 32

In an embodiment, preparation of Intermediate 36 ((Z)-N'-hydroxypyrimidine-2-carboximidamide) was performed. Hydroxylamine hydrochloride (390 mg, 5.7 mmol) was slowly added to a solution of nitrile starting material (0.5 g, 4.7 mmol) in a mixture of MeOH (5 mL) and water (1 mL). Sodium hydrogencarbonate (470 mg, 5.7 mmol) was added and the mixture was allowed to stir at room temperature for 1h and at 85 °C over a duration of 20 hours. The reaction mixture was evaporated in vacuum and the resulting residue was suspended in water and was filtered. Intermediate 36 was isolated as a colourless solid (447 mg, 68%).

In an embodiment, preparation of compound 32 (5-(1-(8-methoxyquinolin-2-yl)piperidin-4-yl)-3-(pyrimidin-2-yl)-1,2,4-oxadiazole) was performed. CDI (510 mg, 3.2 mmol) was added to a solution of Intermediate 18 (600 mg, 2.1 mmol) in a mixture of PhMe (9 mL) and DMF (4 mL) and the reaction mixture was allowed to stir at room temperature over a duration of 1 hours. Then intermediate 36 (440 mg, 3.2 mmol) was added and the reaction mixture was allowed to stir at 90 °C over a duration of 2 hours. The solvent was evaporated in high vacuum and the resulting residue was purified by column chromatography (silica gel, DCM/MeOH 2%) in order to obtain compound 32 as an off-white solid (270 mg, 33%).

HPLC-MS $[M+H]^+$ 389.12; MP: 167-169 °C; $^1H$ NMR (500 MHz, DMSO-$d_6$) δ 9.05 - 9.00 (m, 2H), 8.02 (d, *J* = 9.2 Hz, 1H), 7.75 - 7.68 (m, 1H), 7.33 - 7.23 (m, 2H), 7.14 (t, *J* = 7.8 Hz, 1H), 7.03 (dd, *J* = 7.8, 1.4 Hz, 1H), 4.56 (dt, *J* = 13.4, 3.7 Hz, 2H), 3.91 (s, 3H), 3.51 (tt, *J* = 11.1, 3.9 Hz, 1H), 3.20 (ddd, *J* = 13.7, 11.5, 2.7 Hz, 2H), 2.24 (dd, *J* = 13.5, 3.6 Hz, 2H), 1.92 - 1.80 (m, 2H); $^{13}C$ NMR (126 MHz, DMSO-$d_6$) δ 183.23, 167.75, 158.67, 156.56, 155.88, 153.75, 139.24, 137.92, 123.95, 123.27, 122.41, 119.80, 110.72, 109.82, 56.16, 44.45, 34.37, 28.98.

In an embodiment, the method of synthesizing compound 33 (5-(1-(8-methoxyquinolin-2-yl)piperidin-4-yl)-3-(6-(piperidin-1-yl)pyridin-3-yl)-1,2,4-oxadiazole) is as follows:

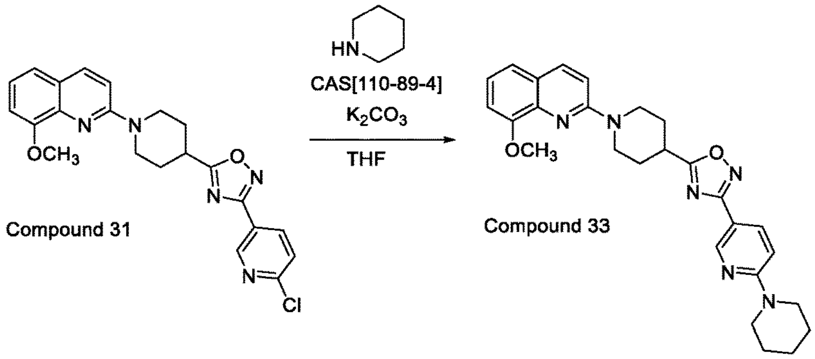


Compound 31

Compound 33

In an embodiment, preparation of compound 33 (5-(1-(8-methoxyquinolin-2-yl)piperidin-4-yl)-3-(6-(piperidin-1-yl)pyridin-3-yl)-1,2,4-oxadiazole) was performed. Piperidine (0.2 mL, 1.54 mmol) was added to a stirring dispersion of Compound 31 (300 mg, 0.7 mmol) and potassium carbonate (213 mg, 1.54 mmol) in THF (5 mL) at room temperature. The mixture was allowed to stir at 50 °C over a duration of 20 hours. The solvent was evaporated in high vacuum and the residue was dissolved in EtOAc and was washed with water, followed by brine. The organic layer was separated, dried over anhydrous sodium sulphate, was filtered and evaporated in high vacuum to give an oily solid. The solid was triturated with diethylether and was separated by filtration. Compound 33 was isolated as an off-white solid (247 mg, 75%).

HPLC-MS $[M+H]^+$ 471.20; MP: 165-166 °C; $^1$H NMR (400 MHz, $CDCl_3$) δ 8.83 (bs, 1H), 8.03 (dd, *J* = 9.0, 2.4 Hz, 1H), 7.90 (d, *J* = 9.1 Hz, 1H), 7.26 - 7.12 (m, 2H), 7.05 (d, *J* = 9.1 Hz, 1H), 6.96 (dd, *J* = 7.6, 1.4 Hz, 1H), 6.67 (d, *J* = 9.0 Hz, 1H), 4.58 (dt, *J* = 13.5, 3.8 Hz, 2H), 4.03 (s, 3H), 3.68 - 3.60 (m, 4H), 3.33 - 3.18 (m, 3H), 2.29 (dt, *J* = 13.7, 3.6 Hz, 2H), 2.14 - 2.02 (m, 2H), 1.69 - 1.63 (m, 6H); $^{13}$C NMR (101 MHz, $CDCl_3$) δ 180.97, 166.86, 160.15, 156.78, 153.55, 147.97, 139.24, 137.67, 135.98, 123.96, 122.27, 119.45, 110.92, 110.05, 108.74, 105.95, 56.14, 45.96, 44.77, 34.87, 29.25, 25.53, 24.70.

In an embodiment, the method of synthesizing compound 34 (5-(1-(8-methoxyquinolin-2-yl)piperidin-4-yl)-3-(6-(piperazin-1-yl)pyridin-3-yl)-1,2,4-oxadiazole) is as follows:

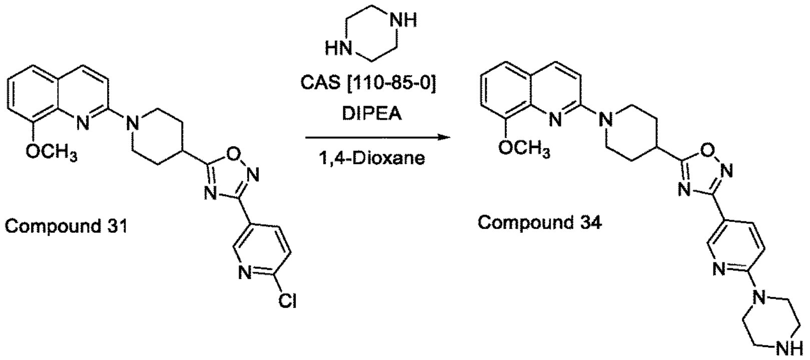


Compound 31

Compound 34

In an embodiment, preparation of compound 34 (5-(1-(8-methoxyquinolin-2-yl)piperidin-4-yl)-3-(6-(piperazin-1-yl)pyridin-3-yl)-1,2,4-oxadiazole) was performed. Piperazine (603 mg, 7 mmol) was added to a stirring dispersion of Compound 31 (300 mg, 0.7 mmol) and DIPEA (0.2 mL, 1.4 mmol) in 1,4-dioxane (6 mL) at room temperature. The mixture was allowed to stir at reflux over a duration of 12 hours. The solvent was evaporated in high vacuum and the resulting oily solid was triturated with diethylether and was separated by filtration. Compound 34 was isolated as an off-white solid (295 mg, 89%).

HPLC-MS $[M+2H]^{2+}$ 236.67; MP: 107-109 °C; $^1$H NMR (400 MHz, $CDCl_3$) δ 8.85 (bs, 1H), 8.07 (d, *J* = 6.6 Hz, 1H), 7.89 (d, *J* = 9.1 Hz, 1H), 7.21 (d, *J* = 8.0 Hz, 1H), 7.16 (t, *J* = 7.7 Hz, 1H), 7.05 (d, *J* = 9.1 Hz, 1H), 6.96 (d, *J* = 7.6 Hz, 1H), 6.66 (d, *J* = 9.0 Hz, 1H), 4.57 (dt, *J* = 13.2, 4.0 Hz, 2H), 4.02 (s, 3H), 3.62 (t, *J* = 5.0 Hz, 4H), 3.28 - 3.21 (m, 3H), 2.97 (t, *J* = 5.0 Hz, 4H), 2.85 (bs, 1H), 2.30 - 2.25 (m, 2H), 2.10 - 2.03 (m, 2H); $^{13}$C NMR (101 MHz, $CDCl_3$) δ 181.12, 166.75, 160.33, 156.78, 153.54, 147.85, 139.23, 137.70, 136.13, 123.97, 122.30, 119.47, 111.86, 110.06, 108.75, 106.02, 56.15, 45.91, 45.89, 44.77, 34.88, 29.26.

In an embodiment, the method of synthesizing compound 35 (2-((5-(5-(1-(8-methoxyquinolin-2-yl)piperidin-4-yl)-1,2,4-oxadiazol-3-yl)pyridin-2-yl)amino)ethan-1-ol hydrochloride) is as follows:

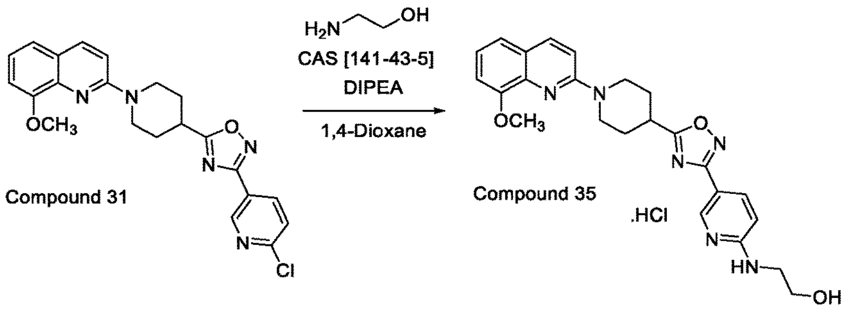


In an embodiment, preparation of compound 35 (2-((5-(5-(1-(8-methoxyquinolin-2-yl)piperidin-4-yl)-1,2,4-oxadiazol-3-yl)pyridin-2-yl)amino)ethan-1-ol hydrochloride) was performed. Ethanolamine (0.3 mL, 5 mmol) was added to a stirring dispersion of Compound 31 (200 mg, 0.5 mmol) and DIPEA (0.2 mL, 1 mmol) in 1,4-dioxane (6 mL) at room temperature. The mixture was allowed to stir at reflux over a duration of 12 hours. The solvent was evaporated in high vacuum and the product was purified by preparative TLC (DCM/MeOH 5%). The resulting oil was dissolved in DCM (5 mL) and a 2M solution of hydrochloric acid in diethyl ether (1 mL) was added dropwise at 0 °C. The precipitate was separated by filtration and was washed with cold diethyl ether. Compound 35 was isolated as a colourless solid (127 mg, 53%).

HPLC-MS $[M+H]^+$ 447.30; MP: 228-231 °C; $^1$H NMR (400 MHz, DMSO-$d_6$) δ 9.27 (bs, 1H), 8.38 (bs, 1H), 8.31 (d, *J* = 9.5 Hz, 1H), 8.18 (dd, *J* = 9.4, 2.2 Hz, 1H), 7.56 (d, *J* = 9.5 Hz, 1H), 7.44 (d, *J* = 7.9 Hz, 1H), 7.40 - 7.26 (m, 2H), 7.17 (d, *J* = 9.3 Hz, 1H), 4.51 - 4.40 (m, 2H), 4.00 (s, 3H), 3.64 - 3.41 (m, 7H), 2.27 (dd, *J* = 13.9, 3.9 Hz, 2H), 2.04 - 1.89 (m, 2H). $^{13}$C NMR (101 MHz, DMSO-$d_6$) δ 182.25, 165.03, 122.80, 120.11, 112.84, 112.23, 111.09, 59.58, 56.71, 46.18, 45.24, 33.39, 28.92.

In an embodiment, the method of synthesizing compound 36 (5-(5-(1-(8-methoxyquinolin-2-yl)piperidin-4-yl)-1,2,4-oxadiazol-3-yl)pyridin-3-ol) is as follows:

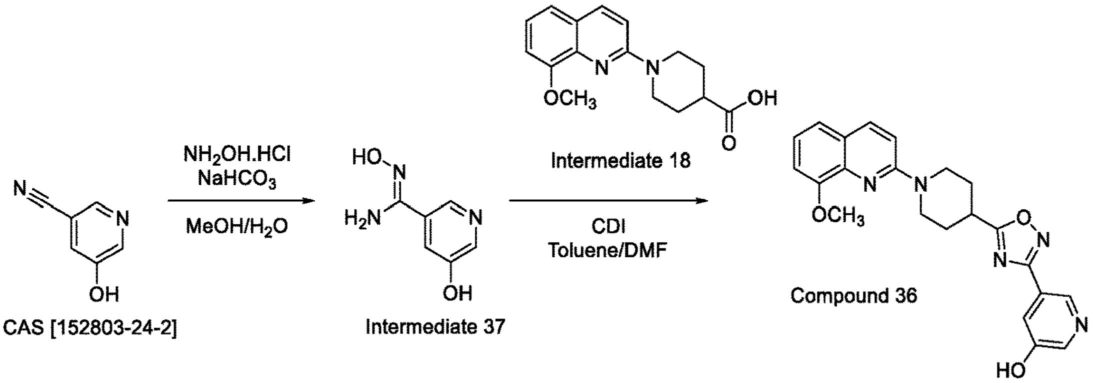


In an embodiment, preparation of Intermediate 37 ((Z)-N',5-dihydroxynicotinimidamide) was performed. Hydroxylamine hydrochloride (695 mg, 10 mmol) was slowly added to a solution of nitrile starting material (1 g, 8.33 mmol) in a mixture of MeOH (10 mL) and water (1 mL). Sodium hydrogencarbonate (840 mg, 10 mmol) was added and the mixture was allowed to stir at 60 °C over a duration of 3 hours. The reaction mixture was evaporated in vacuum and the resulting residue was suspended in water and was filtered. Intermediate 37 was isolated as a colourless solid (1.01 g, 79%).

In an embodiment, preparation of compound 36 (5-(5-(1-(8-methoxyquinolin-2-yl)piperidin-4-yl)-1,2,4-oxadiazol-3-yl)pyridin-3-ol) was performed. CDI (566 mg, 3.5 mmol) was added to a solution of Intermediate 18 (700 mg, 2.9 mmol) in a mixture of PhMe (10 mL) and DMF (2 mL) and the reaction mixture was allowed to stir at room temperature over a duration of 1 hour. Then intermediate 37 (536 mg, 3.5 mmol) was added and the reaction mixture was allowed to stir at room temperature for 30 minutes and at 100 °C over a duration of 5 hours. The solvent was evaporated in high vacuum and the resulting residue was purified by column chromatography (silica gel, EtOAc) in order to obtain compound 36 as a colourless solid (272 mg, 23%).

HPLC-MS $[M+H]^+$ 404.16; MP: 202-204 °C; $^1$H NMR (400 MHz, DMSO-$d_6$) δ 10.43 (bs, 1H), 8.63 (bs, 1H), 8.32 (bs, 1H), 8.02 (d, *J* = 9.2 Hz, 1H), 7.73 - 7.67 (m, 1H), 7.34 - 7.24 (m, 2H), 7.15 (td, *J* = 7.8, 1.6 Hz, 1H), 7.04 (dd, *J* = 7.8, 1.4 Hz, 1H), 4.57 (dt, *J* = 13.4, 3.8 Hz, 2H), 3.91 (s, 3H), 3.50 (tt, *J* = 11.1, 3.8 Hz, 1H), 3.20 (ddd, *J* = 13.7, 11.6, 2.7 Hz, 2H), 2.22 (dd, *J* = 13.4, 3.8 Hz, 2H), 1.92 - 1.77 (m, 2H); $^{13}$C NMR (101 MHz, DMSO-$d_6$) δ 182.84, 166.20, 156.50, 154.30, 153.71, 141.45, 139.21, 138.76, 137.92, 123.92, 123.40, 122.40, 120.12, 119.79, 110.69, 109.77, 56.12, 44.41, 34.34, 28.99.

In an embodiment, the method of synthesizing compound 37 (2 5-(1-(8-methoxyquinolin-2-yl)piperidin-4-yl)-3-(1H-pyrrolo[2,3-b]pyridin-5-yl)-1,2,4-oxadiazole) is as follows:

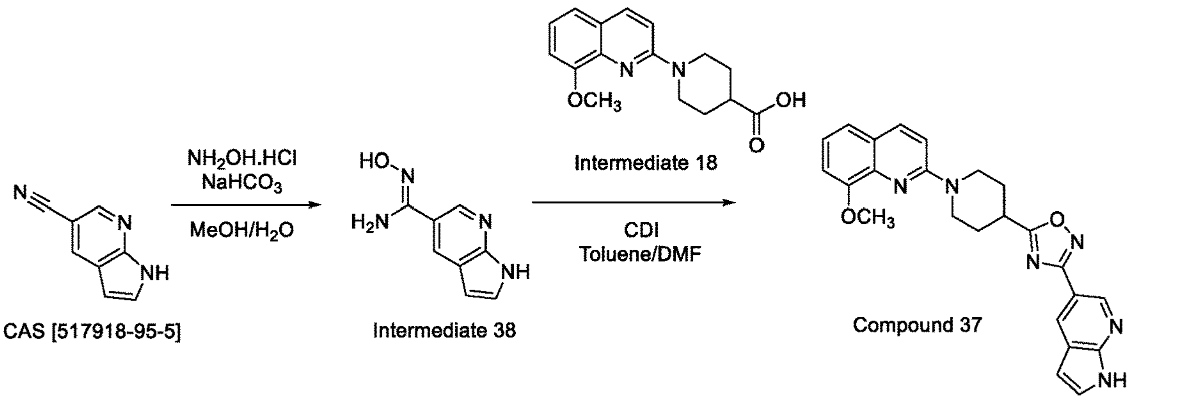


CAS [517918-95-5] Intermediate 38 Compound 37

In an embodiment, preparation of Intermediate 38 (Z)-N'-hydroxy-1H-pyrrolo[2,3-b]pyridine-5-carboximidamide) was performed. Hydroxylamine hydrochloride (585 mg, 8.4 mmol) was slowly added to a solution of nitrile starting material (1g, 7 mmol) in a mixture of MeOH (10 mL) and water (1 mL). Sodium hydrogencarbonate (710 mg, 8.4 mmol) was added and the mixture was allowed to stir at 60 °C over a duration of 4 hours. The reaction mixture was evaporated in vacuum and the resulting residue was suspended in water and was filtered. Intermediate 38 was isolated as off-white solid (1.02 g, 83%).

In an embodiment, preparation of compound 37 (2 5-(1-(8-methoxyquinolin-2-yl)piperidin-4-yl)-3-(1H-pyrrolo [2,3-b]pyridin-5-yl)-1,2,4-oxadiazole) was performed. CDI (476 mg, 2.93 mmol) was added to a solution of Intermediate 18 (700 mg, 2.4 mmol) in a mixture of PhMe (10 mL) and DMF (2 mL) and the reaction mixture was allowed to stir at room temperature over a duration of 1 hour. Then intermediate 38 (516 mg, 2.93 mmol) was added and the reaction mixture was allowed to stir at room temperature for 30 minutes and at 100 °C over a duration of 5 hours. The solvent was evaporated in high vacuum and the resulting residue was purified by column chromatography (silica gel, Petrol ether/EtOAc, 2:1) in order to obtain the product as a colourless solid. The solid was triturated with DCM, was separated by filtration and was washed with diethyl ether. Compound 37 was isolated as a colourless solid (51 mg, 5%).

HPLC-MS $[M+2H]^{2+}$ 214.13; MP: 210-212 °C; $^1H$ NMR (400 MHz, DMSO-$d_6$) δ 12.02 (bs, 1H), 8.84 (bs, 1H), 8.57 (bs, 1H), 8.02 (d, $J$ = 9.2 Hz, 1H), 7.60 (bs, 1H), 7.34 - 7.24 (m, 2H), 7.15 (t, $J$ = 7.8 Hz, 1H), 7.04 (dd, $J$ = 7.8, 1.3 Hz, 1H), 6.61 (bs, 1H), 4.58 (dt, $J$ = 13.5, 3.8 Hz, 2H), 3.91 (s, 3H), 3.49 (tt, $J$ = 10.9, 3.9 Hz, 1H), 3.21 (ddd, $J$ = 13.7, 11.5, 2.7 Hz, 2H), 2.23 (dd, $J$ = 13.5, 3.6 Hz, 2H), 1.93 -1.79 (m, 2H); $^{13}C$ NMR (101 MHz, DMSO-$d_6$) δ 182.29, 167.51, 156.52, 153.72, 150.03, 141.73, 139.22, 137.93, 128.37, 127.52, 123.92, 122.39, 119.93, 119.79, 114.88, 110.71, 109.77, 101.45, 56.13, 44.48, 34.36, 29.07.

In an embodiment, the method of synthesizing compound 38 (3-(furan-2-yl)-5-(1-(8-methoxyquinolin-2-yl) piperidin-4-yl)-1,2,4-oxadiazole) is as follows:

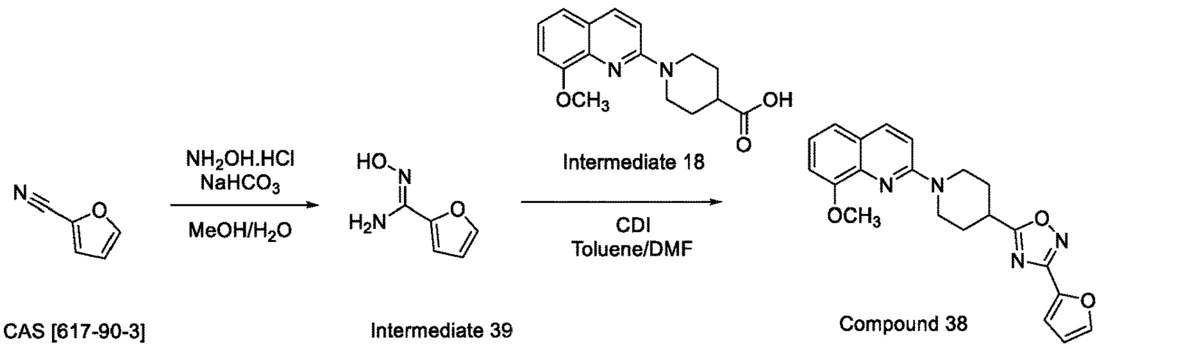


CAS [617-90-3] Intermediate 39 Compound 38

In an embodiment, preparation of Intermediate 39 ((Z)-N'-hydroxyfuran-2-carboximidamide) was performed. Hydroxylamine hydrochloride (440 mg, 6.4 mmol) was slowly added to a solution of nitrile starting material (500 mg, 5.3 mmol) in a mixture of MeOH (5 mL) and water (1 mL). Sodium hydrogencarbonate (530 mg, 6.4 mmol) was added and the mixture was allowed to stir at room temperature for 1h and at 85 °C over a duration of 18 hours. The reaction mixture was evaporated in vacuum and the resulting residue was dissolved in DCM and was washed with water. The organic layer was dried over anhydrous sodium sulphate, was filtered and was evaporated in high vacuum to give Intermediate 39 as an oil (510 mg g, 75%).

In an embodiment, preparation of compound 38 (3-(furan-2-yl)-5-(1-(8-methoxyquinolin-2-yl)piperidin-4-yl)-1,2,4-oxadiazole) was performed. CDI (600 mg, 3.7 mmol) was added to a solution of Intermediate 18 (700 mg,

2.5 mmol) in a mixture of PhMe (10 mL) and DMF (5 mL) and the reaction mixture was allowed to stir at room temperature over a duration of 1 hour. Then intermediate 39 (460 mg, 3.7 mmol) was added and the reaction mixture was allowed to stir at 90 °C over a duration of 20 hours. The solvent was evaporated in high vacuum and the resulting residue was purified by column chromatography (silica gel, DCM/MeOH 2%). Compound 38 was isolated as a brown solid (430 mg, 45%).

HPLC-MS $[M+H]^+$ 377.13; MP: 105-107°C; $^1H$ NMR (500 MHz, DMSO-$d_6$) δ 8.00 (d, *J* = 9.2 Hz, 1H), 7.98 - 7.93 (m, 1H), 7.28 - 7.24 (m, 2H), 7.23 - 7.19 (m, 1H), 7.14 (t, *J* = 7.9 Hz, 1H), 7.03 (dd, *J* = 7.7, 1.2 Hz, 1H), 6.76 - 6.70 (m, 1H), 4.53 (dt, *J* = 13.5, 3.8 Hz, 2H), 3.92 (s, 3H), 3.44 (tt, *J* = 11.1, 4.0 Hz, 1H), 3.20 (ddd, *J* = 13.8, 11.5, 2.8 Hz, 2H), 2.19 (dd, *J* = 13.2, 3.8 Hz, 2H), 1.89 - 1.77 (m, 2H); $^{13}C$ NMR (126 MHz, DMSO-$d_6$) δ 182.39, 161.00, 156.53, 153.83, 146.70, 142.07, 139.36, 137.91, 124.00, 122.41, 119.86, 114.64, 110.66, 110.06, 56.29, 44.48, 34.28, 28.96.

In an embodiment, the method of synthesizing compound 39 (5-(1-(8-methoxyquinolin-2-yl)piperidin-4-yl)-3-(6-methylpyridin-3-yl)-1,2,4-oxadiazole) is as follows:

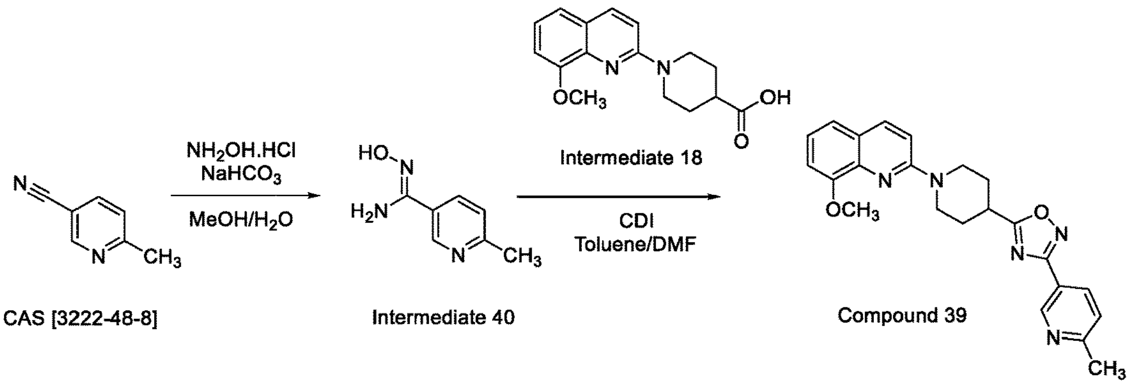

CAS [3222-48-8]
Intermediate 40
Compound 39

In an embodiment, preparation of Intermediate 40 ((Z)-N'-hydroxy-6-methylnicotinimidamide) was performed. Hydroxylamine hydrochloride (340 mg, 5 mmol) was slowly added to a solution of nitrile starting material (500 mg, 4.2 mmol) in a mixture of MeOH (5 mL) and water (1 mL). Sodium hydrogencarbonate (420 mg, 5 mmol) was added and the mixture was allowed to stir at 85 °C over a duration of 4 hours. The reaction mixture was evaporated in vacuum and the resulting residue was suspended in water and was filtered. Intermediate 40 was isolated as a colourless solid (480 mg, 75%).

In an embodiment, preparation of compound 39 (5-(1-(8-methoxyquinolin-2-yl)piperidin-4-yl)-3-(6-methylpyridin-3-yl)-1,2,4-oxadiazole) was performed. CDI (690 mg, 4.2 mmol) was added to a solution of Intermediate 18 (800 mg, 2.8 mmol) in a mixture of PhMe (12 mL) and DMF (5.5 mL) and the reaction mixture was allowed to stir at room temperature over a duration of 1 hour. Then intermediate 40 (630 mg, 4.2 mmol) was added and the reaction mixture was allowed to stir at room temperature for 30 minutes and at 90 °C over a duration of 2 hours. The solvent was evaporated in high vacuum and the resulting residue was recrystallized from Isop. Compound 39 was isolated as an off-white solid (600 mg, 53%).

HPLC-MS $[M+H]^+$402.02; MP: 109-111 °C; $^1H$ NMR (400 MHz, DMSO-$d_6$) δ 9.06-9.00 (m, 1H), 8.23 (dd, *J* = 8.1, 2.3 Hz, 1H), 8.02 (d, *J* = 9.1 Hz, 1H), 7.45 (d, *J* = 8.1 Hz, 1H), 7.33 - 7.24 (m, 2H), 7.14 (t, *J* = 7.8 Hz, 1H), 7.03 (dd, *J* = 7.7, 1.3 Hz, 1H), 4.57 (dt, *J* = 13.2, 3.7 Hz, 2H), 3.91 (s, 3H), 3.50 (tt, *J* = 11.2, 3.9 Hz, 1H), 3.20 (ddd, *J* = 13.7, 11.6, 2.7 Hz, 2H), 2.56 (s, 3H), 2.22 (dd, *J* = 12.9, 3.1 Hz, 2H), 1.92 - 1.77 (m, 2H); $^{13}C$ NMR (101 MHz, DMSO-$d_6$) δ 182.80, 166.27, 161.75, 156.49, 153.71, 147.57, 139.21, 137.93, 135.20, 124.08, 123.92, 122.40, 120.23, 119.79, 110.70, 109.77, 56.13, 44.44, 34.36, 29.01, 24.61.

In an embodiment, the method of synthesizing compound 40 (tert-butyl ((1-(5-(5-(1-(8-methoxyquinolin-2-yl) piperidin-4-yl)-1,2,4-oxadiazol-3-yl)pyridin-2-yl)piperidin-4-yl)methyl)carbamate) is as follows:

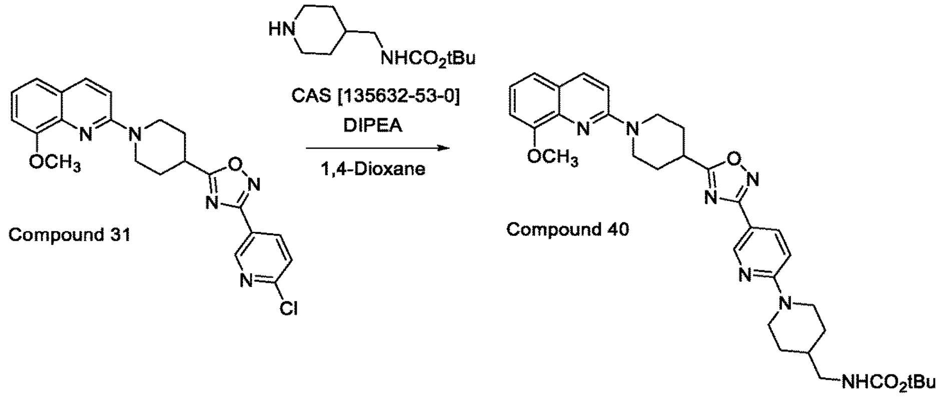


In an embodiment, preparation of compound 40 (*tert*-butyl ((1-(5-(5-(1-(8-methoxyquinolin-2-yl)piperidin-4-yl)-1,2,4-oxadiazol-3-yl)pyridin-2-yl)piperidin-4-yl)methyl)carbamate) was performed. *Tert*-Butyl (piperidin-4-ylmethyl) carbamate (1 g, 4.67 mmol) was added to a stirring dispersion of Compound 31 (200 mg, 0.47 mmol) and DIPEA (0.2 mL, 1.4 mmol) in 1,4-dioxane (6 mL) at room temperature. The mixture was allowed to stir at reflux over a duration of 20 hours. The solvent was evaporated in high vacuum and the product was purified by preparative TLC (silica gel, Petrol ether/EtOAc, 1:2). Compound 40 was isolated as a colourless solid (302 mg, 54%).

HPLC-MS $[M+H]^+$ 600.36; MP: 130-133 °C; $^1H$ NMR (400 MHz, $CDCl_3$) $\delta$ 8.84 (bs, 1H), 8.04 (dd, $J$ = 9.0, 2.4 Hz, 1H), 7.89 (d, $J$ = 9.1 Hz, 1H), 7.26 - 7.12 (m, 2H), 7.05 (d, $J$ = 9.2 Hz, 1H), 6.96 (dd, $J$ = 7.6, 1.4 Hz, 1H), 6.67 (d, $J$ = 9.0 Hz, 1H), 4.65 (bs, 1H), 4.57 (dt, $J$ = 13.6, 3.9 Hz, 2H), 4.44 (dt, $J$ = 13.9, 2.9 Hz, 2H), 4.03 (s, 3H), 3.33 - 3.18 (m, 3H), 3.08 - 3.00 (m, 2H), 2.89 (td, $J$ = 13.2, 12.8, 2.4 Hz, 2H), 2.27 (dt, $J$ = 12.2, 3.7 Hz, 2H), 2.14 - 1.99 (m, 2H), 1.85 - 1.68 (m, 3H), 1.44 (s, 9H), 1.31 - 1.19 (m, 2H); $^{13}C$ NMR (101 MHz, $CDCl_3$) $\delta$ 181.03, 166.79, 159.98, 156.77, 156.05, 153.54, 147.92, 139.23, 137.68, 136.09, 123.96, 122.28, 119.45, 111.31, 110.05, 108.75, 106.09, 56.14, 46.03, 44.84, 36.97, 34.87, 29.32, 28.41.

In an embodiment, the method of synthesizing compound 41 (tert-butyl ((1-(5-(5-(1-(8-methoxyquinolin-2-yl) piperidin-4-yl)-1,2,4-oxadiazol-3-yl)pyridin-2-yl)pyrrolidin-3-yl)methyl)carbamate) is as follows:

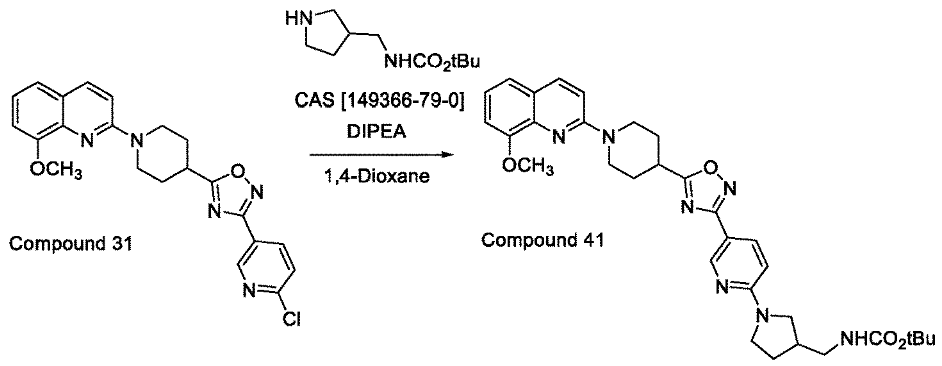


In an embodiment, preparation of compound 41 (tert-butyl (tert-butyl ((1-(5-(5-(1-(8-methoxyquinolin-2-yl) piperidin-4-yl)-1,2,4-oxadiazol-3-yl)pyridin-2-yl)pyrrolidin-3-yl)methyl)carbamate) was performed. 3-N-Boc-aminomethylpyrrolidine (700 mg, 3.56 mmol) was added to a stirring dispersion of Compound 31 (150 mg, 0.36 mmol) and DIPEA (0.1 mL, 0.72 mmol) in 1,4-dioxane (6 mL) at room temperature. The mixture was allowed to stir at reflux over a duration of 20 hours. The solvent was evaporated in high vacuum and the product was purified by column chromatography (silica gel, Petrol ether/EtOAc, 1:2). Compound 41 was isolated as a colourless solid (139 mg, 66%).

HPLC-MS $[M+H]^+$ 586.31; MP: 105-107 °C; $^1H$ NMR (400 MHz, $CDCl_3$) $\delta$ 8.87 - 8.81 (m, 1H), 8.04 (dd, $J$ = 9.0, 2.4 Hz, 1H), 7.90 (d, $J$ = 9.1 Hz, 1H), 7.26 - 7.12 (m, 2H), 7.05 (d, $J$ = 9.2 Hz, 1H), 6.96 (dd, $J$ = 7.6, 1.4 Hz, 1H), 6.40 (d, $J$ = 8.8 Hz, 1H), 4.71 (bs, 1H), 4.58 (dt, $J$ = 13.6, 3.9 Hz, 2H), 4.03 (s, 3H), 3.75 - 3.60 (m, 2H), 3.54 - 3.45 (m, 1H), 3.32 - 3.16 (m, 6H), 2.63 - 2.50 (m, 1H), 2.28 (dd, J = 13.5, 3.7 Hz, 2H), 2.19 - 2.03 (m, 3H), 1.86 - 1.72 (m, 1H), 1.45 (s, 9H); $^{13}C$ NMR (101 MHz, $CDCl_3$) $\delta$ 180.97, 167.01, 158.03, 156.78, 153.54, 148.22, 146.58, 139.23, 137.67, 135.66, 123.96, 122.27, 119.45, 110.70, 110.05, 108.74, 106.09, 56.14, 50.28, 46.25, 44.77, 43.15, 39.11, 34.87, 29.25, 28.99, 28.38.

In an embodiment, the method of synthesizing compound 42 (1-(5-(5-(1-(8-methoxyquinolin-2-yl)piperidin-4-yl)-1,2,4-oxadiazol-3-yl)pyridin-2-yl)pyrrolidin-3-yl)methanamine trifluoroacetate) is as follows:

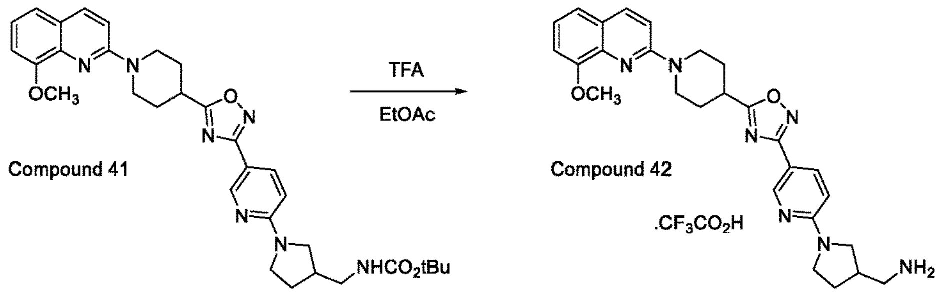


In an embodiment, preparation of compound 42 (tert-butyl (1-(5-(5-(1-(8-methoxyquinolin-2-yl)piperidin-4-yl)-1,2,4-oxadiazol-3-yl)pyridin-2-yl)pyrrolidin-3-yl)methanamine trifluoroacetate) was performed. Trifluoroacetic acid (5 mL) was added to a stirring solution of Compound 41 (100 mg, 0.17 mmol) in EtOAc (1 mL). The mixture was allowed to stir at room temperature for 1 hour. The solvent was evaporated in high vacuum and the product was triturated with diethyl ether. Compound 42 was isolated as an off-white solid (95.7 mg, 97%).

HPLC-MS $[M+2H]^{2+}$ 243.69; MP: 198-201 °C; $^1$H NMR (400 MHz, DMSO-$d_6$) δ 8.58 - 8.52 (m, 1H), 8.39 (d, *J* = 9.6 Hz, 1H), 8.31 (bs, 2H), 8.22 (dd, *J* = 9.2, 2.2 Hz, 1H), 7.63 (d, *J* = 9.6 Hz, 1H), 7.49 (dd, *J* = 7.8, 1.5 Hz, 1H), 7.46 - 7.33 (m, 2H), 6.90 (d, *J* = 9.2 Hz, 1H), 4.49 - 4.41 (m, 2H), 4.03 (s, 3H), 3.83 - 3.68 (m, 2H), 3.68 - 3.48 (m, 4H), 3.48 - 3.37 (m, 1H), 3.00 - 2.87 (m, 2H), 2.77 - 2.65 (m, 1H), 2.34 - 2.13 (m, 3H), 2.05 - 1.84 (m, 3H); $^{13}$C NMR (101 MHz, DMSO-$d_6$) δ 181.93, 165.58, 158.52, 125.21, 122.55, 120.18, 113.27, 112.64, 110.65, 96.29, 56.83, 51.43, 47.56, 46.61, 41.07, 36.70, 33.21, 32.44, 30.56, 28.93, 28.79.

In an embodiment, the method of synthesizing compound 43 ((1-(5-(5-(1-(8-methoxyquinolin-2-yl)piperidin-4-yl)-1,2,4-oxadiazol-3-yl)pyridin-2-yl)piperidin-4-yl)methanamine trifluoroacetate) is as follows:

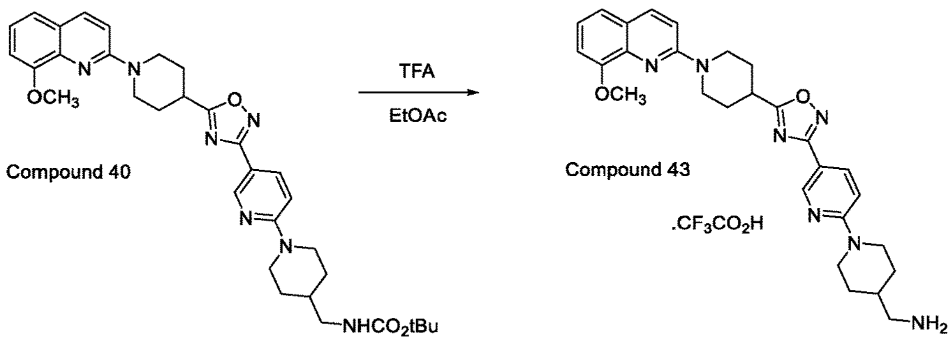


In an embodiment, preparation of compound 43 ((1-(5-(5-(1-(8-methoxyquinolin-2-yl)piperidin-4-yl)-1,2,4-oxadiazol-3-yl)pyridin-2-yl)piperidin-4-yl)methanamine trifluoroacetate) was performed. Trifluoroacetic acid (5 mL) was added to a stirring solution of Compound 40 (220 mg, 0.37 mmol) in EtOAc (1 mL). The mixture was allowed to stir at room temperature for 1 hour. The solvent was evaporated in high vacuum and the product was triturated with diethyl ether. Compound 43 was isolated as an off-white solid (280 mg, 99%).

HPLC-MS $[M+2H]^{2+}$ 250.70; MP: 45-50 °C; $^1$H NMR (400 MHz, DMSO-$d_6$) δ 8.70 - 8.65 (m, 1H), 8.26 (d, *J* = 9.5 Hz, 1H), 8.01 (dd, *J* = 9.1, 2.4 Hz, 1H), 7.81 (bs, 2H), 7.51 (d, *J* = 9.5 Hz, 1H), 7.41 (dd, *J* = 7.9, 1.3 Hz, 1H), 7.32 (t, *J* = 7.9 Hz, 1H), 7.25 (d, *J* = 7.8 Hz, 1H), 7.01 (d, *J* = 9.1 Hz, 1H), 4.52 - 4.45 (m, 4H), 3.99 (s, 3H), 3.54 (tt, *J* = 11.0, 3.9 Hz, 1H), 3.46 - 3.34 (m, 2H), 2.99 - 2.87 (m, 2H), 2.81 - 2.70 (m, 2H), 2.25 (dd, *J* = 13.8, 3.8 Hz, 2H), 1.99 - 1.85 (m, 3H), 1.80 (dd, *J* = 13.6, 3.4 Hz, 2H), 1.25 - 1.12 (m, 2H); $^{13}$C NMR (101 MHz, DMSO-$d_6$) δ 181.65, 166.41, 159.72, 158.87, 157.00, 147.17, 136.34, 132.72, 124.29, 123.01, 120.04, 117.67, 114.77, 112.36, 110.81, 107.33, 56.57, 45.86, 44.40, 44.06, 34.55, 33.61, 29.01, 28.90.

In an embodiment, the method of synthesizing compound 44 (3-(6-methoxybenzo[d]thiazol-2-yl)-5-(1-(8-methoxyquinolin-2-yl)piperidin-4-yl)-1,2,4-oxadiazole) is as follows:

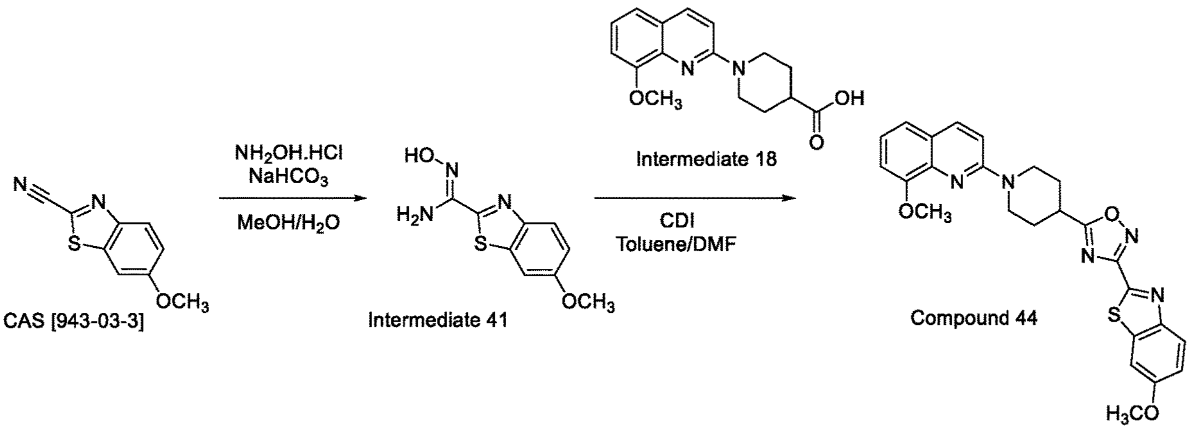


CAS [943-03-3]

Intermediate 41

Compound 44

In an embodiment, preparation of Intermediate 41 ((Z)-N'-hydroxy-6-methoxybenzo[d]thiazole-2-carboximidamide) was performed. Hydroxylamine hydrochloride (210 mg, 3.1 mmol) was slowly added to a solution of nitrile starting material (500 mg, 2.6 mmol) in a mixture of MeOH (5 mL) and water (1 mL). Sodium hydrogencarbonate (260 mg, 3.1 mmol) was added and the mixture was allowed to stir at 85 °C over a duration of 3 hours. The reaction mixture was evaporated in vacuum and the resulting residue was suspended in water and was filtered. Intermediate 41 was isolated as a colourless solid (480 mg, 82%).

In an embodiment, preparation of compound 44 (3-(6-methoxybenzo[d]thiazol-2-yl)-5-(1-(8-methoxyquinolin-2-yl)piperidin-4-yl)-1,2,4-oxadiazole) was performed. CDI (690 mg, 4.2 mmol) was added to a solution of Intermediate 18 (800 mg, 2.8 mmol) in a mixture of PhMe (12 mL) and DMF (5.5 mL) and the reaction mixture was allowed to stir at room temperature over a duration of 1 hour. Then intermediate 41 (930 mg, 4.2 mmol) was added and the reaction mixture was allowed to stir at room temperature for 30 minutes and at 90 °C over a duration of 2 hours. The solvent was evaporated in high vacuum and the resulting residue was purified by column chromatography (silica gel, DCM/MeOH 2%) and Compound 44 was isolated as a pale yellow solid (80 mg, 6%).

HPLC-MS $[M+H]^+$ 474.12; MP: 141-143 °C; $^1$H NMR (400 MHz, DMSO-$d_6$) δ 8.10 (d, $J$ = 9.0 Hz, 1H), 8.02 (d, $J$ = 9.2 Hz, 1H), 7.85 - 7.79 (m, 1H), 7.34 - 7.19 (m, 3H), 7.15 (t, $J$ = 7.9 Hz, 1H), 7.04 (dd, $J$ = 7.9, 1.3 Hz, 1H), 4.58 (dt, $J$ = 13.5, 3.8 Hz, 2H), 3.91 (s, 3H), 3.88 (s, 3H), 3.54 (tt, $J$ = 11.0, 3.9 Hz, 1H), 3.21 (ddd, $J$ = 13.7, 11.5, 2.7 Hz, 2H), 2.25 (dd, $J$ = 13.5, 3.7 Hz, 2H), 1.95 - 1.80 (m, 2H); $^{13}$C NMR (101 MHz, DMSO-$d_6$) δ 183.51, 164.09, 159.16, 156.51, 153.73, 151.41, 148.05, 139.21, 137.93, 137.23, 125.17, 123.94, 123.79, 122.41, 119.79, 117.72, 110.70, 109.77, 105.13, 56.33, 56.13, 44.41, 34.40, 28.89.

In an embodiment, the method of synthesizing compound 45 (5-(1-(8-methoxyquinolin-2-yl)piperidin-4-yl)-3-(thiophen-2-yl)-1,2,4-oxadiazole) is as follows:

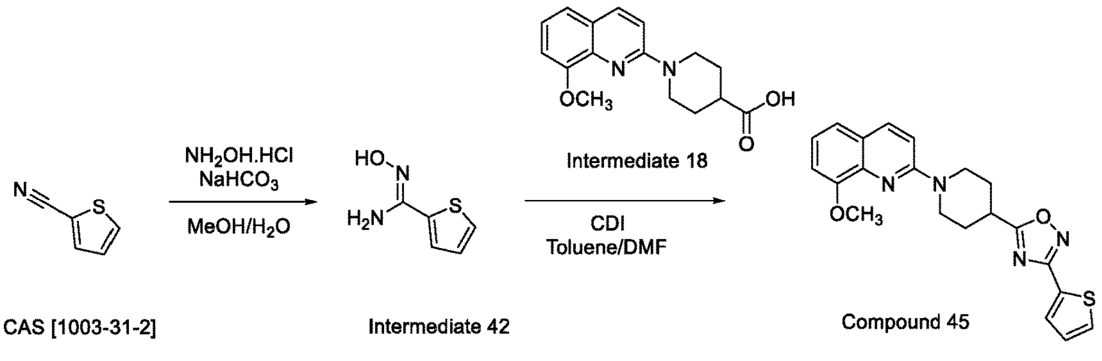


CAS [1003-31-2]

Intermediate 42

Compound 45

In an embodiment, preparation of Intermediate 42 ((Z)-N'-hydroxythiophene-2-carboximidamide) was performed. Hydroxylamine hydrochloride (380 mg, 5.5 mmol) was slowly added to a solution of nitrile starting material (500 mg, 4.5 mmol) in a mixture of MeOH (5 mL) and water (1 mL). Sodium hydrogencarbonate (460 mg, 5.5 mmol) was added and the mixture was allowed to stir at 85 °C over a duration of 18 hours. The reaction mixture was evaporated in vacuum and the resulting residue was suspended in water and was filtered. Intermediate 42 was isolated as a colourless solid (224 mg, 35%).

In an embodiment, preparation of compound 45 (5-(1-(8-methoxyquinolin-2-yl)piperidin-4-yl)-3-(thiophen-2-yl)-1,2,4-oxadiazole) was performed. CDI (420 mg, 2.6 mmol) was added to a solution of Intermediate 18 (500 mg, 1.7 mmol) in a mixture of PhMe (9 mL) and DMF (4 mL) and the reaction mixture was allowed to stir at room temperature over a

duration of 1 hour. Then intermediate 42 (380 mg, 2.6 mmol) was added and the reaction mixture was allowed to stir at room temperature for 30 minutes and at 90 °C over a duration of 2 hours. The solvent was evaporated in high vacuum and the product was recrystallized from Isop. Compound 45 was isolated as brown solid (400 mg, 60%).

HPLC-MS $[M+H]^+$ 393.14; MP: 100-105 °C; $^1H$ NMR (400 MHz, DMSO-$d_6$) δ 8.02 (d, *J* = 9.2 Hz, 1H), 7.91 - 7.76 (m, 2H), 7.33 - 7.22 (m, 3H), 7.15 (t, *J* = 7.8 Hz, 1H), 7.04 (dd, *J* = 7.8, 1.3 Hz, 1H), 4.56 (dt, *J* = 13.5, 3.8 Hz, 2H), 3.91 (s, 3H), 3.47 (tt, *J* = 11.2, 3.9 Hz, 1H), 3.18 (ddd, *J* = 13.8, 11.6, 2.7 Hz, 2H), 2.20 (dd, *J* = 13.3, 3.6 Hz, 2H), 1.90 - 1.75 (m, 2H); $^{13}C$ NMR (101 MHz, DMSO-$d_6$) δ 182.50, 164.05, 156.49, 153.71, 139.21, 137.93, 131.09, 130.29, 128.97, 127.95, 123.92, 122.39, 119.79, 110.71, 109.77, 56.13, 44.45, 34.32, 28.98.

In an embodiment, the method of synthesizing compound 46 (4-(5-(5-(1-(8-methoxyquinolin-2-yl)piperidin-4-yl)-1,2,4-oxadiazol-3-yl)pyridin-2-yl)morpholine) is as follows:

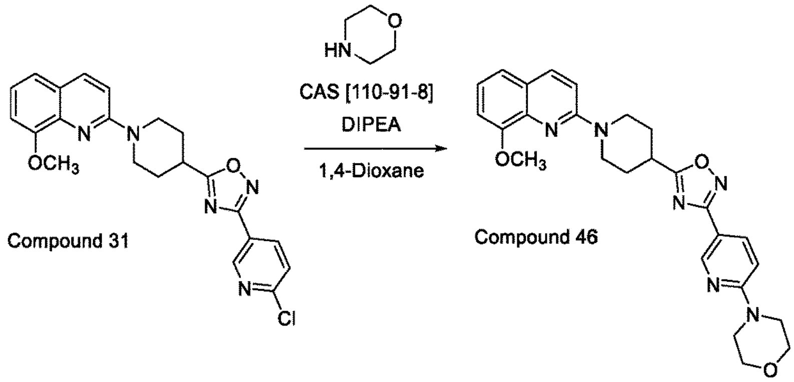

Compound 31
Compound 46

In an embodiment, preparation of compound 46 (5 4-(5-(5-(1-(8-methoxyquinolin-2-yl)piperidin-4-yl)-1,2,4-oxadiazol-3-yl)pyridin-2-yl)morpholine) was performed. Morpholine (410 mg, 4.7 mmol) was added to a stirring dispersion of Compound 31 (200 mg, 0.47 mmol) and DIPEA (0.2 mL, 0.94 mmol) in 1,4-dioxane (10 mL) at room temperature. The mixture was allowed to stir at reflux over a duration of 20 hours. The solvent was evaporated in high vacuum and the product was purified by column chromatography (silica gel, Petrol ether/EtOAc, 2:1). Compound 46 was isolated as an off-white solid (138 mg, 62%).

HPLC-MS $[M+2H]^{2+}$ 237.15; MP: 187-190 °C; $^1H$ NMR (400 MHz, $CDCl_3$) δ 8.89 - 8.83 (m, 1H), 8.11 (dd, *J* = 8.9, 2.4 Hz, 1H), 7.97 (d, *J* = 9.3 Hz, 1H), 7.25 7.22 (m, 2H), 7.08 (d, *J* = 9.3 Hz, 1H), 7.05 - 7.00 (m, 1H), 6.68 (dd, *J* = 9.0, 0.8 Hz, 1H), 4.54 (dt, *J* = 13.7, 4.0 Hz, 2H), 4.03 (s, 3H), 3.83 (dd, *J* = 5.7, 4.1 Hz, 4H), 3.63 (dd, J = 5.7, 4.1 Hz, 4H), 3.43 - 3.38 (m, 2H), 3.32 (ddd, *J* = 10.7, 6.7, 4.0 Hz, 1H), 2.33 (dd, *J* = 13.6, 3.8 Hz, 2H), 2.19 - 2.04 (m, 2H); $^{13}C$ NMR (101 MHz, $CDCl_3$) δ 180.80, 166.61, 160.05, 155.54, 152.23, 147.54, 140.76, 139.33, 136.41, 123.43, 123.40, 119.38, 112.47, 110.56, 109.91, 106.17, 66.61, 56.19, 45.56, 45.18, 34.33, 29.10.

In an embodiment, the method of synthesizing compound 47 (3-(5-(1-(8-methoxyquinolin-2-yl)piperidin-4-yl)-1,2,4-oxadiazol-3-yl)aniline hydrochloride) is as follows:

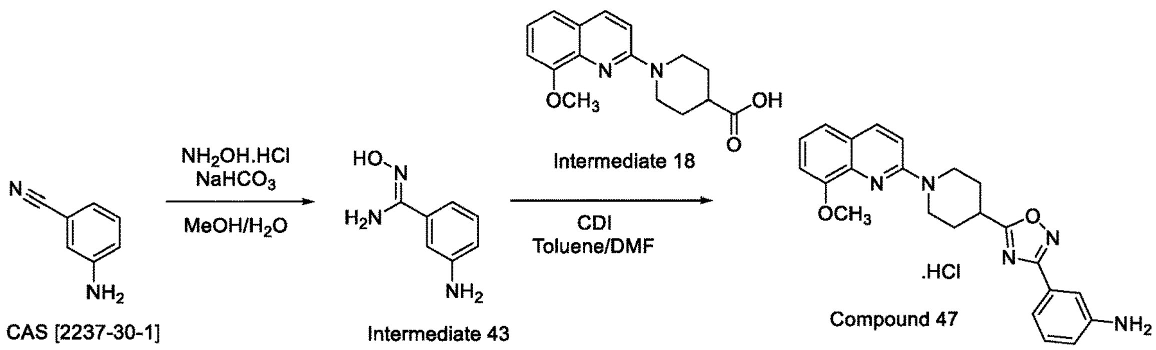

CAS [2237-30-1]
Intermediate 43
Intermediate 18
Compound 47

In an embodiment, preparation of Intermediate 43 (Z)-3-amino-N'-hydroxybenzimidamide) was performed. Hydroxylamine hydrochloride (1.4 g, 20 mmol) was slowly added to a solution of nitrile starting material (2 g, 17 mmol) in a mixture of MeOH (20 mL) and water (10 mL). Sodium hydrogencarbonate (1.7 g, 20 mmol) was added and the mixture was allowed to stir at 60 °C over a duration of 2 hours. The reaction mixture was evaporated in vacuum and the resulting residue was suspended in water and was filtered. Intermediate 43 was isolated as a pale yellow solid (1.87g, 73%).

In an embodiment, preparation of compound 47 (3-(5-(1-(8-methoxyquinolin-2-yl)piperidin-4-yl)-1,2,4-oxadiazol-3-yl)aniline hydrochloride) was performed. CDI (615 mg, 3.77 mmol) was added to a solution of Intermediate 18 (900

mg, 3.1 mmol) in a mixture of PhMe (10 mL) and DMF (2 mL) and the reaction mixture was allowed to stir at room temperature over a duration of 1 hour. Then intermediate 43 (570 mg, 3.77 mmol) was added and the reaction mixture was allowed to stir at room temperature for 30 minutes and at 90 °C over a duration of 5 hours. The solvent was evaporated in high vacuum and the resulting residue was purified by column chromatography (silica gel, Petrol ether 1:2). The resulting oil was dissolved in EtOAc (5 mL) and a 2M solution of hydrochloric acid in diethyl ether (1 mL) was added dropwise at 0 °C. The precipitate was separated by filtration and was washed with cold diethyl ether. Compound 47 was isolated as a pale brown solid (100 mg, 10%).

HPLC-MS $[M+H]^+$ 402.20; MP: 195-202 °C; $^1H$ NMR (400 MHz, DMSO-$d_6$) δ 8.41 (d, $J$ = 9.6 Hz, 1H), 7.90 (bs, 1H), 7.87 (d, $J$ = 7.9 Hz, 1H), 7.66 (d, $J$ = 9.6 Hz, 1H), 7.60 (t, $J$ = 7.9 Hz, 1H), 7.49 (dd, $J$ = 16.5, 8.0 Hz, 2H), 7.43 (t, $J$ = 7.9 Hz, 1H), 7.39 (d, $J$ = 8.0 Hz, 1H), 4.44 (dt, $J$ = 14.0, 3.9 Hz, 2H), 4.04 (s, 3H), 3.82 (bs, 2H), 3.65 (tt, $J$ = 10.9, 4.1 Hz, 1H), 3.60 - 3.54 (m, 2H), 2.32 (dd, $J$ = 13.9, 3.9 Hz, 2H), 2.06 - 1.99 (m, 2H); $^{13}C$ NMR (101 MHz, DMSO-$d_6$) δ 182.22, 167.38, 153.07, 148.92, 142.39, 131.18, 131.11, 127.78, 127.72, 125.41, 125.10, 124.54, 122.48, 120.40, 120.22, 113.47, 112.81, 56.90, 46.74, 33.17, 28.91.

In an embodiment, the method of synthesizing compound 48 (3-(6-methoxypyridin-3-yl)-5-(1-(8-methoxyquinolin-2-yl)piperidin-4-yl)-1,2,4-oxadiazole) is as follows:

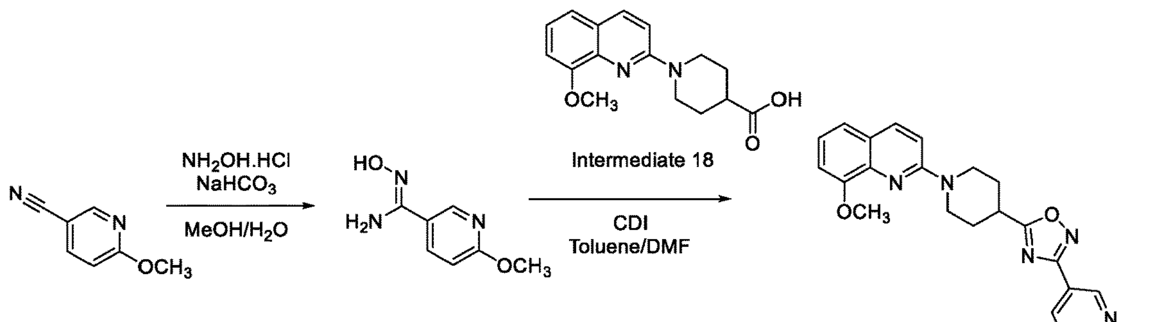


CAS [15871-85-9] Intermediate 44 Compound 48

In an embodiment, preparation of Intermediate 44 ((Z)-N'-hydroxy-6-methoxynicotinimidamide) was performed. Hydroxylamine hydrochloride (300 mg, 4.4 mmol) was slowly added to a solution of nitrile starting material (500 mg, 3.7 mmol) in a mixture of MeOH (5 mL) and water (1 mL). Sodium hydrogencarbonate (360 mg, 4.4 mmol) was added and the mixture was allowed to stir at 85 °C over a duration of 2 hours. The reaction mixture was evaporated in vacuum and the resulting residue was suspended in water and was filtered. Intermediate 44 was isolated as a colourless solid (344 mg, 54%).

In an embodiment, preparation of compound 48 (3-(6-methoxypyridin-3-yl)-5-(1-(8-methoxyquinolin-2-yl)piperidin-4-yl)-1,2,4-oxadiazole) was performed. CDI (330 mg, 2 mmol) was added to a solution of Intermediate 18 (380 mg, 1.3 mmol) in a mixture of PhMe (7 mL) and DMF (3 mL) and the reaction mixture was allowed to stir at room temperature over a duration of 1 hour. Then intermediate 44 (330 mg, 2 mmol) was added and the reaction mixture was allowed to stir at 80 °C over a duration of 24 hours. The solvent was evaporated in high vacuum and the product was recrystallized from Isop. Compound 48 was isolated as a pale brown solid (294 mg, 54%).

HPLC-MS $[M+H]^+$ 418.23; MP: 97-101 °C; $^1H$ NMR (400 MHz, DMSO-$d_6$) δ 8.82 - 8.75 (m, 1H), 8.23 (dd, $J$ = 8.7, 2.4 Hz, 1H), 8.02 (d, $J$ = 9.2 Hz, 1H), 7.33 - 7.24 (m, 2H), 7.14 (t, $J$ = 7.8 Hz, 1H), 7.09 - 6.96 (m, 2H), 4.57 (dt, $J$ = 13.5, 3.8 Hz, 2H), 3.93 (s, 3H), 3.91 (s, 3H), 3.48 (tt, $J$ = 11.2, 3.9 Hz, 1H), 3.19 (ddd, $J$ = 13.7, 11.6, 2.7 Hz, 2H), 2.21 (dd, $J$ = 13.4, 3.8 Hz, 2H), 1.91 - 1.76 (m, 2H); $^{13}C$ NMR (101 MHz, DMSO-$d_6$) δ 182.58, 166.06, 165.80, 156.48, 153.71, 146.60, 139.21, 137.99, 137.93, 123.91, 122.39, 119.79, 116.67, 111.81, 110.69, 109.77, 56.12, 54.21, 44.44, 34.36, 29.03.

In an embodiment, the method of synthesizing compound 49 (3-(5-fluoropyridin-3-yl)-5-(1-(8-methoxyquinolin-2-yl)piperidin-4-yl)-1,2,4-oxadiazole) is as follows:

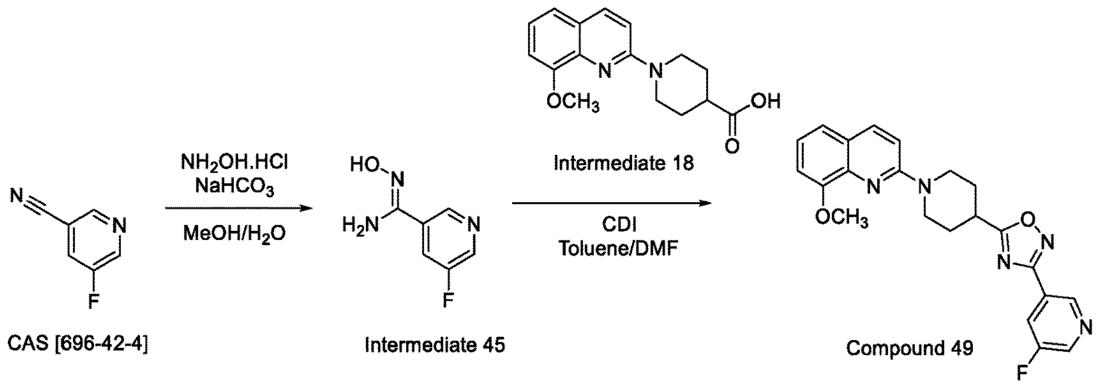


CAS [696-42-4]

Intermediate 45

Compound 49

In an embodiment, preparation of Intermediate 45 ((Z)-5-fluoro-N'-hydroxynicotinimidamide) was performed. Hydroxylamine hydrochloride (300 mg, 4.9 mmol) was slowly added to a solution of nitrile starting material (500 mg, 4 mmol) in a mixture of MeOH (5 mL) and water (1 mL). Sodium hydrogencarbonate (400 mg, 4.9 mmol) was added and the mixture was allowed to stir at 85 °C over a duration of 2 hours. The reaction mixture was evaporated in vacuum and the resulting residue was suspended in water and was filtered. Intermediate 45 was isolated as a colourless solid (400 mg, 65%).

In an embodiment, preparation of compound 49 (3-(5-fluoropyridin-3-yl)-5-(1-(8-methoxyquinolin-2-yl)piperidin-4-yl)-1,2,4-oxadiazole) was performed. CDI (400 mg, 2.5 mmol) was added to a solution of Intermediate 18 (470 mg, 1.6 mmol) in a mixture of PhMe (9 mL) and DMF (4 mL) and the reaction mixture was allowed to stir at room temperature over a duration of 1 hour. Then intermediate 45 (380 mg, 2.5 mmol) was added and the reaction mixture was allowed to stir at 80 °C over a duration of 24 hours. The solvent was evaporated in high vacuum and the product was recrystallized from Isop. Compound 49 was isolated as an off-white solid (414 mg, 62%).

HPLC-MS $[M+H]^+$ 406.21; MP: 143-152 °C; $^1H$ NMR (400 MHz, DMSO-$d_6$) δ 9.07 - 9.01 (m, 1H), 8.87 - 8.79 (m, 1H), 8.27 - 8.19 (m, 1H), 8.02 (d, *J* = 9.2 Hz, 1H), 7.34 - 7.24 (m, 2H), 7.14 (t, *J* = 7.8 Hz, 1H), 7.03 (dd, *J* = 7.7, 1.3 Hz, 1H), 4.57 (dt, *J* = 13.5, 3.8 Hz, 2H), 3.91 (s, 3H), 3.53 (tt, *J* = 11.1, 3.9 Hz, 1H), 3.21 (ddd, *J* = 13.7, 11.5, 2.7 Hz, 2H), 2.22 (dd, *J* = 13.4, 3.7 Hz, 2H), 1.94 - 1.78 (m, 2H); $^{13}C$ NMR (101 MHz, DMSO-$d_6$) δ 183.26, 165.39, 160.68, 156.48, 153.71, 144.38, 141.29, 139.21, 137.94, 123.92, 122.40, 121.76, 119.79, 110.70, 109.77, 56.13, 44.41, 34.36, 28.95.

In an embodiment, the method of synthesizing compound 50 (5-(1-(8-methoxyquinolin-2-yl)piperidin-4-yl)-3-(6-(pyrrolidin-1-yl)pyridin-3-yl)-1,2,4-oxadiazole hydrochloride) is as follows:

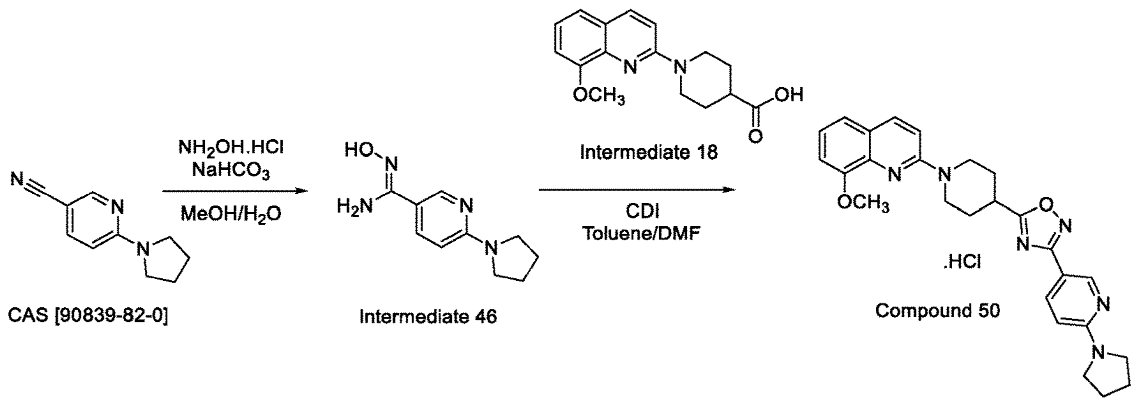


CAS [90839-82-0]

Intermediate 46

Compound 50

In an embodiment, preparation of Intermediate 46 ((Z)-N'-hydroxy-6-(pyrrolidin-1-yl)nicotinimidamide) was performed. Hydroxylamine hydrochloride (610 mg, 8.7 mmol) was slowly added to a solution of nitrile starting material (1 g, 5.8 mmol) in a mixture of MeOH (10 mL) and water (2 mL). Sodium hydrogencarbonate (731 mg, 8.7 mmol) was added and the mixture was allowed to stir at 85 °C over a duration of 2 hours. The reaction mixture was evaporated in vacuum and the resulting residue was suspended in water and was filtered. Intermediate 46 was isolated as a colourless solid (1.07 g, 89%).

In an embodiment, preparation of compound 50 (5-(1-(8-methoxyquinolin-2-yl)piperidin-4-yl)-3-(6-(pyrrolidin-1-yl)pyridin-3-yl)-1,2,4-oxadiazole hydrochloride) was performed. CDI (592 mg, 3.64 mmol) was added to a solution of Intermediate 18 (867 mg, 3.03 mmol) in a mixture of PhMe (10 mL) and DMF (2 mL) and the reaction mixture was allowed to stir at room temperature over a duration of 1 hour. Then intermediate 46 (750 mg, 3.64 mmol) was added and the reaction mixture was allowed to stir at room temperature for 30 minutes and at 80 °C over a duration of 2 hours. The solvent was

evaporated in high vacuum and the product was purified by column chromatography (silica gel, Petrol ether/EtOAc, 1:2). The resulting oil was dissolved in EtOAc (5 mL) and a 2M solution of hydrochloric acid in diethyl ether (1 mL) was added dropwise at 0 °C. The precipitate was separated by filtration and was washed with cold diethyl ether. Compound 50 was isolated as a yellow solid (212 mg, 14%).

HPLC-MS $[M+2H]^{2+}$ 229.22; MP: 139-143 °C; $^1H$ NMR (400 MHz, DMSO-$d_6$) δ 8.61 - 8.55 (m, 1H), 8.22 (d, *J* = 9.4 Hz, 1H), 8.11 (dd, *J* = 9.1, 2.3 Hz, 1H), 7.47 (d, *J* = 9.4 Hz, 1H), 7.39 (dd, *J* = 8.0, 1.3 Hz, 1H), 7.29 (t, *J* = 7.9 Hz, 1H), 7.22 (d, J = 7.8 Hz, 1H), 6.80 (d, *J* = 9.1 Hz, 1H), 4.53 - 4.47 (m, 2H), 3.98 (s, 3H), 3.57 - 3.47 (m, 5H), 3.43 - 3.32 (m, 2H), 2.30 - 2.19 (m, 2H), 2.01 - 1.88 (m, 6H); $^{13}C$ NMR (101 MHz, DMSO-$d_6$) δ 181.92, 180.45, 166.01, 157.85, 151.88, 136.89, 126.77, 125.86, 123.17, 120.00, 110.20, 56.51, 47.80, 45.62, 33.74, 29.00, 25.32.

In an embodiment, the preferred compounds of general formula I are as follows:

5-(1-(8-methoxyquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-(trifluoromethyl)phenyl)-1,2,4-oxadiazole hydrochloride;
3-(3,5-dimethoxyphenyl)-5-(1-(8-methoxyquinolin-2-yl)piperidin-4-yl)-1,2,4-oxadiazole;
5-(1-(8-methoxyquinolin-2-yl)piperidin-4-yl)-3-(3-(trifluoromethyl)phenyl)-1,2,4-oxadiazole;
5-(1-(8-methoxyquinolin-2-yl)piperidin-4-yl)-3-(pyridin-3-yl)-1,2,4-oxadiazole hydrochloride;
5-(1-(8-methoxyquinolin-2-yl)piperidin-4-yl)-3-(6-(trifluoromethyl)pyridin-3-yl)-1,2,4-oxadiazole;
5-(1-(8-chloroquinolin-2-yl)piperidin-4-yl)-3-(pyridin-3-yl)-1,2,4-oxadiazole hydrochloride;
5-(1-(6-methoxyquinolin-2-yl)piperidin-4-yl)-3-(pyridin-3-yl)-1,2,4-oxadiazole;
4-(5-(1-(8-methoxyquinolin-2-yl)piperidin-4-yl)-1,2,4-oxadiazol-3-yl)phenol;
3-(pyridin-3-yl)-5-(1-(6-(pyridin-4-yl)quinolin-2-yl)piperidin-4-yl)-1,2,4-oxadiazole;
3-(6-chloropyridin-3-yl)-5-(1-(8-methoxyquinolin-2-yl)piperidin-4-yl)-1,2,4-oxadiazole hydrochloride;
5-(1-(8-methoxyquinolin-2-yl)piperidin-4-yl)-3-(6-(piperidin-1-yl)pyridin-3-yl)-1,2,4-oxadiazole;
5-(1-(8-methoxyquinolin-2-yl)piperidin-4-yl)-3-(6-(piperazin-1-yl)pyridin-3-yl)-1,2,4-oxadiazole;
2-((5-(5-(1-(8-methoxyquinolin-2-yl)piperidin-4-yl)-1,2,4-oxadiazol-3-yl)pyridin-2-yl)amino)ethan-1-ol hydrochloride;
5-(1-(8-methoxyquinolin-2-yl)piperidin-4-yl)-3-(6-(pyrrolidin-1-yl)pyridin-3-yl)-1,2,4-oxadiazole hydrochloride; or
a pharmaceutically acceptable salt, hydrate, solvate, N-oxide, stereoisomer, stereoisomer, in particular diastereoisomer, enantiomer or atropisomers, or mixtures thereof, polymorph or ester thereof.

## Example 2

In an embodiment, *in vitro* cytotoxicity assay was carried out. *In vitro* cytotoxicity assay was performed in HepG2 cell line using a tetrazolium reduction assay for cell viability evaluation (MTS reduction as described by Cory A. et al (Cancer Commun. 1991, 3 (7), 207-212)). The assay was performed in 96-well plates for 48 h at 37 °C and 5% $CO_2$ in incubation medium (low glucose DMEM with Glutamax, 10% FBS, 1% Pen/Strep and DMSO). Cells were exposed to 8 different compound concentrations in successive dilutions (usually, base 2), plus a positive control (a compound in a concentration known to decrease cell viability above 50%) and a negative control (incubation medium). Six replicate wells were assayed for each condition. Compound stock solutions were prepared in DMSO at 20 mM, whenever possible, filter sterilized, aliquoted and stored at - 20 °C. The maximum compound concentrations tested depend on its solubility in incubation medium, and on the maximum DMSO percentage allowable without loss of cell viability. At the end of the incubation period, tetrazolium reagent was added (CellTiter 96° $AQ_{ueous}$ Solution from Promega); cells were incubated for another 3 hours in order to promote tetrazolium reduction to formazan by viable cells. The amount of soluble formazan produced was evaluated by 490 nm absorbance reading in a spectrophotometer plate reader. The average absorbance from the negative control wells corresponded to 100% cell viability; the absorbance obtained from each test condition were then transformed into a cell viability percentage - these results were plotted against compound concentration and a non-linear regression fit was made. Whenever possible, $IC_{50}$ was calculated as the concentration of compound that reduces cell viability by 50%.

## Example 3

In an embodiment, *Mycobacterium tuberculosis* susceptibility assay REMA was carried out. *M. tuberculosis* susceptibility was accessed by exposing susceptible H37Rv *M. tuberculosis,* in particular *M. tuberculosis* strain ATCC 25618 (for detail https://www.uniprot.org/taxonomy/83332, or order in https://www.szabo-scandic.com/en/recombinant-mycobacterium-tuberculosis-probable-cutinase-rv1984c-rv1984c), to the different compounds and evaluating its growth inhibition by REMA (resazurin microtiter assay) method based on Palomino JC et al (Mycobacterium tuberculosis Antimicrob. Agents Chemother. 2002, 46, 8, 2720-2722). The assay was performed in 96-well plates for 6 days at 37 °C in Middlebrook 7H9 medium. Cells were exposed to 8 different compound concentrations in two-fold serial dilutions;

incubation medium was used as negative control and 2 positive controls were accessed: Rifampicin in a concentration known to inhibit growth and Moxifloxacin in 8 different concentrations, from no growth inhibition to full growth inhibition. Compound stock solutions were prepared in DMSO in a concentration at least 40X higher than the maximum concentration tested. At the end of the incubation period, resazurin was added and cells were incubated for another 2 days in order to promote resazurin colour change from blue to pink every time growth has occured . Colours were recorded and the lowest concentration in which no colour change was observed (the last blue well) was assumed to be the minimum inhibitory concentration (MIC) for that compound in *M. tuberculosis.*

**Example 4**

In an embodiment, metabolic stability assay was carried out. Metabolic stability was assessed by incubating test compounds in 96-well plates in 5 aliquots of 40 μL each (one for each time point) in liver microsomal incubation medium. This medium contained $MgCl_2$ (3.3 mM), NADPH- cofactor system (NADPH (3 mM), glucose-6-phosphate (5.3 mM), glucose-6-phosphate dehydrogenase (0.67 units/ml)) and 0.42 mg of liver microsomal protein per ml in phosphate buffer (100 mM, pH 7.4). Control incubations were performed replacing the NADPH-cofactor system by phosphate buffer. Test compounds (1 μM, final solvent concentration 1.6 %) were incubated with microsomes at 37 °C and 100 rpm orbital shaking. Imipramine and Propranolol were incubated in parallel as reference compounds. Each reaction was performed in duplicate. Five time points over a duration 60 minutes were analysed. The reactions were stopped by adding 12 volumes of 90% acetonitrile in water to incubation aliquots, followed by protein sedimentation by centrifugation. Supernatants were analysed using a Shimadzu VP HPLC system with a reverse phase HPLC column, coupled with tandem mass spectrometer API 3000 (PE Sciex). The TurbolonSpray source was used in both positive and negative ion modes. Acquisition and analysis of the data were performed using Analyst 1.5.2 software (PE Sciex). The elimination constant ($k_{el}$), half-life ($t_{1/2}$) and intrinsic clearance ($Cl_{int}$) were determined in plot of ln(AUC) *versus* time, using linear regression analysis for each test and reference compound.

**Example 5**

In an embodiment, distribution coefficient (LogD) determination assay was carried out. Test compounds were incubated in Eppendorf-type polypropylene microtubes in triplicates: a 5 μL aliquot of a 10 mM DMSO stock of each test compound were added into the previously mutually saturated mixture containing 500 μL of phosphate buffer saline (pH 7.4) and 500 μL of octanol. The solution was allowed to mix in a rotator for 1 hour at 30 rpm. Phase separation was assured by centrifugation. The octanol phase was diluted 100-fold with 40% acetonitrile in water for analysis, and aqueous phase was analysed without dilution. Mebendazole was used as a reference compound. All samples (both phases) were analysed in a Shimadzu VP HPLC system with a reverse phase column, coupled with tandem mass spectrometer API 3000 (PE Sciex). The TurbolonSpray ion source was used in positive ion mode. Acquisition and analysis of the data were performed using Analyst 1.5.2 software. Distribution coefficients of the tested compounds were calculated as the logarithm of the partition ratios (D) in both solvents, according to the equation below.

$$D = \frac{100 \,.\, S_O}{S_P}$$

where: $S_O$ - peak area of the compound in octanol phase

$S_P$ - peak area of the compound in phosphate buffer saline phase

**Example 6**

In an embodiment, pKa determination assay was carried out. The tendency of a compound to donate a proton is measured as its acid ionization constant (dissociation constant), or Ka. A more practical scale of representing acidity is pKa which is the negative logarithm of the Ka (pKa = -log Ka). The pKa determinations of the test compounds and 3 reference compounds were assessed by potentiometric titration, in accordance with the technical protocols for pKa measurement provided by Pion Inc. and Sirius Analytical Inc. Briefly, compounds pKa were determined by pH-metric method based on potentiometric acid-base titration at 25 °C. The test and reference compounds were dissolved in acidified MeOH-water (1:4) solution of NaCl (150 mM, pH 2) and slowly titrated with 10 mM NaOH MeOH-water (1:4) solution. The recorded pH of the solution as a function of NaOH volume used during the titration were used to construct the titration curve. Titration of acidified NaCl solution in absence of any compound was used for blank plotting. Buffering capacity was calculated in each

point of titration curve as the ratio of the NaOH flow (constant) to the pH rise velocity. The pKa value is determined from resulting plot of buffering capacity *versus* pH as the maximum of buffering capacity. pH-Metric method allows to measure pKa's in range between 2 and 12. Acquisition and analysis of the data were performed using SmartLoggerII1.0.14 software (Beckman Coulter). Data analysis was done using GraphPad Prism 5.01 and Excel 2010 software.

**Example 7**

In an embodiment, stability in human plasma (PS) assay was carried out. Test compounds (1 μM, final DMSO concentration 1%) were incubated in 5 aliquots of 70 μL each (one for each time point), in duplicates, at 37 °C with 100 rpm orbital shaking. Five time points over a duration 120 minutes have been analysed. Two reference compounds were analysed in parallel. The reactions were stopped by the addition of 420 μL of acetonitrile-water mixture (90:10) and subsequent plasma proteins sedimentation were achieved by centrifugation. Supernatants were analysed in a Shimadzu VP HPLC system with a reverse phase column, coupled with a tandem mass spectrometer API 3000 (PE Sciex). Both the positive and negative ion modes of the TurbolonSpray ion source were used. Acquisition and analysis of the data were performed using Analyst 1.5.2 software (PE Sciex). The percentage of the test compounds remaining after incubation in plasma and their half-lives ($T_{1/2}$) were calculated.

**Example 8**

In an embodiment, human plasma protein binding (PPB) assay was carried out. The assay was performed in a multiple-use 96-well dialysis unit (HTD96b dialyzer). Each individual well unit consisted of 2 chambers separated by a vertically aligned dialysis membrane of predetermined pore size (MWCO 12-14 kDa). 120 μl of non-diluted plasma spiked with the compound (1 μM, 1% final DMSO concentration) was added to one chamber and the same volume of phosphate buffer saline pH 7.4 to the other chamber. HTD96b dialyzer was covered with adhesive sealing film and incubated at 37 °C and 100 rpm orbital shaking for 5 hours. Verapamil was also evaluated in parallel as reference compound. For sample preparation, an aliquot of the content of each chamber was mixed with the same volume of the blank opposite matrix. In order to define non-specific loss of the compound during this assay, standard solution was created by mixing an aliquot of spiked plasma with blank buffer without dialysis. Samples were diluted 10-fold with 100% acetonitrile with subsequent plasma proteins sedimentation by centrifugation. Supernatants were analysed using a Shimadzu VP HPLC system with a reverse phase column, coupled with a tandem mass spectrometer API 3000 (PE Sciex). The both positive and negative ion modes of the TurbolonSpray ion source were used. Acquisition and analysis of the data were performed using Analyst 1.5.2 software (PE Sciex).

The percentage of plasma protein bounded compound and its recovery were calculated using the equations below:

$$\text{Protein binding} = \left(1 - \frac{\text{Peak area in buffer}}{\text{Peak area in plasma}}\right) - 100\%$$

$$\text{Recovery} = \left(\frac{\text{Peak area in buffer} + \text{Peak area in plasma}}{\text{Peak area in standard solution}}\right) - 100\%$$

The percentage of plasma protein bounded compound and its recovery were calculated using the values obtained for each compound in the quantification of the 3 samples (in buffer, plasma and standard solution).

**Example 9**

In an embodiment, PAMPA assay was carried. All steps of PAMPA assay were carried out according to piON Inc. PAMPA Explorer™ Manual. The main principle of the assay is the compound incubation in the donor chamber with aqueous buffer, which is separated from the acceptor chamber, with another buffer, by a phospholipid or hydrocarbon membrane fixed on a filter support. After the test, concentrations in the corresponding donor and acceptor chambers are measured and permeability is calculated. GIT model was simulated using GIT-0 phospholipid mix. Verapamil and quinidine (high permeability) and ranitidine (low permeability) were used as reference compounds. All compounds were tested in quadruplicates. Prisma HT buffer (pH 7.4) containing 50 μM test compound (final 0.5% DMSO concentration) was added into the donor chamber. Acceptor Sink Buffer was added into each acceptor chamber. Incubation was done at room temperature for 4 hours without stirring. After incubation, aliquots from both chambers were transferred to optic UV-Vis

plates and absorbance was recorded in the range of 202-500 nm in 4 nm steps. Compounds with low UV-Vis signal were evaluated by LC-MS/MS (using a Shimadzu VP HPLC system with a reverse phase HPLC column, coupled with tandem mass spectrometer API 3000 (PE Sciex) and TurbolonSpray ion source used in positive ion mode; acquisition and analysis of the data were performed using Analyst 1.5.2 software (PE Sciex)).

The apparent permeability coefficient was calculated using the formula below.

$$logP_{app} = log_{10}\left(-\frac{2.303 \times V_D}{2 \times Area \times Time}\right) \times log_{10}\left(1 - 2 \times \frac{[drug]_{acc}}{[drug]_d}\right)$$

***$V_D$*-** volume of transport buffer in donor well (0.3 ml);
***Area* -** surface area of the lipids in the insert (effective growth area of the insert - 0.3 sq.cm);
***Time* -** time of the assay, seconds;
***$[drug]_{acr}$*** - final OD of test compound in acceptor wall;
***$[drug]_d$*-** starting OD of test compound in a donor well.

The apparent permeability coefficient was calculated for each compound ($logP_{app}$).

In an embodiment, the compounds of the present disclosure, when tested in cytotoxicity assay, typically have IC50 value not less than 50 μM.

In an embodiment, compounds of the disclosure, when tested in susceptibility assay described in example 3 may typically have MIC lower than 31.25 μM. Compounds of the disclosure when tested in assay described in example 3 may have MIC between 1.95 μM and 31.25 μM.

In an embodiment, the compounds of the present disclosure were tested in assays outlined in examples 2 and 3 as described above. Table 1 shows the results obtained:

**Table 1 -** REMA and $IC_{50}$ results for selected compounds

| Compound Number | Name | REMA MIC | $IC_{50}$ |
|---|---|---|---|
| 3 | 5-(1-(8-methoxyquinolin-2-yl)piperidin-4-yl)-3-(4-(trifluoromethoxy) phenyl)-1,2,4-oxadiazole | +++ | ++++ |
| 5 | 5-(1-(8-methoxyquinolin-2-yl)piperidin-4-yl)-3-(4-(trifluoromethyl) phenyl)-1,2,4-oxadiazole hydrochloride | +++ | +++ |
| 6 | 3-(3,5-dimethoxyphenyl)-5-(1-(8-methoxyquinolin-2-yl)piperidin-4-yl)-1,2,4-oxadiazole | +++ | ++++ |
| 7 | 5-(1-(8-methoxyquinolin-2-yl)piperidin-4-yl)-3-(3-(trifluoromethyl) phenyl)-1,2,4-oxadiazole | ++ | ++++ |
| 10 | 5-(1-(8-methoxyquinolin-2-yl)piperidin-4-yl)-3-(pyridin-3-yl)-1,2,4-oxadiazole hydrochloride | ++ | +++ |
| 11 | 5-(1-(8-methoxyquinolin-2-yl)piperidin-4-yl)-3-(6-(trifluoromethyl) pyridin-3-yl)-1,2,4-oxadiazole | ++++ | ++ |
| 18 | 5-(1-(8-chloroquinolin-2-yl)piperidin-4-yl)-3-(pyridin-3-yl)-1,2,4-oxadiazole hydrochloride | ++ | +++ |
| 21 | 5-(1-(6-methoxyquinolin-2-yl)piperidin-4-yl)-3-(pyridin-3-yl)-1,2,4-oxadiazole | ++++ | ++++ |
| 24 | 4-(5-(1-(8-methoxyquinolin-2-yl)piperidin-4-yl)-1,2,4-oxadiazol-3-yl)phenol | +++ | ++++ |
| 27 | 3-(pyridin-3-yl)-5-(1-(6-(pyridin-4-yl)quinolin-2-yl)piperidin-4-yl)-1,2,4-oxadiazole | ++++ | ++++ |
| 31 | 3-(6-chloropyridin-3-yl)-5-(1-(8-methoxyquinolin-2-yl)piperidin-4-yl)-1,2,4-oxadiazole hydrochloride | ++ | ++ |
| 33 | 5-(1-(8-methoxyquinolin-2-yl)piperidin-4-yl)-3-(6-(piperidin-1-yl) pyridin-3-yl)-1,2,4-oxadiazole | ++++ | ++++ |

(continued)

| **Compound Number** | **Name** | **REMA MIC** | **$IC_{50}$** |
|---|---|---|---|
| 34 | 5-(1-(8-methoxyquinolin-2-yl)piperidin-4-yl)-3-(6-(piperazin-1-yl) pyridin-3-yl)-1,2,4-oxadiazole | ++ | + |
| 35 | 2-((5-(5-(1-(8-methoxyquinolin-2-yl)piperidin-4-yl)-1,2,4-oxadiazol-3-yl)pyridin-2-yl)amino)ethan-1-ol hydrochloride | ++ | ++++ |
| 50 | 5-(1-(8-methoxyquinolin-2-yl)piperidin-4-yl)-3-(6-(pyrrolidin-1-yl) pyridin-3-yl)-1,2,4-oxadiazole hydrochloride | +++ | ++++ |

In **Table 1,** the results of REMA and cytotoxicity are set out as follows:

| | ++ | +++ | ++ | + |
|---|---|---|---|---|
| MTB growth REMA (μM) | < 7.8 | 7.8- 15.6 | 15.6-31.5 | 31.5-62.5 |
| $IC_{50}$ (μM) | > 100 | 50-100 | 20-50 | < 20 |

**Table 2** -REMA and $IC_{50}$ results for the selected compounds

| Compound Number | Name | REMA MIC | $IC_{50}$ |
|---|---|---|---|
| 3 | 5-(1-(8-methoxyquinolin-2-yl)piperidin-4-yl)-3-(4-(trifluoromethoxy) phenyl)-1,2,4-oxadiazole | +++ | ++++ |
| 6 | 3-(3,5-dimethoxyphenyl)-5-(1-(8-methoxyquinolin-2-yl)piperidin-4-yl)-1,2,4-oxadiazole | +++ | ++++ |
| 21 | 5-(1-(6-methoxyquinolin-2-yl)piperidin-4-yl)-3-(pyridin-3-yl)-1,2,4-oxadiazole | ++++ | ++++ |
| 24 | 4-(5-(1-(8-methoxyquinolin-2-yl)piperidin-4-yl)-1,2,4-oxadiazol-3-yl) phenol | +++ | ++++ |
| 27 | 3-(pyridin-3-yl)-5-(1-(6-(pyridin-4-yl)quinolin-2-yl)piperidin-4-yl)-1,2,4-oxadiazole | ++++ | ++++ |
| 33 | 5-(1-(8-methoxyquinolin-2-yl)piperidin-4-yl)-3-(6-(piperidin-1-yl)pyridin-3-yl)-1,2,4-oxadiazole | ++++ | ++++ |
| 51 | 5-(1-(8-methoxyquinolin-2-yl)piperidin-4-yl)-3-(6-(pyrrolidin-1-yl)pyridin-3-yl)-1,2,4-oxadiazole hydrochloride | +++ | ++++ |

In the **table 2,** the results of REMA and cytotoxicity are set out as follows:

| | ++++ | +++ | ++ | + |
|---|---|---|---|---|
| MTB growth REMA (μM) | < 7.8 | 7.8- 15.6 | 15.6-31.5 | 31.5-62.5 |
| $IC_{50}$(μM) | > 100 | 50-100 | 20-50 | < 20 |

In an embodiment, the compounds of the present disclosure have been shown to inhibit the growth of *Mycobacterium tuberculosis* in a standard REMA assay (Palomino JC et al, Mycobacterium tuberculosis Antimicrob. Agents Chemother. 2002, 46, 8, 2720-2722) with minimum inhibitory concentration (MIC) of not more than 31.5 μM. Surprisingly, the compounds of the present disclosure presented MIC not more than 15.6 μM. Even more surprising, the embodiments for better results of the present disclosure have MIC of not more than 7.8 μM. The compounds of the disclosure have also been found to be non-cytotoxic for HepG2 cell line, metabolically stable in human microsomes, stable in plasma and relatively permeable under gastrointestinal tract parallel artificial membrane. LogD and pKa, have been described. The compounds disclosed are promising antimycobacterial agents.

In an embodiment, the compounds of the disclosure were tested in assay outlined in example 4 as described above and the following results were obtained.

**Table 3 -** $Cl_{int}$ and $t_{1/2}$ results for selected compound

| Compound Number | Name | $Cl_{int}$ (μl/min/mg) | $t_{1/2}$ (min) |
|---|---|---|---|
| 3 | 5-(1-(8-methoxyquinolin-2-yl)piperidin-4-yl)-3-(4-(trifluoromethoxy)phenyl)-1,2,4-oxadiazole | 9.2 | 182 |
| n.d. = not determined | | | |

In an embodiment, the compounds of the disclosure were tested in assay outlined in example 5, 6, 7, 8 and 9 as described above and the following results were obtained:

**Table 4 -** Further biological data for selected compound

| Compound Number | Name | $t_{1/2}$ (min) hPS | hPPB | GIT PAMPA log [$10^{-6}$ cm/s] | LogD | pKa |
|---|---|---|---|---|---|---|
| 3 | 5-(1-(8-methoxyquinolin-2-yl)piperidin-4-yl)-3-(4-(trifluoromethoxy)phenyl)-1,2,4-oxadiazole | >240 | ≥99.9 | -4.8 | 5.34 | 3.9 |
| n.d.-Not determined. | | | | | | |

The term "comprising" whenever used in this document is intended to indicate the presence of stated features, integers, steps, components, but not to preclude the presence or addition of one or more other features, integers, steps, components or groups thereof.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

The disclosure should not be seen in any way restricted to the embodiments described and a person with ordinary skill in the art will foresee many possibilities to modifications thereof. The embodiments described above are combinable.

The following claims further set out particular embodiments of the disclosure.

## Claims

1. Compound of general formula I or a pharmaceutically acceptable salt, hydrate, solvate, N-oxide, stereoisomer, diastereoisomer, enantiomer, atropisomer, polymorph or ester thereof:

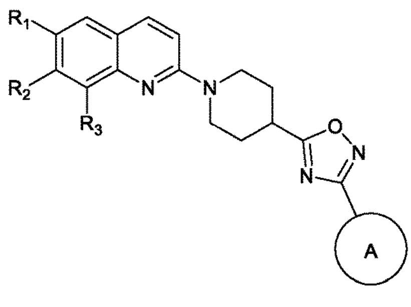

Formula I

wherein

$R_1$, $R_2$, $R_3$ and ring A are independently selected from each other;
Ring A is a substituted or unsubstituted ring selected from pyridin-2-yl, pyridin-3-yl, pyridin-4-yl, pyrazin-2-yl, 1H-pyrrolo[2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Halogen, $C_1$-$C_6$ alkoxy, or unsubstituted heteroaryl;

$R_2$ is selected from H or OH;
$R_3$ is selected from H, Halogen, $C_1$-$C_6$alkoxy, or unsubstituted heteroaryl;
and 'pharmaceutically acceptable esters' of compounds are compounds in which one or more carboxyl groups (e.g. -COOH) are modified by reaction with an alcoholic substituent A-OH so as to yield -COOA groups, wherein A is. $C_1$-$C_6$ alkyl.

**2.** Compounds of the formula I according to claim 1, wherein

Ring A is a substituted or unsubstituted ring selected from pyridin-2-yl, pyridin-3-yl, pyridin-4-yl, pyrazin-2-yl, 1H-pyrrolo[2,3-b]pyridine-5-yl, furan-2-yl, 6-methoxybenzo[d]thiazol-2-yl, thiophen-2-yl, 3-pyridine 1-oxide, pyrimidin-2-yl or phenyl;
$R_1$ is selected from H, Cl, $OCH_3$ or unsubstituted heteroaryl;
$R_2$ is selected from H or OH;
$R_3$ is selected from H, Cl, $OCH_3$ or unsubstituted heteroaryl.

**3.** Compounds according to any of the claims 1 - 2, wherein

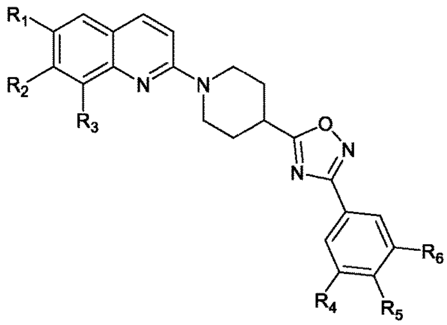
Formula II

$R_1$, $R_2$, $R_3$, $R_4$, $R_5$ and $R_6$ are independently selected from each other;
$R_1$ is selected from H, Cl, $OCH_3$ or pyridine-4-yl;
$R_2$ is selected from H or OH;
$R_3$ is selected from H, Cl, $OCH_3$ or pyridine-4-yl;
$R_4$ is selected from H, $OCH_3$, $CF_3$, $OCF_3$, $NO_2$ or $NH_2$;
$R_5$ is selected from H, $OCH_3$, $CF_3$, $OCF_3$, F, $NO_2$ or OH;
$R_6$ is selected from H, $OCH_3$, $CF_3$ or $OCF_3$.

**4.** Compound according to any of the claims 1 -2, wherein

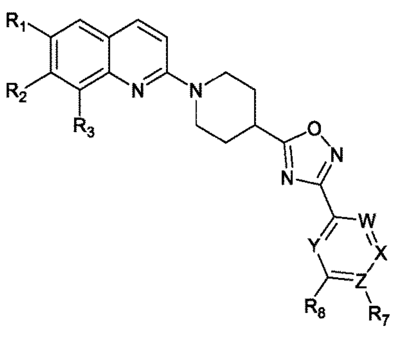
Formula III

$R_1$, $R_2$, $R_3$, $R_7$, $R_8$ are independently selected from each other;
X, Y, Z and W are independently selected from C or N;
$R_1$ is selected from H, Cl, $OCH_3$ or pyridine-4-yl;
$R_2$ is selected from H or OH;
$R_3$ is selected from H, Cl, $OCH_3$ or pyridine-4-yl;
$R_7$ is selected from H, $OCH_3$, $CF_3$, $NHCH_3$, $NH_2$, Cl, piperidinyl, piperazinyl, morpholinyl, pyrrolidinyl, $NH(CH_2)OH$ or $CH_3$;

$R_8$ is selected from H, F or OH.

**5.** Compounds according to any of the claims 1 -2, wherein

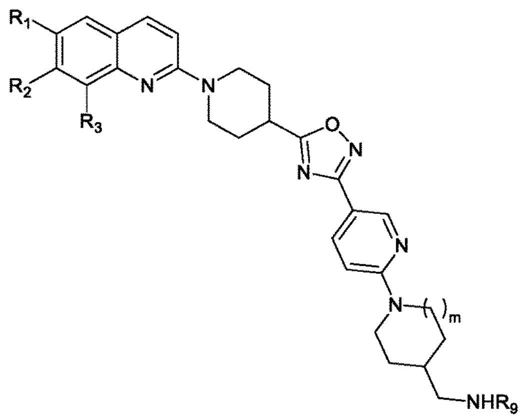

Formula IV

$R_1$ is selected from H, Cl, $OCH_3$ or pyridine-4-yl;
$R_2$ is selected from H or OH;
$R_3$ is selected from H, Cl, $OCH_3$ or pyridine-4-yl;
m is selected from 0 or 1;
$R_9$ is selected from H or tert-butyl carbamate.

**6.** Compounds according to any of the claims 1 -2, wherein

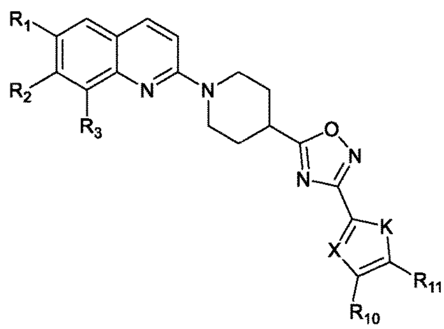

Formula V

$R_1$ is selected from H, Cl, $OCH_3$ or pyridine-4-yl;
$R_2$ is selected from H or OH;
$R_3$ is selected from H, Cl, $OCH_3$ or pyridine-4-yl;
X is C or N;
K is S or O;
$R_{10}$ and $R_{11}$ are selected from H or attached together with carbons to form 6-methoxybenzo[d]thiazol-2-yl.

**7.** Compound according to claim 3, wherein $R_3$ is H or $OCH_3$.

**8.** Compound according to claim 3, wherein $R_4$ is H, $OCH_3$ or $CF_3$.

**9.** Compound according to claim 3, wherein $R_5$ is selected from H, $CF_3$, $OCF_3$ or OH.

**10.** Compound according to claim 3, wherein $R_6$ is selected from H, $OCH_3$ or $CF_3$.

**11.** Compound according to claim 4, wherein X is N.

**12.** Compound according to claim 4, wherein $R_1$ is H, Cl, $OCH_3$ or pyridine-4-yl.

**13.** Compound according to claim 4, wherein $R_3$ is H, Cl, or $OCH_3$.

**14.** Compound according to claim 4, wherein $R_7$ is H, $CF_3$, Cl, piperidinyl, piperazinyl, pyrrolidinyl or $NH(CH_2)OH$.

**15.** Compound according to any of the previous claims, wherein the salt is a hydrochloride or a trifluoroacetate.

**16.** Compound according to any of the previous claims, wherein the compound is:

5-(1-(6-chloroquinolin-2-yl)piperidin-4-yl)-3-phenyl-1,2,4-oxadiazole;
5-(1-(6-chloroquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-(trifluoromethoxy)phenyl)-1,2,4-oxadiazole;
5-(1-(8-chloroquinolin-2-yl)piperidin-4-yl j-3-(4-(trifluoromethoxy)phenyl)-1,2,4-oxadiazole;
5-(1-(8-methoxyquinolin-2-yl)piperidin-4-yl)-3-(4-(trifluoromethyl)phenyl)-1,2,4-oxadiazole hydrochloride;
3-(3,5-dimethoxyphenyl)-5-(1-(8-methoxyquinolin-2-yl)piperidin-4-yl)-1,2,4-oxadiazole;
5-(1-(8-methoxyquinolin-2-yl)piperidin-4-yl)-3-(3-(trifluoromethyl)phenyl)-1,2,4-oxadiazole;
3-(3,5-bis(trifluoromethyl)phenyl)-5-(1-(8-methoxyquinolin-2-yl)piperidin-4-yl)-1,2,4-oxadiazole;
5-(1-(8-methoxyquinolin-2-yl)piperidin-4-yl)-3-(pyridin-4-yl)-1,2,4-oxadiazole;
5-(1-(8-methoxyquinolin-2-yl)piperidin-4-yl)-3-(pyridin-3-yl)-1,2,4-oxadiazole hydrochloride;
5-(1-(8-methoxyquinolin-2-yl)piperidin-4-yl)-3-(6-(trifluoromethyl)pyridin-3-yl)-1,2,4-oxadiazole;
3-(3-methoxyphenyl)-5-(1-(8-methoxyquinolin-2-yl)piperidin-4-yl)-1,2,4-oxadiazole hydrochloride;
5-(1-(8-methoxyquinolin-2-yl)piperidin-4-yl)-3-(3-(trifluoromethoxy)phenyl)-1,2,4-oxadiazole;
3-(4-methoxyphenyl)-5-(1-(8-methoxyquinolin-2-yl)piperidin-4-yl)-1,2,4-oxadiazole;
5-(1-(6-chloroquinolin-2-yl)piperidin-4-yl)-3-(pyridin-3-yl)-1,2,4-oxadiazole hydrochloride;
5-(1-(8-methoxyquinolin-2-yl)piperidin-4-yl)-3-(pyrazin-2-yl)-1,2,4-oxadiazole hydrochloride;
7-hydroxy-8-methoxy-2-(4-(3-(pyridin-3-yl)-1,2,4-oxadiazol-5-yl)piperidin-1-yl)quinoline 1-oxide;
5-(1-(8-chloroquinolin-2-yl)piperidin-4-yl)-3-(pyridin-3-yl)-1,2,4-oxadiazole hydrochloride;
5-(1-(8-chloroquinolin-2-yl)piperidin-4-yl)-3-(4-fluorophenyl)-1,2,4-oxadiazole;
3-(pyridin-3-yl)-5-(1-(8-(pyridin-4-yl)quinolin-2-yl)piperidin-4-yl)-1,2,4-oxadiazole hydrochloride;
5-(1-(6-methoxyquinolin-2-yl)piperidin-4-yl)-3-(pyridin-3-yl)-1,2,4-oxadiazole;
5-(1-(6-methoxyquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-nitrophenyl)-1,2,4-oxadiazole;
4-(5-(1-(8-methoxyquinolin-2-yl)piperidin-4-yl)-1,2,4-oxadiazol-3-yl)phenol;
5-(1-(8-methoxyquinolin-2-yl)piperidin-4-yl)-3-(pyridin-2-yl)-1,2,4-oxadiazole hydrochloride;
3-(5-(1-(8-methoxyquinolin-2-yl)piperidin-4-yl)-1,2,4-oxadiazol-3-yl)pyridine 1-oxide;
3-(pyridin-3-yl)-5-(1-(6-(pyridin-4-yl)quinolin-2-yl)piperidin-4-yl)-1,2,4-oxadiazole;
5-(5-(1-(8-methoxyquinolin-2-yl)piperidin-4-yl)-1,2,4-oxadiazol-3-yl)-N-methylpyridin-2-amine;
5-(1-(8-methoxyquinolin-2-yl)piperidin-4-yl)-3-(3-nitrophenyl)-1,2,4-oxadiazole;
5-(5-(1-(8-methoxyquinolin-2-yl)piperidin-4-yl)-1,2,4-oxadiazol-3-yl)pyridin-2-amine hydrochloride;
3-(6-chloropyridin-3-yl)-5-(1-(8-methoxyquinolin-2-yl)piperidin-4-yl)-1,2,4-oxadiazole hydrochloride;
5-(1-(8-methoxyquinolin-2-yl)piperidin-4-yl)-3-(pyrimidin-2-yl)-1,2,4-oxadiazole;
5-(1-(8-methoxyquinolin-2-yl)piperidin-4-yl)-3-(6-(piperidin-1-yl)pyridin-3-yl)-1,2,4-oxadiazole;
5-(1-(8-methoxyquinolin-2-yl)piperidin-4-yl)-3-(6-(piperazin-1-yl)pyridin-3-yl)-1,2,4-oxadiazole;
2-((5-(5-(1-(8-methoxyquinolin-2-yl)piperidin-4-yl)-1,2,4-oxadiazol-3-yl)pyridin-2-yl)amino)ethan-1-ol hydrochloride;
5-(5-(1-(8-methoxyquinolin-2-yl)piperidin-4-yl)-1,2,4-oxadiazol-3-yl)pyridin-3-ol;
5-(1-(8-methoxyquinolin-2-yl)piperidin-4-yl)-3-(1H-pyrrolo[2,3-b]pyridin-5-yl)-1,2,4-oxadiazole;
3-(furan-2-yl)-5-(1-(8-methoxyquinolin-2-yl)piperidin-4-yl)-1,2,4-oxadiazole;
5-(1-(8-methoxyquinolin-2-yl)piperidin-4-yl)-3-(6-methylpyridin-3-yl)-1,2,4-oxadiazole;
tert-butyl ((1-(5-(5-(1-(8-methoxyquinolin-2-yl)piperidin-4-yl)-1,2,4-oxadiazol-3-yl)pyridin-2-yl)piperidin-4-yl)methyl)carbamate;
tert-butyl ((1-(5-(5-(1-(8-methoxyquinolin-2-yl)piperidin-4-yl)-1,2,4-oxadiazol-3-yl)pyridin-2-yl)pyrrolidin-3-yl)methyl)carbamate;
(1-(5-(5-(1-(8-methoxyquinolin-2-yl)piperidin-4-yl)-1,2,4-oxadiazol-3-yl)pyridin-2-yl)pyrrolidin-3-yl)methanamine trifluoroacetate;
(1-(5-(5-(1-(8-methoxyquinolin-2-yl)piperidin-4-yl)-1,2,4-oxadiazol-3-yl)pyridin-2-yl)piperidin-4-yl)methanamine trifluoroacetate;
3-(6-methoxybenzo[d]thiazol-2-yl)-5-(1-(8-methoxyquinolin-2-yl)piperidin-4-yl)-1,2,4-oxadiazole;
5-(1-(8-methoxyquinolin-2-yl)piperidin-4-yl)-3-(thiophen-2-yl)-1,2,4-oxadiazole;

4-(5-(5-(1-(8-methoxyquinolin-2-yl)piperidin-4-yl)-1,2,4-oxadiazol-3-yl)pyridin-2-yl)morpholine;
3-(5-(1-(8-methoxyquinolin-2-yl)piperidin-4-yl}-1,2,4-oxadiazol-3-yl}aniline hydrochloride;
3-(6-methoxypyridin-3-yl)-5-(1-(8-methoxyquinolin-2-yl)piperidin-4-yl)-1,2,4-oxadiazole;
3-(5-fluoropyridin-3-yl)-5-(1-(8-methoxyquinolin-2-yl)piperidin-4-yl)-1,2,4-oxadiazole; or
5-(1-(8-methoxyquinolin-2-yl)piperidin-4-yl)-3-(6-(pyrrolidin-1-yl)pyridin-3-yl)-1,2,4-oxadiazole hydrochloride.

**17.** Compound according to any of the previous claims, wherein the compound is selected from:

5-(1-(8-methoxyquinolin-2-yl)piperidin-4-yl)-3-(4-(trifluoromethoxy)phenyl)-1,2,4-oxadiazole;
5-(1-(8-methoxyquinolin-2-yl)piperidin-4-yl)-3-(4-(trifluoromethyl)phenyl)-1,2,4-oxadiazole hydrochloride;
3-(3,5-dimethoxyphenyl)-5-(1-(8-methoxyquinolin-2-yl)piperidin-4-yl)-1,2,4-oxadiazole;
5-(1-(8-methoxyquinolin-2-yl)piperidin-4-yl)-3-(3-(trifluoromethyl)phenyl)-1,2,4-oxadiazole;
5-(1-(8-methoxyquinolin-2-yl)piperidin-4-yl)-3-(pyridin-3-yl)-1,2,4-oxadiazole hydrochloride;
5-(1-(8-methoxyquinolin-2-yl)piperidin-4-yl)-3-(6-(trifluoromethyl)pyridin-3-yl)-1,2,4-oxadiazole;
5-(1-(8-chloroquinolin-2-yl)piperidin-4-yl)-3-(pyridin-3-yl)-1,2,4-oxadiazole hydrochloride;
5-(1-(6-methoxyquinolin-2-yl)piperidin-4-yl)-3-(pyridin-3-yl)-1,2,4-oxadiazole;
4-(5-(1-(8-methoxyquinolin-2-yl)piperidin-4-yl)-1,2,4-oxadiazol-3-yl)phenol;
3-(pyridin-3-yl)-5-(1-(6-(pyridin-4-yl)quinolin-2-yl)piperidin-4-yl)-1,2,4-oxadiazole;
3-(6-chloropyridin-3-yl)-5-(1-(8-methoxyquinolin-2-yl)piperidin-4-yl)-1,2,4-oxadiazole hydrochloride;
5-(1-(8-methoxyquinolin-2-yl)piperidin-4-yl)-3-(6-(piperidin-1-yl)pyridin-3-yl)-1,2,4-oxadiazole;
5-(1-(8-methoxyquinolin-2-yl)piperidin-4-yl)-3-(6-(piperazin-1-yl)pyridin-3-yl)-1,2,4-oxadiazole;
2-((5-(5-(1-(8-methoxyquinolin-2-yl)piperidin-4-yl)-1,2,4-oxadiazol-3-yl)pyridin-2-yl)amino)ethan-1-ol hydrochloride; or
5-(1-(8-methoxyquinolin-2-yl)piperidin-4-yl)-3-(6-(pyrrolidin-1-yl)pyridin-3-yl)-1,2,4-oxadiazole hydrochloride.

**18.** Compound according to any of the previous claims for use in medicine or veterinary.

**19.** Compound according to any of the previous claims for use in the treatment or prevention of tuberculosis.

**20.** Compound for use according to any of the previous claims 18-19 in combination with at least one anti-HIV agent, wherein the anti-HIV agent is selected from a HIV protease inhibitor, a HIV nucleoside reverse transcriptase inhibitor, a HIV non-nucleoside reverse transcriptase inhibitor, or a HIV integrase inhibitor.

**21.** Compound for use according to claims 18-20 in combination with at least a second tuberculosis drug, wherein the further tuberculosis drug is selected from the group of isoniazid, rifamycin and derivatives, pyrazinamide, ethambutol, cycloserine, ethionamide, streptomycin, amikacin, kanamycin, rifampin (rifampicin), aminoglycosides, capreomycin, p-aminosalicyclic acid, fluoroquinolones such as levofloxacin, moxafloxacin or gatifloxacin, or mixtures thereof.

**22.** Pharmaceutical composition comprising a compound or a compound for use according to any of the previous claims 1-21 and a pharmaceutical acceptable excipient, wherein the compound is a therapeutically effective amount of a compound described in any of the previous claims or a pharmaceutically acceptable salt or solvate thereof.

## Patentansprüche

**1.** Verbindung der allgemeinen Formel I oder ein pharmazeutisch verträgliches Salz, Hydrat, Solvat, N-Oxid, Stereoisomer, Diastereoisomer, Enantiomer, Atropoisomer, Polymorph oder Esther davon:

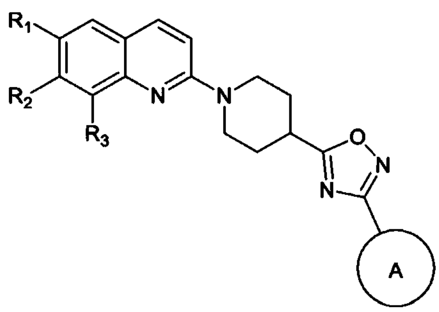

Formel I

wobei

$R_1$, $R_2$, $R_3$ und der A-Ring unabhängig voneinander ausgewählt sind;
der A-Ring ein substituierter oder unsubstituierter Ring ist, ausgewählt aus Pyridin-2-yl, Pyridin-3-yl, Pyridin-4-yl, Pyrazin-2-yl, 1H-Pyrrolo[2,3-b]pyridin-5-yl, Furan-2-yl, 6-Methoxybenzo[d]thiazol-2-yl, Thiophen-2-yl, 3-Pyridin-1-oxid, Pyrimidin-2-yl oder Phenyl; $R_1$ ausgewählt ist aus H, Halogen, $C_1$-$C_6$ -Alkoxy, oder unsubstituiertem Heteroaryl;
$R_2$ ausgewählt ist aus H oder OH;
$R_3$ ausgewählt ist aus H, Halogen, $C_1$-$C_6$ -Alkoxy, oder unsubstituiertem Heteroaryl;
und "pharmazeutisch verträgliche Esther" von Verbindungen Verbindungen sind, in denen eine oder mehrere Carboxylgruppen (z. B. -COOH) durch Reaktion mit einem alkoholischen Substituenten A-OH modifiziert sind, so dass -COOA-Gruppen entstehen, wobei A $C_1$-$C_6$-Alkyl ist.

**2.** Verbindungen der Formel I nach Anspruch 1, wobei

der A-Ring ein substituierter oder unsubstituierter Ring ist, ausgewählt aus Pyridin-2-yl, Pyridin-3-yl, Pyridin-4-yl, Pyrazin-2-yl, 1H-Pyrrolo[2,3-b]pyridin-5-yl, Furan-2-yl, 6-Methoxybenzo[d]thiazol-2-yl, Thiophen-2-yl, 3-Pyridin-1-oxid, Pyrimidin-2-yl oder Phenyl; $R_1$ ausgewählt ist aus H, Cl, $OCH_3$ oder unsubstituiertem Heteroaryl;
$R_2$ ausgewählt ist aus H oder OH;
$R_3$ ausgewählt ist aus H, Cl, $OCH_3$ oder unsubstituiertem Heteroaryl.

**3.** Verbindungen nach einem der Ansprüchen 1 - 2, wobei

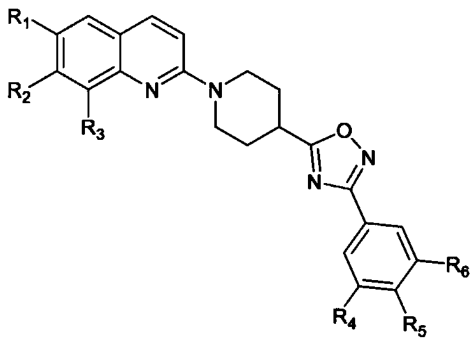

Formel II

$R_1$, $R_2$, $R_3$, $R_4$, $R_5$ und $R_6$ unabhängig voneinander ausgewählt sind;
$R_1$ ausgewählt ist aus H, Cl, $OCH_3$ oder Pyridin-4-yl;
$R_2$ ausgewählt ist aus H oder OH;
$R_3$ ausgewählt ist aus H, Cl, $OCH_3$ oder Pyridin-4-yl;
$R_4$ ausgewählt ist aus H, $OCH_3$, $CF_3$, $OCF_3$, $NO_2$ oder $NH_2$;
$R_5$ ausgewählt ist aus H, $OCH_3$, $CF_3$, $OCF_3$, F, $NO_2$ oder OH;
$R_6$ ausgewählt ist aus H, $OCH_3$, $CF_3$ oder $OCF_3$.

**4.** Verbindung nach einem der Ansprüchen 1 - 2, wobei

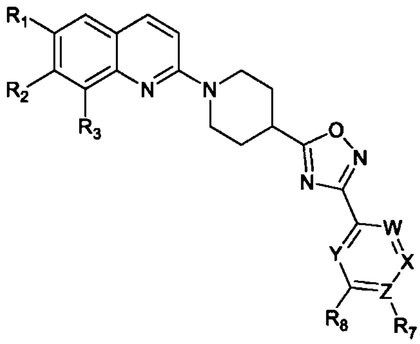

Formel III

$R_1$, $R_2$, $R_3$, $R_7$, $R_8$ unabhängig voneinander ausgewählt sind;
X, Y, Z und W unabhängig von C oder N ausgewählt sind;
$R_1$ ausgewählt ist aus H, Cl, $OCH_3$ oder Pyridin-4-yl;
$R_2$ ausgewählt ist aus H oder OH;
$R_3$ ausgewählt ist aus H, Cl, $OCH_3$ oder Pyridin-4-yl;
$R_7$ ausgewählt ist aus H, $OCH_3$, $CF_3$, $NHCH_3$, $NH_2$, Cl, Piperidinyl, Piperazinyl, Morpholinyl, Pyrrolidinyl, $NH(CH_2)OH$ oder $CH_3$;
$R_8$ ausgewählt ist aus H, F oder OH.

**5.** Verbindungen nach einem der Ansprüchen 1 - 2, wobei

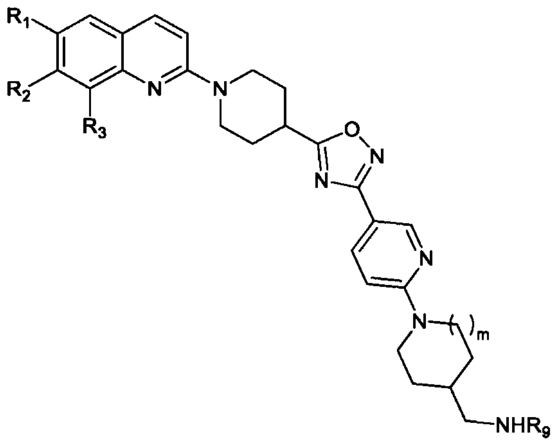

Formel IV

$R_1$ ausgewählt ist aus H, Cl, $OCH_3$ oder Pyridin-4-yl;
$R_2$ ausgewählt ist aus H oder OH;
$R_3$ ausgewählt ist aus H, Cl, $OCH_3$ oder Pyridin-4-yl;
m ausgewählt ist aus 0 oder 1;
$R_9$ ausgewählt ist aus H oder tert-Butylcarbamat.

**6.** Verbindungen nach einem der Ansprüchen 1 - 2, wobei

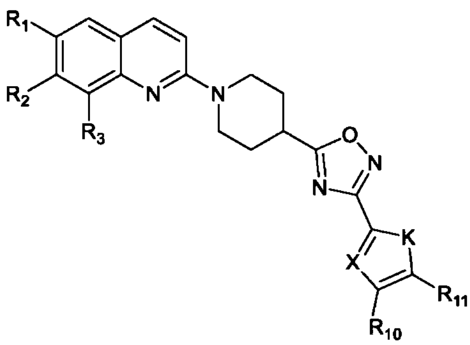

Formel V

$R_1$ ausgewählt ist aus H, Cl, $OCH_3$ oder Pyridin-4-yl;
$R_2$ ausgewählt ist aus H oder OH;
$R_3$ ausgewählt ist aus H, Cl, $OCH_3$ oder Pyridin-4-yl;
X ist C oder N;
K ist S oder O;
$R_{10}$ und $R_{11}$ ausgewählt sind aus H oder zusammen mit Kohlenstoffen unter Bildung von 6-Methoxybenzo[d] thiazol-2-yl gebunden sind.

**7.** Verbindung nach Anspruch 3, wobei $R_3$ ist H oder $OCH_3$.

**8.** Verbindung nach Anspruch 3, wobei $R_4$ ist H, $OCH_3$ oder $CF_3$.

**9.** Verbindung nach Anspruch 3, wobei $R_5$ ausgewählt ist aus H, $CF_3$, $OCF_3$ oder OH.

**10.** Verbindung nach Anspruch 3, wobei $R_6$ ausgewählt ist aus H, $OCH_3$ oder $CF_3$.

**11.** Verbindung nach Anspruch 4, wobei X ist N.

**12.** Verbindung nach Anspruch 4, wobei $R_1$ ist H, Cl, $OCH_3$ oder Pyridin-4-yl.

**13.** Verbindung nach Anspruch 4, wobei $R_3$ ist H, Cl oder $OCH_3$.

**14.** Verbindung nach Anspruch 4, wobei $R_7$ ist H, $CF_3$, Cl, Piperidinyl, Piperazinyl, Pyrrolidinyl oder $NH(CH_2)OH$.

**15.** Verbindung nach einem der vorangehenden Ansprüche, wobei das Salz ein Hydrochlorid oder ein Trifluoroacetat ist.

**16.** Verbindung nach einem der vorangehenden Ansprüche, wobei die Verbindung ist:

5-(1-(6-Chlorchinolin-2-yl)piperidin-4-yl)-3-phenyl-1,2,4-oxadiazol;
5-(1-(6-Chlorchinolin-2-yl)piperidin-4-yl)-3-(4-(trifluormethoxy)phenyl)-1,2,4-oxadiazol;
5-(1-(8-Methoxychinolin-2-yl)piperidin-4-yl)-3-(4-(trifluormethoxy)phenyl)-1,2,4-oxadiazol;
5-(1-(8-Chlorchinolin-2-yl)piperidin-4-yl)-3-(4-(trifluormethoxy)phenyl)-1,2,4-oxadiazol;
5-(1-(8-Methoxychinolin-2-yl)piperidin-4-yl)-3-(4-(trifluormethyl)phenyl)-1,2,4-oxadiazol-hydrochlorid;
3-(3,5-Dimethoxyphenyl)-5-(1-(8-methoxychinolin-2-yl)piperidin-4-yl)-1,2,4-oxadiazol;
5-(1-(8-Methoxychinolin-2-yl)piperidin-4-yl)-3-(3-(trifluormethyl)phenyl)-1,2,4-oxadiazol;
3-(3,5-Bis(trifluormethyl)phenyl)-5-(1-(8-methoxychinolin-2-yl)piperidin-4-yl)-1,2,4-oxadiazol;
5-(1-(8-Methoxyquinolin-2-yl)piperidin-4-yl)-3-(pyridin-4-yl)-1,2,4-oxadiazol;
5-(1-(8-Methoxychinolin-2-yl)piperidin-4-yl)-3-(pyridin-3-yl)-1,2,4-oxadiazol-hydrochlorid;
5-(1-(8-Methoxychinolin-2-yl)piperidin-4-yl)-3-(6-(trifluormethyl)pyridin-3-yl)-1,2,4-oxadiazol;
3-(3-Methoxyphenyl)-5-(1-(8-methoxychinolin-2-yl)piperidin-4-yl)-1,2,4-oxadiazol-hydrochlorid;
5-(1-(8-Methoxychinolin-2-yl)piperidin-4-yl)-3-(3-(trifluormethoxy)phenyl)-1,2,4-oxadiazol;
3-(4-Methoxyphenyl)-5-(1-(8-Methoxychinolin-2-yl)piperidin-4-yl)-1,2,4-oxadiazol;
5-(1-(6-Chlorchinolin-2-yl)piperidin-4-yl)-3-(pyridin-3-yl)-1,2,4-oxadiazol-hydrochlorid;
5-(1-(8-Methoxychinolin-2-yl)piperidin-4-yl)-3-(pyrazin-2-yl)-1,2,4-oxadiazol-hydrochlorid;
7-Hydroxy-8-methoxy-2-(4-(3-(pyridin-3-yl)-1,2,4-oxadiazol-5-yl)piperidin-1-yl)quinolin 1-oxid;
5-(1-(8-Chlorchinolin-2-yl)piperidin-4-yl)-3-(pyridin-3-yl)-1,2,4-oxadiazol-hydrochlorid;
5-(1-(8-Chlorchinolin-2-yl)piperidin-4-yl)-3-(4-fluorphenyl)-1,2,4-oxadiazol;
3-(Pyridin-3-yl)-5-(1-(8-(pyridin-4-yl)quinolin-2-yl)piperidin-4-yl)-1,2,4-oxadiazol hydrochlorid;
5-(1-(6-Methoxyquinolin-2-yl)piperidin-4-yl)-3-(pyridin-3-yl)-1,2,4-oxadiazol;
5-(1-(6-Methoxychinolin-2-yl)piperidin-4-yl)-3-(4-(trifluormethoxy)phenyl)-1,2,4-oxadiazol;
5-(1-(8-Methoxychinolin-2-yl)piperidin-4-yl)-3-(4-nitrophenyl)-1,2,4-oxadiazol;
4-(5-(1-(8-Methoxyquinolin-2-yl)piperidin-4-yl)-1,2,4-oxadiazol-3-yl)phenol;
5-(1-(8-Methoxychinolin-2-yl)piperidin-4-yl)-3-(pyridin-2-yl)-1,2,4-oxadiazol-hydrochlorid;
3-(5-(1-(8-Methoxychinolin-2-yl)piperidin-4-yl)-1,2,4-oxadiazol-3-yl)pyridin-1-oxid;
3-(Pyridin-3-yl)-5-(1-(6-(pyridin-4-yl)quinolin-2-yl)piperidin-4-yl)-1,2,4-oxadiazol;
5-(5-(1-(8-Methoxyquinolin-2-yl)piperidin-4-yl)-1,2,4-oxadiazol-3-yl)-N-methylpyridin-2-amin;
5-(1-(8-Methoxychinolin-2-yl)piperidin-4-yl)-3-(3-nitrophenyl)-1,2,4-oxadiazol;
5-(5-(1-(8-Methoxychinolin-2-yl)piperidin-4-yl)-1,2,4-oxadiazol-3-yl)pyridin-2-amin-hydrochlorid;
3-(6-Chlorpyridin-3-yl)-5-(1-(8-methoxychinolin-2-yl)piperidin-4-yl)-1,2,4-oxadiazol-hydrochlorid;
5-(1-(8-Methoxychinolin-2-yl)piperidin-4-yl)-3-(pyrimidin-2-yl)-1,2,4-oxadiazol;
5-(1-(8-Methoxyquinolin-2-yl)piperidin-4-yl)-3-(6-(piperidin-1-yl)pyridin-3-yl)-1,2,4-oxadiazol;
5-(1-(8-Methoxyquinolin-2-yl)piperidin-4-yl)-3-(6-(piperazin-1-yl)pyridin-3-yl)-1,2,4-oxadiazol;
2-((5-(5-(1-(8-Methoxychinolin-2-yl)piperidin-4-yl)-1,2,4-oxadiazol-3-yl)pyridin-2-yl)amino)ethan-1-ol-hydrochlorid;
5-(5-(1-(8-Methoxyquinolin-2-yl)piperidin-4-yl)-1,2,4-oxadiazol-3-yl)pyridin-3-ol;
5-(1-(8-Methoxyquinolin-2-yl)piperidin-4-yl)-3-(1H-pyrrolo[2,3-b]pyridin-5-yl)-1,2,4-oxadiazol;
3-(Furan-2-yl)-5-(1-(8-methoxyquinolin-2-yl)piperidin-4-yl)-1,2,4-oxadiazol;
5-(1-(8-Methoxyquinolin-2-yl)piperidin-4-yl)-3-(6-methylpyridin-3-yl)-1,2,4-oxadiazol;
tert-Butyl ((1-(5-(5-(1-(8-methoxychinolin-2-yl)piperidin-4-yl)-1,2,4-oxadiazol-3-yl)pyridin-2-yl)piperidin-4-yl)methyl)carbamat;

tert-Butyl ((1-(5-(5-(1-(8-methoxychinolin-2-yl)piperidin-4-yl)-1,2,4-oxadiazol-3-yl)pyridin-2-yl)pyrrolidin-3-yl) methyl)carbamat;
(1-(5-(5-(1-(8-Methoxychinolin-2-yl)piperidin-4-yl)-1,2,4-oxadiazol-3-yl)pyridin-2-yl)pyrrolidin-3-yl)}methanamin-trifluoracetat;
(1-(5-(5-(1-(8-Methoxychinolin-2-yl)piperidin-4-yl)-1,2,4-oxadiazol-3-yl)pyridin-2-yl)piperidin-4-yl)methanamin-trifluoracetat;
3-(6-Methoxybenzo[d]thiazol-2-yl)-5-(1-(8-methoxychinolin-2-yl)piperidin-4-yl)-1,2,4-oxadiazol;
5-(1-(8-Methoxyquinolin-2-yl)piperidin-4-yl)-3-(thiophen-2-yl)-1,2,4-oxadiazol;
4-(5-(5-(1-(8-Methoxychinolin-2-yl)piperidin-4-yl)-1,2,4-oxadiazol-3-yl)pyridin-2-yl)morpholin;
3-(5-(1-(8-Methoxychinolin-2-yl)piperidin-4-yl)-1,2,4-oxadiazol-3-yl)anilin-hydrochlorid;
3-(6-Methoxypyridin-3-yl)-5-(1-(8-methoxychinolin-2-yl)piperidin-4-yl)-1,2,4-oxadiazol;
3-(5-Fluorpyridin-3-yl)-5-(1-(8-methoxychinolin-2-yl)piperidin-4-yl)-1,2,4-oxadiazol; oder
5-(1-(8-Methoxychinolin-2-yl)piperidin-4-yl)-3-(6-(pyrrolidin-1-yl)pyridin-3-yl)-1,2,4-oxadiazol-hydrochlorid.

**17.** Verbindung nach einem der vorangehenden Ansprüche, wobei die Verbindung ausgewählt ist aus:

5-(1-(8-Methoxychinolin-2-yl)piperidin-4-yl)-3-(4-(trifluormethoxy)phenyl)-1,2,4-oxadiazol;
5-(1-(8-Methoxychinolin-2-yl)piperidin-4-yl)-3-(4-(trifluormethyl)phenyl)-1,2,4-oxadiazol-hydrochlorid;
3-(3,5-Dimethoxyphenyl)-5-(1-(8-methoxychinolin-2-yl)piperidin-4-yl)-1,2,4-oxadiazol;
5-(1-(8-Methoxychinolin-2-yl)piperidin-4-yl)-3-(3-(trifluormethyl)phenyl)-1,2,4-oxadiazol;
5-(1-(8-Methoxychinolin-2-yl)piperidin-4-yl)-3-(pyridin-3-yl)-1,2,4-oxadiazol-hydrochlorid;
5-(1-(8-Methoxychinolin-2-yl)piperidin-4-yl)-3-(6-(trifluormethyl)pyridin-3-yl)-1,2,4-oxadiazol;
5-(1-(8-Chlorchinolin-2-yl)piperidin-4-yl)-3-(pyridin-3-yl)-1,2,4-oxadiazol-hydrochlorid;
5-(1-(6-Methoxyquinolin-2-yl)piperidin-4-yl)-3-(pyridin-3-yl)-1,2,4-oxadiazol;
4-(5-(1-(8-Methoxyquinolin-2-yl)piperidin-4-yl)-1,2,4-oxadiazol-3-yl)phenol;
3-(Pyridin-3-yl)-5-(1-(6-(pyridin-4-yl)quinolin-2-yl)piperidin-4-yl)-1,2,4-oxadiazol;
3-(6-Chlorpyridin-3-yl)-5-(1-(8-methoxychinolin-2-yl)piperidin-4-yl)-1,2,4-oxadiazol-hydrochlorid;
5-(1-(8-Methoxyquinolin-2-yl)piperidin-4-yl)-3-(6-(piperidin-1-yl)pyridin-3-yl)-1,2,4-oxadiazol;
5-(1-(8-Methoxyquinolin-2-yl)piperidin-4-yl)-3-(6-(piperazin-1-yl)pyridin-3-yl)-1,2,4-oxadiazol;
2-((5-(5-(1-(8-Methoxychinolin-2-yl)piperidin-4-yl)-1,2,4-oxadiazol-3-yl)pyridin-2-yl)amino)ethan-1-ol-hydrochlorid; oder
5-(1-(8-Methoxychinolin-2-yl)piperidin-4-yl)-3-(6-(pyrrolidin-1-yl)pyridin-3-yl)-1,2,4-oxadiazol-hydrochlorid.

**18.** Verbindung nach einem der vorangehenden Ansprüche zur Verwendung in der Medizin oder Tiermedizin.

**19.** Verbindung nach einem der vorangehenden Ansprüche zur Verwendung bei der Behandlung oder Prävention von Tuberkulose.

**20.** Verbindung zur Verwendung nach einem der vorangehenden Ansprüche 18-19 in Verbindung mit mindestens einem Anti-HIV-Wirkstoff, wobei der Anti-HIV-Wirkstoff aus einem HIV-Protease-Inhibitor, einem nukleosidischen HIV-Reverse-Transkriptase-Inhibitor, einem nicht nukleosidischen HIV-Reverse-Transkriptase-Inhibitor, oder einem HIV-Integrase-Inhibitor ausgewählt ist.

**21.** Verbindung zur Verwendung nach Ansprüche 18-20 in Verbindung mit mindestens einem zweiten Tuberkulosearzneimittel, wobei das Tuberkulosearzneimittel ausgewählt ist aus der Gruppe von Isoniazid, Rifamycin und Derivaten, Pyrazinamid, Ethambutol, Cycloserin, Ethionamid, Streptomycin, Amikacin, Kanamycin, Rifampin (Rifampicin), Aminoglykoside, Capreomycin, p-Aminosalicylsäure, Fluorchinolone wie Levofloxacin, Moxifloxacin oder Gatifloxacin, oder Mischungen davon.

**22.** Pharmazeutische Zusammensetzung, umfassend eine Verbindung oder eine Verbindung zur Verwendung nach einem der vorangehenden Ansprüche 1 - 21 und einen pharmazeutisch verträglichen Hilfsstoff, wobei die Verbindung eine therapeutisch wirksame Menge einer in einem der vorangehenden Ansprüche beschriebenen Verbindung oder ein pharmazeutisch verträgliches Salz oder Solvat davon ist.

## Revendications

**1.** Composé de formule générale 1 ou un sel, hydrate, solvate, N-oxyde, stéréoisomère, diastéréoisomère, énantiomère, atropoisomère, polymorphe ou ester pharmaceutiquement acceptable de celui-ci :

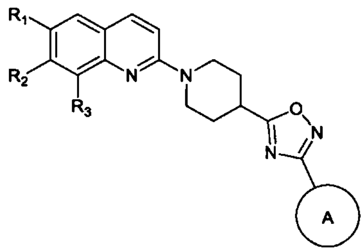

Formule I

dans lequel

$R_1$, $R_2$, $R_3$ et le chaînon A sont choisis indépendamment les uns des autres ;
le chaînon A est un chaînon substitué ou non substitué choisi parmi le pyridin-2-yle, pyridin-3-yle, pyridin-4-yle, pyrazin-2-yle, 1H-pyrrolo[2,3-b]pyridine-5-yle, furan-2-yle, 6-méthoxybenzo[d]thiazol-2-yle, thiophen-2-yle, 3-pyridine 1-oxyde, pyrimidin-2-yle ou phényle ;
$R_1$ est choisi parmi H, Halogène, $C_1$-$C_6$ alkoxy, ou un hétéroaryle non substitué ;
$R_2$ est choisi parmi H ou OH ;
$R_3$ est choisi parmi H, Halogène, $C_1$-$C_6$ alkoxy, ou un hétéroaryle non substitué ;
et des « esters pharmaceutiquement acceptables » de composés sont des composés dans lesquels un ou plusieurs groupes carboxyles (e.g. -COOH) sont modifiés par réaction avec un substituant alcoolique A-OH afin de former des groupes -COOA, dans lesquels A est un $C_1$-$C_6$ alkyle.

**2.** Composés de formule I selon la revendication 1, dans lequel

le chaînon A est un chaînon substitué ou non substitué choisi parmi le pyridin-2-yle, pyridin-3-yle, pyridin-4-yle, pyrazin-2-yle, 1H-pyrrolo[2,3-b]pyridine-5-yle, furan-2-yle, 6-méthoxybenzo[d]thiazol-2-yle, thiophen-2-yle, 3-pyridine 1-oxyde, pyrimidin-2-yle ou phényle ;
$R_1$ est choisi parmi H, Cl, $OCH_3$ ou un hétéroaryle non substitué ;
$R_2$ est choisi parmi H ou OH ;
$R_3$ est choisi parmi H, Cl, $OCH_3$ ou un hétéroaryle non substitué.

**3.** Composés selon l'une quelconque des revendications 1 - 2, dans lequel

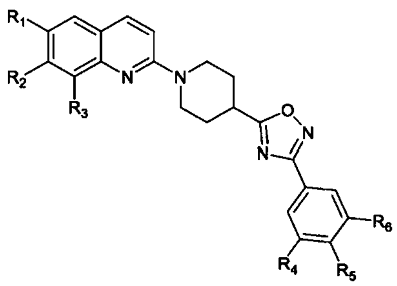

Formule II

$R_1$, $R_2$, $R_3$, $R_4$, $R_5$ et $R_6$ sont choisis indépendamment les uns des autres ;
$R_1$ est choisi parmi H, Cl, $OCH_3$ ou pyridine-4-yle ;
$R_2$ est choisi parmi H ou OH ;
$R_3$ est choisi parmi H, Cl, $OCH_3$ ou pyridine-4-yle ;
$R_4$ est choisi parmi H, $OCH_3$, $CF_3$, $OCF_3$, $NO_2$, ou NHz ;
$R_5$ est choisi parmi H, $OCH_3$, $CF_3$, $OCF_3$, F, $NO_2$ ou OH ;
$R_6$ est choisi parmi H, $OCH_3$, $CF_3$ ou $OCF_3$.

**4.** Composé selon l'une quelconque des revendications 1 - 2, dans lequel

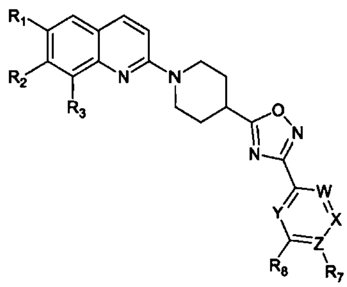
Formule III

$R_1$, $R_2$, $R_3$, $R_7$, $R_8$ sont choisis indépendamment les uns des autres ;
X, Y, Z et W sont choisis indépendamment parmi C ou N ;
$R_1$ est choisi parmi H, Cl, $OCH_3$ ou pyridine-4-yle ;
$R_2$ est choisi parmi H ou OH ;
$R_3$ est choisi parmi H, Cl, $OCH_3$ ou pyridine-4-yle ;
$R_7$ est choisi parmi H, $OCH_3$, $CF_3$, $NHCH_3$, $NH_2$, Cl, pipéridinyle, pipérazinyle, morpholinyle, pyrrolidinyle, $NH(CH_2)OH$ ou $CH_3$ ;
$R_3$ est choisi parmi H, F ou OH.

**5.** Composés selon l'une quelconque des revendications 1 - 2, dans lesquels

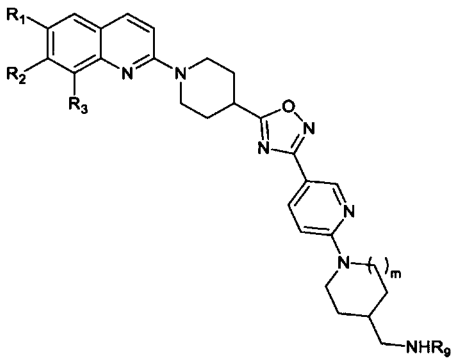
Formule IV

$R_1$ est choisi parmi H, Cl, $OCH_3$ ou pyridine-4-yle ;
$R_2$ est choisi parmi H ou OH ;
$R_3$ est choisi parmi H, Cl, $OCH_3$ ou pyridine-4-yle ;
m est choisi parmi 0 ou 1;
$R_9$ est choisi parmi H ou carbamate de tert-butyle.

**6.** Composés selon l'une quelconque des revendications 1 - 2, dans lesquels

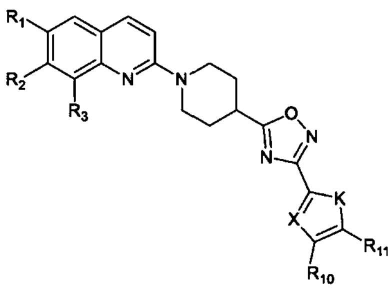
Formule V

$R_1$ est choisi parmi H, Cl, $OCH_3$ ou pyridine-4-yle ;
$R_2$ est choisi parmi H ou OH ;
$R_3$ est choisi parmi H, Cl, $OCH_3$ ou pyridine-4-yle ;
X est C ou N ;

K est 5 ou O ;
$R_{10}$ et $R_{11}$ sont choisis parmi H ou fixés ensemble avec des atomes de carbone pour former du méthoxybenzo[d] thiazol-2-yle.

**7.** Composé selon la revendication 3, dans lequel $R_3$ est H ou $OCH_3$.

**8.** Composé selon la revendication 3, dans lequel $R_4$ est H, $OCH_3$ ou $CF_3$.

**9.** Composé selon la revendication 3, dans lequel $R_3$ est choisi parmi H, $CF_3$, $OCF_3$ ou OH.

**10.** Composé selon la revendication 3, dans lequel $R_6$ est choisi parmi H, $OCH_3$ ou $CF_3$.

**11.** Composé selon la revendication 4, dans lequel X est N.

**12.** Composé selon la revendication 4, dans lequel $R_1$ est H, Cl, $OCH_3$ ou pyridine-4-yle.

**13.** Composé selon la revendication 4, dans lequel $R_3$ est H, Cl, ou $OCH_3$.

**14.** Composé selon la revendication 4, dans lequel $R_7$ est H, $CF_3$, Cl, pipéridinyle, pipérazinyle, pyrrolidinyle ou $NH(CH_2)OH$.

**15.** Composé selon l'une quelconque des revendications précédentes, dans lequel le sel est un chlorhydrate ou un trifluoracétate.

**16.** Composé selon l'une quelconque des revendications précédentes, dans lequel le composé est le :

5-(1-(6-chloroquinolin-2-yl)pipéridin-4-yl)-3-phényl-1,2,4-oxadiazole ;
5-(1-(6-chloroquinolin-2-yl)pipéridin-4-yl)-3-(4-(trifluorométhoxy)phényl)-1,2,4-oxadiazole;
5-(1-(8-méthoxyquinolin-2-yl)pipéridin-4-yl)-3-(4-(trifluorométhoxy)phényl)-1,2,4-oxadiazole ;
5-(1-(8-chloroquinolin-2-yl)pipéridin-4-yl)-3-(4-(trifluorométhoxy)phényl)-1,2,4-oxadiazole;
chlorhydrate de 5-(1-(8-méthoxyquinolin-2-yl)pipéridin-4-yl)-3-(4-(trifluorométhyl)phényl)-1,2,4-oxadiazole ;
3-(3,5-diméthoxyphényl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(3-(trifluorométhyl)phényl)-1,2,4-oxadiazole ;
3-(3,5-bis(trifluorométhyl)phényl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(pyridin-4-yl)-1,2,4-oxadiazole ;
chlorhydrate de 5-(1-(8-méthoxyquinolin-2-yl)pipéridin-4-yl)-3-(pyridin-3-yl)-1,2,4-oxadiazole ;
5-(1-(8-méthoxyquinolin-2-yl)pipéridin-4-yl)-3-(6-(trifluorométhyl)pyridin-3-yl)-1,2,4-oxadiazole ;
chlorhydrate de 3-(3-méthoxyphényl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(3-(trifluorométhoxy)phényl)-1,2,4-oxadiazole ;
3-(4-méthoxyphényl)-5-(1-(8-méthoxyquinolin-2-yl)pipéridin-4-yl)-1,2,4-oxadiazole ;
chlorhydrate de 5-(1-(6-chloroquinolin-2-yl)pipéridin-4-yl)-3-(pyridin-3-yl)-1,2,4-oxadiazole ;
chlorhydrate de 5-(1-(8-méthoxyquinolin-2-yl)pipéridin-4-yl)-3-(pyrazin-2-yl)-1,2,4-oxadiazole ;
7-hydroxy-8-méthoxy-2-(4-(3-(pyridin-3-yl)-1,2,4-oxadiazol-5-yl)pipéridin-1-yl)quinoline 1-oxide ;
chlorhydrate de 5-(1-(8-chloroquinolin-2-yl)pipéridin-4-yl)-3-(pyridin-3-yl)-1,2,4-oxadiazole ;
5-(1-(8-chloroquinolin-2-yl)pipéridin-4-yl)-3-(4-fluorophényl)-1,2,4-oxadiazole ;
chlorhydrate de 3-(pyridin-3-yl)-5-(1-(8-(pyridin-4-yl)quinolin-2-yl)pipéridin-4-yl)-1,2,4-oxadiazole ;
5-(1-(6-méthoxyquinolin-2-yl)pipéridin-4-yl)-3-(pyridin-3-yl)-1,2,4-oxadiazole ;
5-(1-(6-méthoxyquinolin-2-yl)pipéridin-4-yl)-3-(4-(trifluorométhoxy)phényl)-1,2,4-oxadiazole ;
5-(1-(8-méthoxyquinolin-2-yl)pipéridin-4-yl)-3-(4-nitrophényl)-1,2,4-oxadiazole ;
4-(5-(1-(8-méthoxyquinolin-2-yl)pipéridin-4-yl)-1,2,4-oxadiazol-3-yl)phénol ;
chlorhydrate de 5-(1-(8-méthoxyquinolin-2-yl)pipéridin-4-yl)-3-(pyridin-2-yl)-1,2,4-oxadiazole ;
3-(5-(1-(8-méthoxyquinolin-2-yl)pipéridin-4-yl)-1,2,4-oxadiazol-3-yl)pyridine 1-oxide ;
3-(pyridin-3-yl)-5-(1-(6-(pyridin-4-yl)quinolin-2-yl)pipéridin-4-yl)-1,2,4-oxadiazole ;
5-(5-(1-(8-méthoxyquinolin-2-yl)pipéridin-4-yl)-1,2,4-oxadiazol-3-yl)-N-méthylpyridin-2-amine ;
5-(1-(8-méthoxyquinolin-2-yl)pipéridin-4-yl)-3-(3-nitrophényl)-1,2,4-oxadiazole ;
chlorhydrate de 5-(5-(1-(8-méthoxyquinolin-2-yl)pipéridin-4-yl)-1,2,4-oxadiazol-3-yl)pyridin-2-amine ;
chlorhydrate de 3-(6-chloropyridin-3-yl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(pyrimidin-2-yl)-1,2,4-oxadiazole ;

5-(1-(8-méthoxyquinolin-2-yl)pipéridin-4-yl)-3-(6-(pipéridin-1-yl)pyridin-3-yl)-1,2,4-oxadiazole ;
5-(1-(8-méthoxyquinolin-2-yl)pipéridin-4-yl)-3-(6-(pipérazin-1-yl)pyridin-3-yl)-1,2,4-oxadiazole ;
chlorhydrate de 2-((5-(5-(1-(8-méthoxyquinolin-2-yl)pipéridin-4-yl)-1,2,4-oxadiazol-3-yl)pyridin-2-yl)amino) éthan-1-ol ;
5-(5-(1-(8-méthoxyquinolin-2-yl)pipéridin-4-yl)-1,2,4-oxadiazol-3-yl)pyridin-3-ol ;
5-(1-(8-méthoxyquinolin-2-yl)pipéridin-4-yl)-3-(1H-pyrrolo[2,3-b]pyridin-5-yl)-1,2,4-oxadiazole ;
3-(furan-2-yl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(6-méthylpyridin-3-yl)-1,2,4-oxadiazole ;
tert-butyl ((1-(5-(5-(1-(8-méthoxyquinolin-2-yl)pipéridin-4-yl)-1,2,4-oxadiazol-3-yl)pyridin-2-yl)pipéridin-4-yl) méthyl)carbamate;
tert-butyl ((1-(5-(5-(1-(8-méthoxyquinolin-2-yl)pipéridin-4-yl)-1,2,4-oxadiazol-3-yl)pyridin-2-yl)pyrrolidin-3-yl) méthyl)carbamate ;
trifluoroacétate de (1-(5-(5-(1-(8-méthoxyquinolin-2-yl)pipéridin-4-yl)-1,2,4-oxadiazol-3-yl)pyridin-2-yl)pyrrolidin-3-yl)méthanamine ;
trifluoroacétate de (1-(5-(5-(1-(8-méthoxyquinolin-2-yl)pipéridin-4-yl)-1,2,4-oxadiazol-3-yl)pyridin-2-yl)pipéridin-4-yl)méthanamine;
3-(6-méthoxybenzo[d]thiazol-2-yl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(thiophen-2-yl)-1,2,4-oxadiazole ;
4-(5-(5-(1-(8-méthoxyquinolin-2-yl)pipéridin-4-yl)-1,2,4-oxadiazol-3-yl)pyridin-2-yl)morpholine ;
chlorhydrate de 3-(5-(1-(8-méthoxyquinolin-2-yl)pipéridin-4-yl)-1,2,4-oxadiazol-3-yl)aniline ;
3-(6-méthoxypyridin-3-yl)-5-(1-(8-méthoxyquinolin-2-yl)pipéridin-4-yl)-1,2,4-oxadiazole ;
3-(5-fluoropyridin-3-yl)-5-(1-(8-méthoxyquinolin-2-yl)pipéridin-4-yl)-1,2,4-oxadiazole ; ou
chlorhydrate de 5-(1-(8-méthoxyquinolin-2-yl)pipéridin-4-yl)-3-(6-(pyrrolidin-1-yl)pyridin-3-yl)-1,2,4-oxadiazole.

**17.** Composé selon l'une quelconque des revendications précédentes, dans lequel le composé est choisi parmi :

5-(1-(8-méthoxyquinolin-2-yl)pipéridin-4-yl)-3-(4-(trifluorométhoxy)phényl)-1,2,4-oxadiazole ;
chlorhydrate de 5-(1-(8-méthoxyquinolin-2-yl)pipéridin-4-yl)-3-(4-(trifluorométhyl)phényl)-1,2,4-oxadiazole ;
3-(3,5-diméthoxyphényl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(3-(trifluorométhyl)phényl)-1,2,4-oxadiazole ;
chlorhydrate de 5-(1-(8-méthoxyquinolin-2-yl)pipéridin-4-yl)-3-(pyridin-3-yl)-1,2,4-oxadiazole ;
5-(1-(8-méthoxyquinolin-2-yl)pipéridin-4-yl)-3-(6-(trifluorométhyl)pyridin-3-yl)-1,2,4-oxadiazole ;
chlorhydrate de 5-(1-(8-chloroquinolin-2-yl)pipéridin-4-yl)-3-(pyridin-3-yl)-1,2,4-oxadiazole ;
5-(1-(6-méthoxyquinolin-2-yl)pipéridin-4-yl)-3-(pyridin-3-yl)-1,2,4-oxadiazole ;
4-(5-(1-(8-méthoxyquinolin-2-yl)pipéridin-4-yl)-1,2,4-oxadiazol-3-yl)phénol ;
3-(pyridin-3-yl)-5-(1-(6-(pyridin-4-yl)quinolin-2-yl)pipéridin-4-yl)-1,2,4-oxadiazole ;
chlorhydrate de 3-(6-chloropyridin-3-yl)-5-(1-(8-méthoxyquinolin-2-yl)pipéridin-4-yl)-1,2,4-oxadiazole ;
5-(1-(8-méthoxyquinolin-2-yl)pipéridin-4-yl)-3-(6-(pipéridin-1-yl)pyridin-3-yl)-1,2,4-oxadiazole ;
5-(1-(8-méthoxyquinolin-2-yl)pipéridin-4-yl)-3-(6-(pipérazin-1-yl)pyridin-3-yl)-1,2,4-oxadiazole ;
chlorhydrate de 2-((5-(5-(1-(8-méthoxyquinolin-2-yl)pipéridin-4-yl)-1,2,4-oxadiazol-3-yl)pyridin-2-yl)amino) éthan-1-ol ; ou
chlorhydrate de 5-(1-(8-méthoxyquinolin-2-yl)pipéridin-4-yl)-3-(6-(pyrrolidin-1-yl)pyridin-3-yl)-1,2,4-oxadiazole.

**18.** Composé selon l'une quelconque des revendications précédentes destiné à être utilisé en médecine ou en médecine vétérinaire.

**19.** Composé selon l'une quelconque des revendications précédentes destiné à être utilisé dans le traitement ou la prévention de la tuberculose.

**20.** Composé destiné à être utilisé selon l'une quelconque des revendications précédentes 18-19 en combinaison avec au moins un agent anti-VIH, dans lequel l'agent anti-VIH est choisi parmi un inhibiteur de protéase du VIH, un inhibiteur nucléosidique de la transcriptase inverse du VIH, un inhibiteur non nucléosidique de la transcriptase inverse du VIH, ou un inhibiteur d'intégrase du VIH.

**21.** Composé destiné à être utilisé selon les revendications 18-20 en combinaison avec au moins un second médicament antituberculeux, dans lequel l'autre médicament antituberculeux est choisi parmi le groupe de isoniazide, rifamycine et ses dérivés, pyrazinamide, éthambutol, cyclosérine, éthionamide, streptomycine, amikacine, kanamycine, rifampine (rifampicine), aminosides, capréomycine, acide p-aminosalicylique, fluoroquinolones tels que la lévofloxacine,

moxyfloxacine ou gatifloxacine, ou des mélanges de ceux-ci.

**22.** Composition pharmaceutique comprenant un composé ou un composé destiné à être utilisée selon l'une quelconque des revendications précédentes 1-21 et un excipient pharmaceutiquement acceptable, dans lequel le composé est une quantité thérapeutiquement efficace d'un composé décrit dans l'une quelconque des revendications précédentes ou un sel ou solvate pharmaceutiquement acceptable de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1527050 B1
- US 2016113919 A1
- US 7868022 B2
- WO 2012025237 A9
- US 7989628 B2
- WO 2009001060 A3
- WO 2017001661 A1
- WO 2019243971 A


### Non-patent literature cited in the description


- **COMAS et al.** *Nat. Genet.*, 2013, vol. 45, 1176-1182
- *WHO Global Tuberculosis Report*, 2017, vol. 2017
- **HOAGLAND DT et al.** *Adv Drug Deliv Rev.*, 01 July 2016, vol. 102, 55-72
- **HOAGLAND DT et al.** *Adv Drug Deliv Rev*, 01 July 2016, vol. 102, 55-72
- **TANTRY, S. J. et al.** *Med. Chem. Commun.*, 2016, vol. 7, 1022-1032
- **REDASANI, V.K. et al.** Prodrug Design. Elsevier, 2015
- **PETER G. M. WUTS**. Greener's Protecting Groups in Organic synthesis. Wiley, 2014
- **CORY A. et al.** *Cancer Commun.*, 1991, vol. 3 (7), 207-212
- **PALOMINO JC et al.** *Mycobacterium tuberculosis Antimicrob. Agents Chemother.*, 2002, vol. 46 (8), 2720-2722
- **PALOMINO JC et al.** Mycobacterium tuberculosis Antimicrob.. *Agents Chemother.*, 2002, vol. 46 (8), 2720-2722